(19) Europäisches Patentamt
European Patent Office
Office européen des brevets

(11) Publication number: 0 073 451 B1

(12) EUROPEAN PATENT SPECIFICATION

(45) Date of publication of patent specification: 13.05.87

(21) Application number: 82107732.8

(22) Date of filing: 24.08.82

(51) Int. Cl.[4]: C 07 D 487/04, C 07 D 205/08 // C07D403/04, C07F7/18, C07D498/04, C07D498/10, C07D491/04, C07D405/06, C07F9/65 ,(C07D487/04, 209:00, 205:00)

(54) **Carbapenam compound and processes for the preparation thereof.**

(30) Priority: 27.08.81 US 296840

(43) Date of publication of application: 09.03.83 Bulletin 83/10

(45) Publication of the grant of the patent: 13.05.87 Bulletin 87/20

(84) Designated Contracting States: AT BE CH DE FR GB IT LI LU NL SE

(56) References cited:
EP-A-0 007 973
EP-A-0 026 816
EP-A-0 032 400
EP-A-0 037 080
EP-A-0 074 599

The file contains technical information submitted after the application was filed and not included in this specification

(73) Proprietor: Fujisawa Pharmaceutical Co., Ltd.
3, Doshomachi 4-chome Higashi-ku
Osaka-shi, Osaka 541 (JP)

(72) Inventor: Hashimoto, Masashi
No. 6-17, Satsukidai 1-chome
Nakayama Takarazuki-shi Hyogo (JP)
Inventor: Aratani, Matsuhiko
No. 21-203, Kawanakashinmachi
Daitoh-shi Osaka (JP)
Inventor: Sawada, Kozo
c/o Mr. Sakagami No. 11-8, Nishimachi 1-chome
Hotarugaike Toyonaka-shi Osaka (JP)

(74) Representative: Türk, Gille, Hrabal
Bruckner Strasse 20
D-4000 Düsseldorf 13 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to a new carbapenam compound and salt thereof. More particularly, it relates to a new carbapenam compound and salt thereof, which are useful intermediates for preparing carbapenem antibiotics, and to processes for the preparation thereof.

Accordingly, one object of this invention is to provide a new carbapenam compound and salt thereof, which are useful intermediates for preparing carbapenem antibiotics having a strong antimicrobial activity against pathogenic microorganisms and β-lactamase inhibitory activity.

Another object of this invention is to provide processes for the preparation of the carbapenam compound and salt thereof.

The object carbapenam compound can be represented by the following formula:

(I)

or a salt thereof in which

$R^1$ is 1-protected hydroxy-1-methylethyl and

$R^2$ is carboxy or a protected carboxy.

In the object compounds and the starting compounds in Processes 1 to 3 and methods A to B mentioned below, it is to be understood that there may be one or more stereoisomeric pair(s) such as optical and/or geometrical isomers due to asymmetric carbon atom(s) and double bond(s) in those molecules, and these isomers are also included within the scope of the present invention.

With regard to the object compounds (I), it is to be noted that the 3,7-dioxo-1-azabicyclo[3.2.0]heptane ring system of the following formula (I) is well known to lie in tautomeric relation with the 3-hydroxy-7-oxo-1-azabicyclo[3.2.0]hept-2-ene ring system of the following formula (I'), and accordingly, it is to be understood that both of these ring systems are substantially the same.

Tautomerism

(I) (I')

in which $R^1$ and $R^2$ are each as defined above.

Suitable salts of the object compounds (I) are conventional basic and may include, e.g., an inorganic basic salt, for example, a metal salt such as an alkali metal salt (e.g. sodium salt, potassium salt) and an alkaline earth metal salt (e.g. calcium salt, magnesium salt), and an ammonium salt and an organic basic salt, for example, an organic amine salt [e.g. trimethylamine salt, triethylamine salt, pyridine salt, picoline salt, dicyclohexylamine salt, N,N'-dibenzylethylenediamine salt, N-methylglucamine salt, diethanolamine salt, triethanolamine salt, tris(hydroxymethylamino)methane salt].

According to the present invention, the object compounds (I) and salts thereof can be prepared by the processes as illustrated by the following reaction schemes.

(II)
or a salt thereof

Malonic acid
or its derivative or a salt thereof
(Process 1)

(III)
or a salt thereof

Acid azide
(Process 2)

(IV)
or a salt thereof

Cyclization
(Process 3)

(I)

or a salt thereof in which $R^1$ and $R^2$ are each as defined above.

The starting compound (IIa) and other useful intemediate compounds for preparing carbapenem compound (e.g. carpetimycin, thienamycin) can be prepared, for example, by the following methods.

Method A

CH2CH=CH2
CH3
CH3
R3
(V)
or a salt thereof
CN
(VII)
or a salt thereof
Alkylation
(Method A-1)
Alkylation
(Method A-2)
(VIII)
or a salt thereof
Removal of the
isocyano group
(Method A-3)
(VI)
or a salt thereof
Introduction of the
hydroxy-protective group
(If desired)
(Method A-4)
(IX)
or a salt thereof

Oxidation
(Method A-5)

(IIa)
or a salt thereof

Method B

(X)

Alkylation
(Method B-1)

(XI)

Removal of the isocyano group
(Method B-2)

(XII)

Introduction of the hydroxy-protective group (Method B-3) (If desired)

(XIII)

Hydrolysis (Method B-4)

(XIV) or a salt thereof

Oxidation (Method B-5)

(IIa) or a salt thereof

in which

$R_a^1$ is lower alkyl or hydroxy(lower)alkyl,

$R_b^1$ is lower alkyl, hydroxy(lower)alkyl or a protected hydroxy(lower)alkyl,

$R^3$ is carboxy or a protected carboxy, and

$R^4$ and $R^5$ are each lower alkyl.

Further, the object compounds (I) or a salt thereof can be led to the carbapenem antibiotics and salts thereof, for example, by the process as illustrated by the following reaction schemes.

Processes for preparing carbapenam antibiotics

(I) or a salt thereof — Acylating agent → (XV) or a salt thereof

(XV) — $R^7SH$ or a salt thereof → (XVI) or a salt thereof

(XVI) — Removal of the protective group(s) (If desired) → (XVII) or a salt thereof

(XVI) — Oxidation → (XVIII) or a salt thereof

in which

$R^1$ and $R^2$ are each as defined above,

$R^6$ is acyl,

$R^7$ is acylamino(lower)alkyl or acylamino(lower)alkenyl, and

$R^7_a$ is amino(lower)alkyl, acylamino(lower)alkyl or acylamino(lower)alkenyl.

In the above and subsequent descriptions of the present specification, suitable examples and illustrations of the various definitions which the present invention include within the scope thereof are explained in details as follows.

The term "lower" is intended to mean 1 to 6 carbon atoms, unless otherwise indicated.

Suitable "protected hydroxy moiety" in the terms "1-protected hydroxy-1-methylethyl" and "protected hydroxy(lower)alkyl" may include, e.g., a hydroxy group substituted by a conventional hydroxy-protective group, which is commonly used in β-lactam compounds such as thienamycin or carpetimycin derivatives or its analogue, for example, acyl such as mono (or di or tri)phenyl(lower)alkoxycarbonyl optionally substituted by nitro (e.g. benzyloxycarbonyl, benzhydryloxycarbonyl, trityloxycarbonyl, 4-nitrobenzyloxycarbonyl), ar(lower)alkyl such as mono(or di- or tri)phenyl(lower)alkyl (e.g. benzyl, benzhydryl, trityl), trisubstituted silyl such as tri(lower)alkylsilyl (e.g. trimethylsilyl, triethylsilyl, isopropyl-dimethylsilyl, tert-butyl-dimethylsilyl, dissopropyl-methylsilyl), triarylsilyl (e.g. triphenylsilyl) or triar(lower)alkylsilyl (e.g. tribenzylsilyl).

Suitable "protected carboxy group" may include an esterified carboxy group which is commonly used in β-lactam compounds such as penicillin and cephalosporin compounds at their 3rd and 4th positions thereof. Suitable "ester moiety" in "esterified carboxy group" may include, e.g., lower alkyl ester (e.g. methyl ester, ethyl ester, propyl ester, isopropyl ester, butyl ester, isobutyl ester, t-butyl ester, pentyl ester, tert-pentyl ester, hexyl ester), lower alkenyl ester (e.g. vinyl ester, allyl ester), lower alkynyl ester (e.g.

ethynyl ester, propynyl ester, etc.), lower alkoxy(lower)alkyl ester (e.g. methoxymethyl ester, ethoxymethyl ester, isopropoxymethyl ester, 1-methoxyethyl ester, 1-ethoxyethyl ester), lower alkylthio(lower)alkyl ester (e.g. methylthiomethyl ester, ethylthiomethyl ester, ethylthioethyl ester isopropylthiomethyl ester), amino- and carboxy-substituted-lower alkyl ester (e.g. 2-amino-2-carboxyethyl ester, 3-amino-3-carboxypropyl ester), protected amino- and protected carboxy-substituted-lower alkyl ester such as lower alkoxycarbonylamino- and mono (or di or tri)phenyl(lower)alkoxycarbonyl-substituted-lower alkyl ester (e.g. 2-tert-butoxycarbonylamino-2-benzhydryloxycarbonylethyl ester, 3-tert-butoxycarbonyl-amino-3-benzhydryloxycarbonylpropyl ester), mono(or di or tri)halo(lower)alkyl ester (e.g. 2-iodoethyl ester, 2,2,2-trichloroethyl ester), lower alkanoyloxy(lower)alkyl ester (e.g. acetoxymethyl ester, propionyloxymethyl ester, butyryloxymethyl ester, isobutyryloxymethyl ester, valeryloxymethyl ester, pivaloyloxymethyl ester, hexanoyloxymethyl ester, 2-acetoxyethyl ester, 2-propionyloxyethyl ester, 1-acetoxypropyl ester), lower alkanesulfonyl(lower)alkyl ester (e.g. mesylmethyl ester, 2-mesylethyl ester), ar(lower)alkyl eser which may have one or more substituent(s) such as mono(or di or tri)phenyl(lower)alkyl ester which may have one or more suitable substituent(s) (e.g. benzyl ester, 4-methoxybenzyl ester, 4-nitrobenzyl ester, phenethyl ester benzhydryl ester, trityl ester, bis(methoxyphenyl)methyl ester, 3,4-dimethoxybenzyl ester, 4-hydroxy-3,5-di-t-butylbenzyl ester), aryl ester which may have one or more suitable substituents (e.g. phenyl ester, tolyl ester, t-butylphenyl ester, xylyl ester, mesityl ester, cumenyl ester, salicyl ester), heterocyclic ester (e.g. phthalidyl ester), trisubstituted silyl ester (e.g. trimethylsilyl ester, triethylsilyl ester, tert-butyl-dimethylsilyl ester, isopropyldimethylsilyl ester and phenyl-dimethylsilyl ester). More preferred example of the protected carboxy group thus defined may be mono(or di or tri)phenyl(lower)alkoxycarbonyl which may have nitro, and the most preferred one may be 4-nitrobenzyloxycarbonyl.

Suitable "lower alkyl" and "lower alkyl moiety" in the terms "hydroxy(lower)alkyl", "protected hydroxy(lower)alkyl", "amino(lower)alkyl" and "acylamino(lower)alkyl" may include, e.g., methyl, ethyl, propyl, isopropyl, butyl, isobutyl, tert-butyl, pentyl, neopentyl, tert-pentyl, hexyl, in which the preferred one may include $C_1$—$C_3$alkyl.

Suitable "lower alkenyl moiety" in the term "acylamino(lower)alkenyl" may include, e.g., vinyl and propenyl.

Suitable "acyl moiety" in the terms "acylamino(lower)alkyl" and "acylamino(lower)alkenyl" may include, e.g., lower alkanoyl (e.g. formyl, acetyl) and phenyl(lower)alkoxycarbonyl which may have nitro (e.g. benzyloxycarbonyl, 4-nitrobenzyloxycarbonyl).

Suitable "acyl" may include a residue of an organic sulfonic or phosphoric acid such as diaryloxyphosphoryl (e.g. diphenoxyphosphoryl).

Suitable salts of the compounds (V) or (IX) and (XV) to (XVIII) may include the same as those for the object compound (I).

Suitable salts of the compounds (II), (IIa), (III) and (IV) are conventional basic or acidic salts and may include, e.g., an inorganic basic salt, for example, a metal salt such as an alkali metal salt (e.g. sodium salt, potassium salt) and an alkaline earth metal salt (e.g. calcium salt, magnesium salt), and an ammonium salt; an organic basic salt, for example an organic amine salt [e.g. trimethylamine salt, triethylamine salt, pyridine salt, picoline salt, dicyclohexylamine salt, N,N'-dibenzylethylenediamine salt, N-methylglucamine salt, diethanolamine salt, triethanolamine salt and tris(hydroxymethylamino)methane salt]; an organic carboxylic or sulfonic acid addition salt (e.g. formate, acetate, maleate, tartrate, methanesulfonate, benzenesulfonate, toluenesulfonate); an inorganic acid addition salt (e.g. hydrochloride, hydrobromide, sulfate, phosphate); and a salt with a baisc or acidic amino acid (e.g. arginine, aspartic acid, glutamic acid).

Suitable salt of the compound (XIV) may include the same acid addition salt as those for the compounds (II), (IIa), (III) and (IV).

The processes for preparing the object compounds (I) of the present invention are explained in detail in the following.

Process 1:

The compound (III) or a salt thereof can be prepared by reacting the compound (II) or a salt thereof with malonic acid or its derivative or a salt thereof.

Suitable salt of the malonic acid or its derivative may include the same salt with a base as that for the compound (I).

Suitable derivative of the malonic acid may include mono- or di-ester such as exemplified in the explanation of "protected carboxy group".

The reaction can be carried out in a conventional solvent such as dioxane, tetrahydrofuran, N,N-dimethylformamide or any other organic solvents which do not adversely influence the reaction, or a mixture thereof, in the presence of a condensing agent such as carbodiimide compounds (e.g. N,N'-diethylcarbodiimide, N,N-diisopropylcarbodiimide, N,N'-dicyclohexylcarbodiimide, N-cyclohexyl-N'-morpholinoethylcarbodiimide, N-cyclohexyl-N'-(4-diethylaminocyclohexyl)-carbodiimide, N-ethyl-N'-(3-dimethylaminopropyl)-carbodiimide); N,N'-carbonylbis-(2-methylimidazole); keteneimine compounds (e.g. pentamethyleneketene-N-cyclohexylimine, diphenylketene-N-cyclohexylimine); ethoxyacetylene; 1-alkoxy-1-chloroethylene; ethyl polyphosphate; isopropyl polyphosphate; phosphorus oxychloride; phosphorus trichloride; thionyl chloride; oxalyl chloride; a combination of triphenylphosphine with carbon tetrachloride or diazenedicarboxylate; 2-ethyl-7-hydroxybenzisoxazolium salt; 2-ethyl-5-(m-sulfophenyl)isoxazolium hydroxide intra-molecular salt; 1-(p-chlorobenzenesulfonyloxy)-6-chloro-1H-benzotriazole; and so-called Vilsmeier reagent prepared by the reaction of N,N-dimethylformamide with, e.g., thionyl chloride, phosgene and phosphorus oxychloride.

The reaction temperature is not critical, and the reaction is usually carried out under from cooling to warming.

Process 2:

The compound (IV) or a salt thereof can be prepared by reacting the compound (III) or a salt thereof with acid azide.

Suitable acid azide may include, e.g., arenesulfonyl azide which may have substituent (e.g. benzenesulfonyl azide, p-toluenesulfonyl azide, p-carboxybenzenesulfonyl azide), lower alkenesulfonyl azide (e.g. methanesulfonyl azide, ethanesulfonyl azide).

The reaction is usually carried out in a conventional solvent such as tetrahydrofuran, dioxane, methylene chloride, acetonitrile, N,N-dimethylformamide or any other organic solvents which do not adversely influence the reaction, or a mixture thereof, in the presence of an inorganic or organic base such as an alkali metal bicarbonate (e.g. sodium bicarbonate, potassium bicarbonate), alkali metal carbonate (e.g. sodium carbonate, potassium carbonate), alkaline earth metal carbonate (e.g. magnesium carbonate, calcium carbonate), tri(lower)alkylamine (e.g. trimethylamine, triethylamine, N,N-diisopropyl-N-ethylamine), pyridine compounds (e.g. pyridine, picoline, lutidine, N,N-di(lower)alkylaminopyridine such as N,N-dimethylaminopyridine), quinoline, N-lower alkylmorphorine (e.g. N-methylmorphorine), and N,N-di(lower)alkylbenzylamine (e.g. N,N-dimethylbenzylamine).

The reaction temperature is not critical, and the reaction is usually carried out under from cooling to warming.

Process 3:

The compound (I) or a salt thereof can be prepared by subjecting the compound (IV) or a salt thereof to cyclization.

The reagent used in this cyclization reaction may include, e.g., metal (e.g. cupper), metallic acetate (e.g. lead acetate, palladium acetate, rhodium acetate, cupric acetate) and metallic sulfate (e.g. cupric sulfate).

The reaction is usually carried out in a conventional solvent such as benzene, toluene, tetrahydrofuran, dioxane, acetonitrile or any other organic solvents which do not adversely influence the reaction, or a mixture thereof.

The reaction temperature is not critical, and the reaction is usually carried out from an ambient temperature to heating.

Methods A to B for preparing the new starting compound (II) used in Process 1 are explained in details in the following.

Method A—1: (V) → (VI)

The compound (VI) or a salt thereof can be prepared by reacting the compound (V) or a salt thereof with a metal amide and then reacting the resultant compound with a lower alkylating agent or a hydroxy(lower)alkylating agent such as an oxo(lower)alkane or a lower alkyl halide.

The metal amide to be used in this reaction may include, e.g., an alkali metal amide (e.g. lithium isopropyl cyclohexylamide).

The oxo(lower)alkane to be used in this reaction may include, e.g., acetone and acetaldehyde.

The lower alkyl halide to be used in this reaction may include, e.g., metal iodide and ethyl iodide.

This reaction is usually carried out in a conventional solvent which does not adversely influence the reaction such as tetrahydrofuran, dimethoxyethane and ether.

The reaction temperature of this reaction is not critical and the reaction is preferably carried out from under cooling to an ambient temperature.

Method A—2: (VII) → (VIII)

Method B—1: (X) → (XI)

The compounds (VIII) or a salt thereof and (XI) can be prepared by reacting the corresponding compounds (VII) or a salt thereof and (XI) with an organo lithium compound, respectively and then reacting the resultant compound with a lower alkylating agent or a hydroxy(lower)alkylating agent such as an oxo(lower)alkane or mono(or di)lower alkyl sulfate.

The organo lithium compound may include, e.g., alkyl lithium (e.g. n-butyl lithium), aryl lithium and aralkyl lithium.

The oxo(lower)alkane to be used in this reaction may include, e.g., acetone and acetaldehyde. Mono(or di)lower alkyl sulfate may include, e.g., mono (or di)-methyl sulfate and mono(or di)ethyl sulfate.

This reaction is usually carried out in a conventional solvent which does not adversely influence the reaction such as tetrahydrofuran, dimethoxyethane and ether.

The reaction temperature of this reaction is not critical and the reaction is preferably carried out from under cooling to at ambient temperature.

Method A—3: (VIII) → (VI)

Method B—2: (XI) → (XII)

The compounds (VI) or a salt thereof and (XII) can be prepared by subjecting the corresponding compounds (VIII) or a salt thereof and (XI) to removal reaction of the isocyano group, respectively.

This removal reaction can be carried out in the presence of a reducing agent such as di(or tri)alkyltin hydride (e.g. tri(n-butyl)tin hydride, triaryltin hydride (e.g. triphenyltin hydride).

The reaction is usually carried out in a conventional solvent which does not adversely influence the reaction such as water, lower alkanol (e.g. methanol, ethanol, propanol), tetrahydrofuran, N,N-dimethylformamide, benzene, toluene, or a mixture thereof.

The reaction temperature of this reaction is not critical and the reaction is preferably carried out from under cooling to under warming.

Method A—4: (VI) → (IX)

Method B—3: (XII) → (XIII)

The compounds (IX) or a salt thereof and (XIII) can be prepared by subjecting the corresponding compounds (VI) or a salt thereof and (XII) to introducing reaction of the hydroxy-protective group, respectively.

The introducing agent of the hydroxy-protective group used in this reaction may include, e.g., an acylating agent [e.g. an organic carboxylic, carbonic and sulfonic acid or a reactive derivative thereof (e.g. acid halide, an acid anhydride, an activated amide, an activated ester)].

Suitable examples of the introducing agent may include, e.g., phenyl(lower)alkyl haloformate which may have nitro (e.g. 4-nitrobenzyl chloroformate).

This introduction reaction is preferably carried out in the presence of an organic or inorganic base such as alkyl lithium (e.g. n-butyl lithium).

The reaction is usually carried out in a conventional solvent which does not adversely influence the reaction such as dioxane, benzene, tetrahydrofuran, N,N-dimethylformamide, pyridine, hexamethylphosphoramido, or a mixture thereof.

This reaction temperature is not critical and the reaction is usually carried out from under cooling to at ambient temperature.

Method B—4: (XIII) → (XIV)

The compound (XIV) or a salt thereof can be prepared by hydrolysing the compound (XIII).

This hydrolysis is usually carried out in the presence of an inorganic or organic acid such as hydrochloric acid, hydrobromic acid and acetic acid.

This hydrolysis can be carried out in a conventional solvent which does not adversely influence the reaction such as water.

This reaction temperature is not critical and the reaction is usually carried out from under cooling to under heating.

Method A—5: (IX) → (II)
Method B—5: (XIV) → (II)

The compound (IIa) or a salt thereof can be prepared by oxidizing the corresponding compounds (IX) or a salt thereof or (XIV) or a salt thereof.

This oxidation is usually carried out in the presence of an oxidizing agent such as ozone in combination with a per acid (e.g. m-chloroperbenzoic acid), alkali metal permanganate (e.g. potassium permanganate), alkali metal permanganate in combination with alkali metal periodate (e.g. sodium periodate), a mixture of chromium trioxide and sulfuric acid or a base salt of dichromic acid (e.g. pyridinium dichromate).

This reaction is usually carried out in the presence of a conventional solvent which does not adversely influence the reaction such as water, methanol, ethanol, tetrahydrofuran, dioxane, acetone, dichloromethane, N,N-dimethylformamide, a mixture thereof.

The suitable reaction solvent is selected according to a kind of the oxidizing agent to be used.

This reaction temperature is not critical and the reaction is usually carried out from under cooling to under heating.

The process for preparing the carbapenem antibiotics from the object compound (I) or a salt thereof are explained in detail in the following.

Processes for preparing the carbapenem antibiotics

The compound (XV) or a salt thereof can be prepared by reacting the compound (I) or a salt thereof with an acylating agent.

The present reaction can be carried out in a conventional acylation manner.

Suitable acylating agents may be, e.g., organic sulfonic or phosphoric acid or its reactive derivative such as acid halide, acid anhydride, for example, diarylphosphoryl halide (e.g. diphenylphosphoryl chloride, ditolylphosphoryl chloride).

The reaction is usually carried out in a conventional solvent such as water, acetone, dioxane, acetonitrile, chloroform, methylene chloride, hexamethylphosphoramide, ethylene chloride, tetrahydrofuran, ethyl acetate, dimethylsulfoxide, N,N-dimethylformamide, pyridine or any other organic solvents which do not adversely influence the reaction, or a mixture thereof.

When the acylating agent is used in a free acid form or its salt form in this reaction, the reaction is preferably carried out in the presence of a conventional condensing agent as exemplified in the explanation of the process 1.

The reaction may also be carried out in the presence of an inorganic or organic base as exemplified in the explanation of the process 2.

The reaction temperature is not critical, and the reaction is usually carried out under from cooling to warming.

The compound (XV) or a salt thereof can be isolated in a conventional manner, but can also be used as the starting compound of the Second Step without any isolation.

The compound (XVI) or a salt thereof can be prepared by reacting the compound (XV) or a salt thereof with a thiol compound of the formula: $R^7SH$ ($R^7$ is the same as defined above) or a salt thereof.

Suitable salt of the thiol compound may include the same salt with a base as that for the compound (I).

The reaction is usually carried out in a conventional solvent such as those given in the explanation of the First Step.

The reaction may also be carried out in the presence of an inorganic or organic base such as those given in the explanation of the First Step.

The reaction temperature is not critical, and the reaction is usually carried out under from cooling to warming.

The compound (XVII) or a salt thereof can be prepared by subjecting the compound (XVI) or a salt thereof to removal reaction of the protective group(s).

The present reaction is carried out in a conventional method such as hydrolysis or reduction.

Hydrolysis is preferably carried out in the presence of a base or an acid in a conventional manner.

The reaction temperature is not critical and the reaction is usually carried out under from cooling to warming.

The reduction method applicable for this removal reaction may include, for example, reduction by using a combination of a metal (e.g. zinc, zinc amalgam) or a salt of chrome compound (e.g. chromous chloride, chromous acetate) and an organic or inorganic acid (e.g. acetic acid, propionic acid, hydrochloric acid, sulfuric acid); and conventional catalytic reduction in the presence of a conventional metallic catalyst such as palladium catalysts (e.g. spongy palladium, palladium black, palladium oxide, palladium on carbon, colloidal palladium, palladium on barium sulfate, palladium on barium carbonate), nickel catalysts (e.g. reduced nickel, nickel oxide, Raney nickel) and platinum catalysts (e.g. platinum plate, spongy platinum, platinum black, colloidal platinum, platinum oxide, platinum wire).

The reaction is usually carried out in a conventional solvent such as water, alkanol (e.g. methanol, ethanol, propanol), dioxane, tetrahydrofuran, acetic acid or any other organic solvents which do not adversely influence the reaction, or a mixture thereof around a neutral condition.

The reaction temperature is not critical, and the reaction is usually carried out under from cooling to warming.

The removal methods can be selected according to the kind of the protective groups to be removed.

The compound (XVIII) or a salt thereof can be prepared by oxidizing the compound (XVI) or a salt thereof. The present oxidation reaction can be carried out in a similar manner to that in Method A—5, accordingly the reaction condition of present oxidation can be referred to that in Method A—5.

The following test data show an antimicrobial activity of the representative compound of the carbapenem compounds (XVI) and (XVII).

Test Method

One loopful of an overnight culture of each test strain in Trypticase-soy broth ($10^8$ viable cells per ml.) was streaked on heart infusion agar (HI-agar) containing graded concentrations of antibiotics, and the minimal inhibitory concentration (MIC) was expressed in terms of µg/ml after incubation at 37°C for 20 hours.

Test Compound

Potassium (5R,6R)-3-(2-aminoethylthio)-6-(1-hydroxy-1-methylethyl)-7-oxo-1-azabicyclo[3.2.0]-hept-2-ene-2-carboxylate.

Test Result

| Test organisms | MIC (μg/ml) |
|---|---|
| Staphylococcus aureus 6 | 1.56 |
| Pseudomonas aeruginosa NCTC-10490 | 12.50 |

The following Preparations and Examples are given for the purpose of illustrating the present invention.

Preparation 1

A mixture of methyl 2-[(3S,4R)-4-chloro-3-phthalimido-2-oxoazetidin-1-yl]-3-methyl-2-beutenoate (3.55 g) and allyltrimethylsilane (2.26 ml) in methylene chloride (25 ml) was cooled to −78°C, and silver tetrafluoroborate (2.29 g) was added. The mixture was stirred for 2 hours during which time the temperature was gradually raised to 0°C, and the stirring was continued for another 3.5 hours. Saturated aqueous sodium chloride (10 ml) was added to the reaction mixture and the pH value of the aqueous layer was adjusted to pH 7 by adding saturated aqueous sodium bicarboante. After stirring for 20 minutes at 0°C, the precipitate was filtered off through a pad of Celite (trade mark) and the filtrate was separated into the organic layer and the aqueous layer. The organic layer was washed with brine, dried over magnesium sulfate and evaporated to leave an oil (4.0 g), which was chromatographed on silica gel (50 g) eluting with a mixture of benzene and acetone (10:1) to give methyl 2-[(3R,4R)-4-allyl-3-phthalimido-2-oxoazetidin-1-yl]-3-methyl-2-butenoate (2.74 g) as an oil.

IR ($CH_2Cl_2$) 1770 (shoulder), 1760, 1720 $cm^{-1}$.

NMR ($CDCl_3$) δ: 2.11 (s, 3H), 2.26 (s, 3H), 2.4—2.6 (m, 2H), 4.40 (m, 1H), 5.0—5.3 (m, 3H), 5.70 (m, 1H).

Preparation 2

To a solution of methyl 2-[(3R,4R)-4-allyl-3-phthalimido-2-oxoazetidin-1-yl]-3-methyl-2-butenoate (2.64 g) in a mixture of methylene chloride (20 ml) and methanol (10 ml) was added N,N-dimethyl-1,3-propanediamine (1.98 ml) and the mixture was left in a refrigerator for 3 days and at room temperature for 24 hours. The reaction mixture was evaporated and the residue was dissolved in ethyl acetate (50 ml), washed with water, dried over magnesium sulfate and evaporated to give an oil, which was chromatographed on silica gel eluting with a mixture of methylene chloride and acetone (6—5:1) to give methyl 2-[(4R)-3-amino-4-allyl-2-oxoazetidin-1-yl]-3-methyl-2-butenoate (873 mg) as an oil.

IR ($CH_2Cl_2$): 1755, 1720 $cm^{-1}$.

Preparation 3

To a solution of methyl 2-[(3R,4R)-4-allyl-3-phthalimido-2-oxoazetidin-1-yl]-3-methylbut-2-enoate (6.78 g) in a mixture of methylene chloride (46 ml) and methanol (23 ml) was added N,N-dimethyl-1,3-propanediamine (6.25 ml) at 0°C and the mixture was left at room temperature for 5 days. The reaction mixture was evaporated and the residue was chromatographed on silica gel eluting with a mixture of methylene chloride and ethyl acetate (10:1—5) to give methyl 2-[(3R,4R)-3-amino-4-allyl-2-oxoazetidin-1-yl]-3-methylbut-2-enoate (3.70 g) as an oil.

IR ($CH_2Cl_2$): 1755, 1720 $cm^{-1}$.

NMR ($CD_3COCD_3$) δ: 1.92 (3H, s), 1.15 (3H, s), 2.45 (broad t, 2H, J=6.5Hz), 3.73 (3H, s), 4.13 (d and t, 1H, J=6.5Hz, 2.5Hz), 4.58 (1H, d, J=2.5Hz), 4.9—5.2 (2H, m), 5.5—6.0 (1H, m).

Preparation 4

To a solution of methyl 2-[(3R,4R)-3-amino-4-allyl-2-oxoazetidin-1-yl]-3-methylbut-2-enoate (500 mg) in dichloromethane (5 ml) was added acetic formic anhydride (0.34 ml) at 0°. After stirring at the same temperature for one hour, the mixture was diluted with ethyl acetate (35 ml) and washed with a dilute aqueous sodium bicarbonate. The aqueous layer was saturated with sodium chloride and extracted with dichloromethane. The extracts were combined, washed with a saturated aqueous sodium chloride, dried over magnesium sulfate and evaporated. The residue was chromatographed on silica gel (2.5 g) eluting with 10—30% acetone in dichloromethane afforded methyl 2-[(3R,4R)-3-formamido-4-allyl-2-oxoazetidin-1-yl]-3-methylbut-2-enoate (485 mg).

IR ($CH_2Cl_2$): 3400, 1760, 1720 (sh), 1695 $cm^{-1}$.

NMR ($CDCl_3$) δ: 1.97 (s, 3H), 2.21 (s, 3H), 2.47 (t, J=7Hz, 2H), 3.76 (s, 3H), 3.95 (dt, J=3, 7Hz, 1H), 4.77 (dd, J=3, 7Hz, 1H), 4.9—5.3 (m, 2H), 5.5—6.0 (m, 1H), 7.15 (brd, J=7Hz, 1H), 8.19 (s, 1H).

Mass Spectrum: m/e 266 ($M^+$).

Preparation 5

2,6-Lutidine (3.82 ml) and phosphorus oxychloride (1.37 ml) were added to a solution of methyl 2-[(3R,4R)-3-formamido-2-oxo-4-allylazetidin-1-yl]-3-methylbut-2-enoate (970 mg) in dichloromethane (10 ml) at 0°C, and the mixture was stirred for three hours at 0°C. Then, the reaction mixture was poured into a mixture of ethyl acetate (100 ml) and brine (50 ml). The organic layer was washed with brine, dried over magnesium sulfate and evaporated in vacuo. The oily residue was chromatographed on silica gel (15 g) eluting with a mixture of hexane and ethyl acetate (10:1—3:1) to give methyl 2-[(3R,4R)-3-isocyano-2-oxo-4-allylazetidin-1-yl]-3-methylbut-2-enoate (766 mg) as an oil.

IR ($CH_2Cl_2$): 2145, 1775, 1720 $cm^{-1}$.

NMR ($CDCl_3$) δ: 1.96 (s, 3H), 2.24 (s, 3H), 2.3—2.6 (m, 2H), 3.80 (s, 3H), 4.20 (dt, 1H, J=2.5, 6.5Hz), 4.98 (d, 1H, J=2.5Hz), 5.0—5.3 (m, 2H), 5.5—6.0 (m, 1H)

Mass Spectrum: m/e 248 ($M^+$)

Preparation 6

2,2'-Azobis(2-methylpropionitrile) (98 mg) and tributyltin hydride (0.95 ml) were added to a solution of methyl 2-[(3R,4R)-3-isocyano-2-oxo-4-allylazetidin-1-yl]-3-methylbut-2-enoate (740 mg) in benzene (15 ml), and the mixture was refluxed for 20 minutes. The reaction mixture was chromatographed on silica gel (15 g) eluting with a mixture of hexane and ethyl acetate (5:1 – 3:1) to give methyl 2-[(4R)-2-oxo-4-allylazetidin-1-yl]-3-methylbut-2-enoate (660 mg).

IR ($CH_2Cl_2$): 1750, 1720 $cm^{-1}$.

NMR ($CDCl_3$)δ: 1.97 (s, 3H), 2.20 (s, 3H), 2.3—2.5 (m, 2H), 2.63 (dd, 1H, J=3, 16Hz), 3.11 (dd, 1H, J=5, 16Hz), 3.77 (s, 3H), 3.7—4.2 (m, 1H), 4.8—5.3 (m, 2H), 5.4—6.1 (m, 1H).

Preparation 7

A 0.9M solution of diethylaluminum chloride in hexane (24.4 ml) was added to a suspension of activated zinc powder (4.0 g) in tetrahydrofuran (60 ml). After ten minutes the mixture was cooled to −20°C. A solution of methyl 2-[(3S,4R)-3-bromo-4-methylthio-2-oxoazetidin-1-yl]-3-methylbut-2-enoate (6.16 g) and acetone (1.84 ml) in tetrahydrofuran (45 ml) was added dropwise during 35 minutes at −20°C under a nitrogen atmosphere. The resulting mixture was stirred at −15 to −10°C for 1.5 hours and at 0°C for 30 minutes. The reaction was quenched by addition of pyridine (3 ml). After ten minutes 2N hydrochloric acid (40 ml) and ethyl acetate (100 ml) were added. The mixture was filtered through diatomaceous earth and the filtrate was diluted with 2N hydrochloric acid (40 ml) and ethyl acetate (100 ml). The mixture was shaken and the organic layer was separated. The aqueous layer was extracted with ethyl acetate (50 ml). The combined extracts were washed three times with brine, dried over magnesium sulfate, and evaporated to give an oil (6.0 g). The residue was chromatographed on silica gel (150 g, eluting with 2 to 25% acetone in dichloromethane) to give 3.79 g (66.0%) of methyl 2-[(3S,4R)-3-(1-hydroxy-1-methylethyl)-4-methylthio-2-oxoazetidin-1-yl]-3-methylbut-2-enoate and 1.36 g of impure material. Rechromatography of the impure fraction on silica gel (100 g, eluting with 2 to 25% acetone in dichloromethane) afforded an additional 0.93 g (16.2%) of the desired product:

IR ($CH_2Cl_2$): 3550, 1745 and 1715 $cm^{-1}$.

NMR ($CDCl_3$) δ: 1.34 (s, 3H), 1.41 (s, 3H), 2.00 (s, 3H), 2.13 (s, 3H), 2.21 (s, 3H), 2.56 (s, 1H), 3.14 (d, J=3Hz, 1H), 3.75 (s, 3H), and 5.01 (d, J=3Hz, 1H).

Preparation 8

A 1.55N solution of n-butyl lithium in hexane (0.44 ml) was added dropwise to a solution of methyl 2-[(3S,4R)-3-(1-hydroxy-1-methylethyl)-4-methylthio-2-oxoazetidin-1-yl]-3-methylbut-2-enoate (164 mg) in

tetrahydrofuran (2 ml) at −78°C under a nitrogen atmosphere. After five minutes a solution of 4-nitrobenzyl chloroformate (147 mg) in tetrahydrofuran (1.5 ml) was added. The mixture was allowed to warm to 0°C during 1.5 hours and stirred at the same temperature for 30 minutes. After being left at ambient temperature for 30 minutes, the reaction was quenched by addition of acetic acid (three drops). The mixture was evaporated, and the residue was taken up into ethyl acetate (20 ml) and washed in turn with a dilute aqueous solution of sodium bicarbonate and brine. Drying over magnesium sulfate and removal of the solvent left an oil which was chromatographed on silica gel (6.5 g; eluting with 1 to 2% ethyl acetate in dichloromethane for the desired product followed by 20% acetone in dichloromethane for the starting material) to give 170 mg of the desired product contaminated with some impurities and 52 mg (31.7% recovery) of the starting material. Further purification of the impure product on silica gel plates (20 cm × 20 cm × 2 mm, two pieces; two developments with 2/1 hexane-ethyl acetate) afforded 138 mg (51.8%; i.e. 83.4% based on consumed starting material) of methyl 3-methyl-2-[(3R,4R)-3-[1-methyl-1-(4-nitrobenzyloxycarbonyloxy)ethyl]-4-methylthio-2-oxoazetidin-1-yl]but-2-enoate.

IR ($CH_2Cl_2$): 1750, 1720, 1520 and 1350 $cm^{-1}$.

NMR ($CDCl_3$) δ: 1.64 (s, 3H), 1.71 (s, 3H), 2.01 (s, 3H), 2.09 (s, 3H), 2.23 (s, 3H), 3.70 (1H, hidden), 3.75 (s, 3H), 5.07 (d, J=3Hz, 1H), 5.20 (s, 2H), 7.57 (d, J=9Hz, 2H), and 8.18 (d, J=9Hz, 2H).

Preparation 9

OCOOPNB, $(CH_3)_2C$, H, H, $SCH_3$, O, N, $COOCH_3$ → OCOOPNB, $(CH_3)_2C$, H, H, Cl, O, N, $COOCH_3$ + OCOOPNB, $(CH_3)_2C$, H, H, Cl, O, N, $COOCH_3$

A solution of chlorine (70 mg) in carbon tetrachloride (1.75 ml) was added to a solution of methyl 3-methyl-2-[(3S,4R)-3-[1-methyl-1-(4-nitrobenzyloxycarbonyloxy)-ethyl]-4-methylthio-2-oxoazetidin-1-yl]but-2-enoate (386 mg) in dichloromethane (4 ml) at −78°C. The mixture was allowed to warm to 0°C during 40 minutes and evaporated. The residue was dissolved in carbon tetrachloride (5 ml) and evaporated. This procedure was repeated once and the residue was chromatographed on silica gel (8 g, eluting with 10 to 30% ethyl acetate in hexane) to give a 4:1 mixture of methyl 2-[(3S,4R)-4-chloro-3-[1-methyl-1-(4-nitrobenzyloxycarbonyloxy)ethyl]-2-oxoazetidin-1-yl]-3-methylbut-2-enoate [(3S,4R)-isomer] and its (3S,4S)-isomer.

IR ($CH_2Cl_2$): 1770, 1745, 1720, 1520 and 1350 $cm^{-1}$.

NMR ($CDCl_3$) δ: 1.67 (s, 2.4H), 1.74 (s, 2.4H), 1.79 (s, 0.6H), 1.82 (s, 0.6H), 2.02 (s, 2.4H), 2.08 (s, 0.6H), 2.30 (s, 3H), 3.74 (s, 2.4H), 3.75 (s, 0.6H), 3.93 (d, J=2Hz, 0.8H), 4.02 (d, J=4Hz, 0.6H), 5.21 (s, 2H), 5.91 (d, J=2Hz, 0.8H), 5.95 (d, partially hidden, 0.2H), 7.53 (d, J=9Hz, 2H), and 8.22 (d, J=9Hz, 2H).

Preparation 10

OCOOPNB, $(CH_3)2C$, H, H, $SCH_3$, O, N, $COOCH_3$ →

OCOOPNB, $(CH_3)_2C$, H, H, Cl, O, N, $COOCH_3$ + OCOOPNB, $(CH_3)2C$, H, H, Cl, O, N, $COOCH_3$

Methyl 3-methyl-2-[(3S,4R)-3-[1-methyl-2-(4-nitrobenzyloxycarbonyloxy)ethyl]-4-methylthio-2-oxoazetidin-1-yl]but-2-enoate (425 mg) was chlorinated in the same manner described above.

Chromatography of the product on silica gel (10 g; eluting with 10 to 30% ethyl acetate in hexane) afforded 294 mg (70.9%) of methyl 2-[(3S,4R)-4-chloro-3-[1-methyl-1-(4-nitrobenzyloxycarbonyloxy)ethyl]-2-oxoazetidin-1-yl]-3-methylbut-2-enoate [(3S,4R)-isomer] and 80 mg of the (3S,4S)-isomer contaminated some impurities. Crystallization of the impure (3S,4S)-isomer from ether gave 35 mg of the pure (3S,4S)-isomer.

Spectral Data *the (3S,4R)-isomer:*

IR ($CH_2Cl$): 1775, 1740, 1720, 1520 and 1350 $cm^{-1}$.

NMR ($CDCl_3$) δ: 1.66 (s, 3H), 1.71 (s, 3H), 2.02 (s, 3H), 2.30 (s, 3H), 3.74 (s, 3H), 3.93 (d, J=2Hz, 1H), 5.20 (s, 3H), 5.91 (d, J=2Hz, 1H), 7.52 (d, J=8Hz, 2H), and 8.20 (d, J=8Hz, 2H).

*the (3S,4S)-isomer:*

IR ($CH_2Cl_2$): 1775, 1740, 1725, 1520, and 1350 $cm^{-1}$.

NMR ($CDCl_3$) δ: 1.78 (s, 3H), 1.81 (s, 3H), 2.07 (s, 3H), 2.29 (s, 3H), 3.74 (s, 3H), 4.00 (d, J=5Hz, 1H), 5.18 (s, 2H), 5.93 (d, J=5Hz, 1H), 7.46 (d, J=9Hz, 2H), and 8.16 (d, J=9Hz, 2H).

Preparation 11

To a solution of methyl 2-[(3S,4R)-3-[(1S)-1-(tertbutyldimethylsilyloxy)ethyl]-4-methylthio-2-oxoazetidin-1-yl]-3-methylbut-2-enoate (60 mg) in dichloromethane (2 ml) was added a solution of chlorine (24 mg) in carbon tetrachloride (0.2 ml) at −50°C. The solution was allowed to warm to −10°C during 30 minutes and cooled to −50°C. An additional solution of chlorine (12 mg) in carbon tetrachloride (0.1 ml) was added. The solution was allowed to warm to −5°C and evaporated. The residue was chromatographed on silica gel (1.5 g, eluting with 8—10% ethyl acetate in hexane) to give 21.0 mg (36.1%) of methyl 2-[(3S,4R)-3-[(1S)-1-(tertbutyldimethylsiloxy)ethyl]-4-chloro-2-oxoazetidin-1-yl]-3-methylbut-2-enoate.

NMR ($CDCl_3$) δ: 0.12 (s, 6H), 0.93 (s, 9H), 1.34 (d, J=6.5Hz, 3H), 2.01 (s, 3H), 2.29 (s, 3H), 3.56 (dd, J=1.5, 5Hz, 1H), 3.77 (s, 3H), 4.30 (dq, J=5, 6.5Hz, 1H), 5.80 (d, J=1.5Hz, 1H).

Preparation 12

1.55M Butyl lithium-hexane solution (1.66 ml) was added to a solution of N-isopropylcyclohexylamine (0.29 ml) in tetrahydrofuran (3 ml) at −70°C and the mixture was stirred for 20 minutes at −70C and allowed to warm up to 0°C. This mixture was added to a solution of methyl 3-methyl-2-[(4R)-2-oxo-4-allylazetidin-1-yl]but-2-enoate (100 mg) in tetrahydrofuran (3 ml) at −70°C, and the mixture was stirred for 1 hour at −70°C

and allowed to warm up to −30°C during 30 minutes. 1N Hydrochloric acid (3.6 ml) was added to the reaction mixture at −70°C, and the mixture was diluted with ethyl acetate (30 ml). The solution was washed with water and brine, dried over magnesium sulfate, and evaporated in vacuo. The residue was chromatographed on silica gel eluting with a mixture of hexane and ethyl acetate (5:1 — 2:1) to give methyl 3-methyl-2-[(3S,4R)-3-methyl-2-oxo-4-allylazetidin-1-yl]but-2-enoate (20 mg).

IR ($CH_2Cl_2$): 1740, 1715 $cm^{-1}$.

NMR ($CDCl_3$) δ: 1.23 (d, 3H, J=8Hz), 1.95 (s, 3H), 2.19 (s, 3H), 1.36 (t, 2H, J=7Hz), 3.36 (dq, 1H, J=6, 8Hz), 3.75 (s, 3H), 4.04 (dt, 1H, J=6, 7Hz), 4.9—5.2 (m, 2H), 5.4—5.9 (m, 2H);

and methyl 3-methyl-2-[(3S,4R)-3-methyl-2-oxo-4-allylazetidin-1-yl]but-3-enoate (28 mg).

IR ($CH_2Cl_2$): 1740 $cm^{-1}$.

NMR ($CDCl_3$) δ: 1.27 and 1.29 (a pair of d, 3H, J=8Hz), 1.83 (broad s, 3H), 2.1—2.9 (m, 2H), 2.85 (dq, 1H, J=2, 8Hz), 3.2—3.4 (m, 1H), 3.74 and 3.76 (a pair of s, 3H), 4.8—5.2 (m, 5H), 5.5—6.0 (m, 1H);

and a mixture of methyl 3-methyl-2-[(3R,4R)-3-methyl-2-oxo-4-allylazetidin-1-yl]but-2-enoate and methyl-3-methyl-2-[(3S,4R)-3-methyl-2-oxo-4-allylazetidin-1-yl]but-3-enoate (36 mg).

IR ($CH_2Cl_2$): 1740 $cm^{-1}$.

Preparation 13

$C_2H_5$ H H   $C_2H_5$ H H

COOCH$_3$   COOCH$_3$   COOCH$_3$

1.55M Butyl lithium-hexane solution (2.97 ml) was added to a solution of N-isopropylcyclohexylamine (0.76 ml) in tetrahydrofuran (6 ml) at −70°C and the mixture was stirred for 20 minutes at −70°C. This mixture was added to a solution of methyl 3-methyl-2-[(4R)-2-oxo-4-allylazetidin-1-yl]but-2-enoate (210 mg) in tetrahydrofuran (6 ml) during 3 minutes at −70°C, and the mixture was stirred for 1 hour at −70°C and for 30 minutes at −30°C. Ethyl iodide (0.37 ml) was then added to the reaction mixture at −70°C and the mixture was stirred for 1 hour at the same temperature and for 30 minutes at −30°C. 1N Hydrochloric acid (10 ml) was added to the reaction mixture at −70°C and the mixture was diluted with ethyl acetate (120 ml). The solution was washed with water, brine, aqueous sodium bicarbonate and brine, dried over magnesium sulfate and evaporated in vacuo. Triethylamine (0.16 ml) was added to a solution of the residue in dichloromethane (2 ml) at 0°C, and the mixture was stirred overnight at ambient temperature. The reaction mixture was diluted with ethyl acetate (20 ml) and the solution was washed with 0.1N hydrochloric acid, brine, aqueous sodium bicarbonate and brine dried over magnesium sulfate, and evaporated in vacuo.

The residue was chromatographed on silica gel (5 g) eluting with a mixture of hexane and ethyl acetate (10:1—2:1) to give 169 mg of a 2.3:1 mixture of methyl 3-methyl-2-[(3R,4R)-3-ethyl-2-oxo-4-allylazetidin-1-yl]but-2-enoate [(3R,4R)-isomer] and its (3S,4R)-isomer.

IR ($CH_2Cl_2$): 1740, 1720 $cm^{-1}$.

NMR ($CDCl_3$) δ: 1.04 and 1.12 (a pair of t, 3H, J=8Hz), 1.6—2.0 (m, 2H), 1.96 (S, 3H), 2.20 (s, 3H, 2.2—2.61 (m, 2H), 2.80 and 3.14 (a pair of dq, 1H, J=2.5, 7Hz and J=6, 7.5H (2.3:1), 3.70 and 4.04 (a pair of dt, 1H, J=2.5, 7Hz and J=6, 7Hz), 3.76 (s, 3H), 4.9—5.3 (m, 2H), 5.5—6.0 (m, 1H).

Preparation 14

$CH_3$ H H   $CH_3$ H H

COOCH$_3$   COOCH$_3$   COOCH$_3$

Methyl 3-methyl-2-[(3R,4R)-3-methyl-2-oxo-4-allylazetdin-1-yl]but-2-enoate [(3R,4R)-isomer] and its (3S,4R)-isomer were prepared in a similar manner to that of Preparation 13.

For the (3R,4R)-isomer:

IR ($CH_2Cl_2$: 1740, 1710 $cm^{-1}$.

NMR ($CDCl_3$) δ: 1.32 (d, 3H, J=7.5Hz), 1.93 (s, 1H) 1.19 (s, 1H), 2.36 (q, 2H, J=8Hz), 2.84 (dq, 1H, J=2.5, 7.5Hz), 3.56 (dt, 1H, J—2.5, 8Hz), 3.72 (s, 3H), 4.9—6.3 (m, 2H), 5.5—6.0 (m, 1H).

For the (3S,4R)-isomer:

IR ($CH_2Cl_2$): 1740, 1715 $cm^{-1}$.

NMR ($CDCl_3$) δ: 1.23 (d, 3H, J=8Hz), 1.95 (s, 3H), 2.19 (s, 3H), 1.36 (t, 2H, J=7Hz), 3.36 (dq, 1H, J=6, 8Hz), 3.75 (s, 3H), 4.04 (dt, 1H, J=6, 7Hz), 4.9—5.2 (m, 2H), 5.4—5.9 (m, 2H).

Preparation 15

1.55M Butyl lithium-hexane solution (2.82 ml) was added to a solution of N-isopropylcyclohexylamine (0.72 ml) in tetrahyrofuran (6 ml) at −70°C, and the mixture was stirred for 20 minutes at −70°C. This mixture was added to a solution of methyl 3-methyl-2-[(4R)-2-oxo-4-allylazetidin-1-yl]but-2-enoate (247 mg) in tetrahydrofuran (6 ml) at −70°C and the mixture was stirred for 1 hour at −70°C, and for 30 minutes at −30°C. Acetone (0.12 ml) was added to the reaction mixture at −70°C, and the mixture was stirred for 1 hour at −70°C and for 30 minutes at −30°C — −15°C. 3N Hydrochloric acid was added to the reaction mixture at −70°C and the mixture was diluted with ethyl acetate (200 ml). The solution was washed with water, brine, aqueous sodium bicarbonate and brine, dried over magnesium sulfate, and evaporated in vacuo. Triethylamine (0.15 ml) was added to a solution of the residue in dichloromethane (3 ml) at 0°C and the mixture was stirred overnight at ambient temperature. The reaction mixture was diluted with ethyl acetate (20 ml) and the solution was washed with 0.1N hydrochloric acid (15 ml) brine, aqueous sodium bicarbonate and brine, dried over magnesium sulfate, and evaporated in vacuo. The residue was chromatographed on silica gel (10 g) eluting with a mixture of benzene and acetone (10:1—2:1) to give methyl 3-methyl-2-[(3S,4R)-3-(1-hydroxy-1-methylethyl)-2-oxo-4-allylazetidin-1-yl]but-2-enoate (100 mg).

IR ($CH_2Cl_2$): 1740, 1715 $cm^{-1}$.

NMR ($CDCl_3$) δ: 1.31 (s, 3H), 1.38 (s, 3H), 1.96 (s, 3H), 2.18 (s, 3H), 2.2—2.5 (m, 3H), 2.90 (d, 1H, J=3Hz), 3.76 (s, 3H), 3,81 (dt, 1H; J=3, 7Hz), 4.9—5.2 (m, 2H), 5.5—6.0 (m, 1H);

and methyl 3-methyl-2-[(3R,4R)-3-(1-hydroxy-1-methylethyl)-2-oxo-4-allylazetidin-1-yl]but-2-enoate (50 mg).

NMR ($CDCl_3$) δ: 1.39 (s, 3H), 1.52 (s, 3H), 1.99 (s, 3H), 2.19 (s, 3H), 2.21 (broad s, 1H), 2.74 (t, 2H, J=7Hz), 3.30 (d, 1H, J=6Hz), 3.74 (s, 3H), 4.12 (dt, 1H, J=6, 7Hz), 4.9—5.2 (m, 2H), 5.5—5.9 (m, 1H).

Preparation 16

Methyl 3-methyl-2-[(3S,4R)-3-[(1RS)-1-hydroxyethyl-2-oxo-4-allylazetidin-1-yl]but-2-enoate [(3S,4R)-isomer] and methyl 3-methyl-2-[(3R,4R)-3-[(1RS)-1-hydroxyethyl-2-oxo-4-allylazetidin-1-yl]but-2-enoate [(3R,4R)-isomer] were prepared in a similar manner to that of Preparation 15.

For the (3S,4R)-isomer:

IR ($CH_2Cl_2$): 1740, 1720 $cm^{-1}$.

NMR ($CDCl_3$) δ: 1.32 (d, 3H, J=7Hz), 1.96 (s, 3H), 2.20 (s, 3H), 2.3—2.5 (m, 2H), 2.6 (broad s, 1H), 2.90 (dd, 1H, J=7, 2.5Hz), 3.76 (s, 3H), 3.82 (dt, 1H, J=2.5, 6Hz), 4.0—4.3 (m, 1H), 5.0—5.2 (m, 2H), 5.5—6.0 (m, 1H).

For the (3R,4R)-isomer:

IR ($CH_2Cl_2$): 1740, 1720 $cm^{-1}$.

NMR ($CDCl_3$) δ: 1.22 (d, 3H, J=5.5Hz), 2.00 (s, 3H), 2.20 (s, 3H), 2.2—2.8 (m, 3H), 3.24 (dq, 1H, J=4.5, 5.5Hz), 3.75 (s, 3H), 3.9—4.3 (m, 2H), 4.9—5.2 (m, 2H), 5.5—6.0 (m, 1H).

Preparation 17

1.55M Butyl lithium-hexane solution (5.63 ml) was added to a solution of N-isopropylcyclohexylamine (1.44 ml) in tetrahydrofuran (10 ml) at −70°C. The solution was stirred for 20 minutes at −70°C and allowed to warm up to −20°C during 5 minutes. This mixture was added to a solution of methyl 3-methyl-2-[(4R)-2-oxo-4-allylazetidin-1-yl]but-2-enoate (650 mg) in tetrahydrofuran (10 ml) during 15 minutes at −70°C, and the mixture was stirred for 1 hour at −70°C and for 30 minutes at −30°C. A solution of acetaldehyde (187 mg) in tetrahydrofuran (4.4 ml) was added to the reaction mixture at −70°C. The mixture was stirred for 1 hour at −70°C and for 30 minutes at −30°C. 1N Hydrochloric acid (20 ml) was added to the reaction mixture at −70°C and the mixture was diluted with ethyl acetate (250 ml). The solution was washed with brine, aqueous sodium bicarbonate and brine, dried over magnesium sulfate and evaporated in vacuo.

The residue was chromatographed on silica gel (20 g) eluting with a mixture of benzene and acetone (10:1—3:1) to give methyl 3-methyl-2-{(3S,4R)-3-[(1RS)-1-hydroxyethyl]-2-oxo-4-allylazetidin-1-yl}but-2-enoate (278 mg) [(3S,4R)-isomer] and its other isomers (257 mg).

For the (3S,4R)-isomer:

IR ($CH_2Cl_2$): 1740, 1720 $cm^{-1}$.

NMR ($CDCl_3$) δ: 1.32 (d, 3H, J=7Hz), 1.96 (s, 3H), 2.20 (s, 3H), 2.3—2.5 (m, 2H), 2.6 (broad s, 1H), 2.90 (dd, 1H, J=7, 2.5Hz), 3.76 (s, 3H), 3.82 (dt, 1H, J=2.5, 6Hz), 4.0—4.3 (m, 1H), 5.0—5.2 (m, 2H), 5.5—6.0 (m, 1H).

Preparation 18

4-Dimethylaminopyridine (79 mg) and 4-nitrobenzyl chloroformate (90 mg) were added to a solution of a 1:3 mixture of methyl 3-methyl-2-[(3S,4R)-3-[(1RS)-1-hydroxyethyl]-2-oxo-allylazetidin-1-yl]but-2-enoate (86 mg) in dichloromethane (4 ml) at 0°C and the mixture was stirred for three hours at 0°C. The reaction mixture was diluted with ethyl acetate (50 ml), and washed in turn with 0.1N hydrochloric acid (20 ml), brine, aqueous sodium bicarbonate and brine. The organic layer was dried over magnesium sulfate and evaporated in vacuo. The residue was chromatographed on silica gel (2 g) eluting with hexane and ethyl acetate (5:1—2:1) to give a 1:3 mixture of methyl 3-methyl-2-{(3S,4R)-3-[(1Rs)-1-(4-nitrobenzyloxy-carbonyloxy)ethyl]-2-oxo-4-allylazetidin-1-yl}but-2-enoate (122 mg) as an oil.

IR ($CH_2Cl_2$): 1745, 1715, 1520 $cm^{-1}$.

NMR ($CDCl_3$) δ: 1.48 (d, 3H, J=7Hz), 1.96 (s, 3H), 1.20 (s, 3H), 2.2—2.6 (m, 2H), 3.03 and 3.18 (dd, J=7.5, 2.5Hz and dd, J=5, 2.5Hz, 1H (1:3)), 3.75 (s, 3H), 3.8—4.0 (m, 1H), 5.0—5.3 (m, 2H), 5.27 (s, 2H), 5.5—5.9 (m, 1H), 7.89 ($A_2B_2$, 4H, J=8.5Hz).

Preparation 19

A 1:3 mixture of methyl 3-methyl-2-[(3S,4R)-3-[(1RS)-1-hydroxyethyl]-2-oxo-4-allylazetidin-1-yl]but-2-enoate (55 mg) was dissolved in tetrahydrofuran (2 ml) and triphenylphosphine (135 mg) and formic acid (19.4 μl) were added to the mixture. This mixture was cooled to 0°C and a solution of diethyl azodicarboxylate (81 μl) in tetrahydrofuran (1 ml) was added. After stirring at the same temperature overnight, the mixture was diluted with ethyl acetate (30 ml), washed with aqueous sodium bicarbonate and brine, dried over magnesium sulfate and evaporated.

The residue was chromatographed on silica gel (5 g) eluting with hexane and ethyl acetate (10:1—2:1) to give 25 mg of methyl 3-methyl-2-[(3S,4R)-3-[(1R)-1-formyloxyethyl]-2-oxo-4-allylazetidin-1-yl]-but-2-enoate.

IR ($CH_2Cl_2$): 1745, 1720 $cm^{-1}$.

NMR ($CDCl_3$) δ: 1.42 (d, 3H, J=6Hz), 1.96 (s, 3H), 2.20 (s, 3H), 2.38 (q, 2H, J=6Hz), 3.00 (dd, 1H, J=7.5, 2.5Hz), 3.76 (s, 3H), 3.7—4.0 (m, 1H), 4.9—5.2 (m, 2H), 5.3—5.9 (m, 2H), 8.00 (s, 1H).

Preparation 20

Methyl 3-methyl-2-[(3S,4R)-3-[(1R)-1-formyloxyethyl]-2-oxo-4-allylazetidin-1-yl]but-2-enoate (23 mg) was dissolved in methanol (1 ml) and cooled to 0°C. To this solution was added a solution of sodium methoxide in methanol (0.49M, 0.16 ml) and the mixture was stirred for 1 hour at the same temperature. An additional 0.08 ml of the sodium methoxide solution was added and the mixture was stirred for 30 minutes at the same temperature. Acetic acid (2 drops) was then added and the mixture was evaporated. The residue was dissolved in ethyl acetate, washed with aqueous sodium bicarbonate and brine, dried over magnesium sulfate and evaporated. The residue was purified by preparative thin layer chromatography (silica gel) developing with methylene chloride and ethyl acetate (2:1) to give methyl 3-methyl-2-[(3S,4R)-3-[(1R)-1-hydroxyethyl]-2-oxo-4-allylazetidin-1-yl]but-2-enoate (20 mg).

IR ($CH_2Cl_2$): 3550, 1740, 1710 $cm^{-1}$.

NMR ($CDCl_3$) δ: 1.32 (d, 3H, J=7Hz), 1.96 (s, 3H), 2.20 (s, 3H), 2.3—2.6 (m, 2H), 2.90 (dd, 1H, J=7, 2.5Hz), 3.76 (s, 3H), 4.00 (dt, 1H, J=2.5, 6.5Hz), 4.1—4.4 (m, 1H), 5.0—5.3 (m, 2H), 5.5—6.0 (m, 1H).

Preparation 21

Methyl 3-methyl-2-[(3S,4R)-3-[(1R)-1-(4-nitrobenzyloxycarbonyloxy)ethyl]-2-oxo-4-allyl-azetidin-1-yl]but-2-enoate was prepared in a similar manner to that of Preparation 18.

IR ($CH_2Cl_2$): 1745, 1715, 1520 $cm^{-1}$.

NMR ($CDCl_3$) δ: 1.45 (d, 3H, J=6Hz), 1.94 (s, 3H), 2.19 (s, 3H), 2.37 (q, 2H, J=6Hz), 3.01 (dd, 1H, J=7.5, 2.5Hz), 3.76 (s, 3H), 3.92 (dt, 1H, J=2.5 6Hz), 4.9—5.3 (m, 3H), 5.4—5.8 (m, 1H), 7.83 ($A_2B_2$, 4H, J=9Hz).

Preparation 22

Silver tetrafluoroborate (205 mg) was added to a stirred solution of 4-nitrobenzyl 3-(trimethylsilylmethyl)but-3-enoate (422 mg) and a 4:1 mixture of methyl 2-[(3S,4R)-4-chloro-3-[1-methyl-1-(4-nitrobenzyloxycarbonyloxy)ethyl]-2-oxoazetidin-1-yl]-3-methylbut-2-enoate and its (3S,4S)-isomer (305 mg) in dichloromethane (3 ml) at −78°C under a nitrogen atmosphere. The mixture was gradually allowed to warm to 0°C during 40 minutes and kept at the same temperature for one hour. The resulting mixture was diluted with dichloromethane (1 ml) and stirred at 0°C for 30 minutes and at room temperature for 30 minutes. The mixture was diluted with ethyl acetate (10 ml) and a saturated aqueous solution of sodium chloride (4 ml) was added. The mixture was brought to pH 7 with a saturated aqueous solution of sodium bicarbonate and filtered through diatomaceous earth. The filtrate was diluted with ethyl acetate (30 ml). The organic layer was separated, washed with brine, dried over magnesium sulfate, and evaporated. The residue was chromatographed on silica gel (12 g, eluting with 1 to 5% acetone in dichloromethane) to give 120 mg of the desired product contaminated with some impurities. Further purification on a TLC plate (20 cm × 20 cm × 2 mm, 1:1 hexane-ethyl acetate) afforded 100 mg (22.8%) of methyl 3-methyl-2-[(3S,4R)-3-[1-methyl-1-(4-nitrobenzyloxycarbonyloxy)ethyl]-4-[2-(4-nitrobenzoyloxycarbonyl-methyl)allyl]-2-oxoazetidin-1-yl]but-2-enoate: IR ($CH_2Cl_2$): 1740, 1720 (sh), 1520, and 1350 $cm^{-1}$; NMR ($CDCl_3$) δ: 1.65 (s, 3H), 1.69 (s, 3H), 1.98 (s, 3H), 2.19 (s, 3H), 2.48 (brd, J=7Hz, 2H), 3.13 (s, 2H), 3.44 (d, J=2.5Hz, 1H), 3.76 (s, 3H), 4.24 (dt, J=2.5 and 7Hz, 1H), 5.01 (m, 2H), 5.19 (s, 2H), 5.22 (s, 2H), 7.52 (d, J=9Hz, 2H), 7.56 (d, J=9Hz, 2H), and 8.22 (d, J=9Hz, 4H).

Preparation 23

In a manner similar to that of Preparation 22, 258 mg of methyl 2-[(3S,4R)-4-chloro-3-[1-methyl-1-(4-nitrobenzyloxycarbonyloxy)ethyl]-2-oxoazetidin-1-yl]-3-methylbut-2-enoate afforded 70.0 mg (18.9%) of methyl 3-methyl-2-[(3S,4R)-3-[1-methyl-1-(4-nitrobenzyloxycarbonyloxy)ethyl]-4-[2-(4-nitrobenzyloxycarbonylmethyl)allyl]-2-oxoazetidine-1-yl]but-2-enoate.

Preparation 24

A stream of ozone was bubbled into a solution of methyl 3-methyl-2-[(3S,4R)-3-[1-methyl-1-(4-nitrobenzyloxycarbonyloxy)ethyl]-4-[2-(4-nitrobenzyloxycarbonylmethyl)allyl]-2-oxoazetidin-1-yl]but-2-enoate (163 mg) in ethyl acetate (10 ml) at −78°C until a permanent blue color developed. After three minutes the excess ozone was removed by bubbling nitrogen. The solution was warmed and poured into a mixture of ethyl acetate (10 ml) and a dilute aqueous solution of sodium bisulfite (900 mg). The mixture was shaken and the organic layer was separated. The solution was washed with brine, dried over magnesium sulfate and evaporated to leave 158 mg of an amorphous solid. This residue was dissolved in dichloromethane (2.5 ml) and cooled to 0°C. Dimethyl sulfide (0.7 ml) and methanol (7.5 ml) were added. The solution was stirred at 0°C for 8 hours and left at ambient temperature for 15 hours under a nitrogen atmosphere. The mixture was then heated to 55°C for 26 hours and evaporated. The residue was chromatographed on a TLC plate (20 cm × 20 cm ×2 mm, 10% acetone in dichloromethane) to give 79 mg (58.3%) of 4-nitrobenzyl 4-[(2R,3S)-3-[1-methyl-1-(4-nitrobenzyloxycarbonyloxy)ethyl]-4-oxoazetidin-2-yl]-3-oxobutanoate: IR ($CH_2Cl_2$): 3370, 1760, 1740, 1710, 1520, and 1345 $cm^{-1}$; NMR ($CDCl_3$) δ: 1.57 (s, 3H), 1.62 (s, 3H), 2.4—3.2 (m, small signals), 2.9 (m, 1.5H), 3.34 (d, J=3Hz, 1H), 3.56 (s, 2H), 3.96 (m, 1H), 5.15 (s, 2H), 5.24 (s, 2H), 6.42 (brs, 1H), 7.50 (d, J=9Hz, 4H), and 8.16 (d, J=9Hz, 4H). (The product may exist as a equilibrium mixture of the keto- and enol-form).

Preparation 25

To a solution of (3R,4R)-4-(2-oxoethyl)-3-phthalimidoazetidin-2-one (52 mg) in a mixture of methanol (3 ml) and dichloromethane (1 ml) was added a solution of sodium borohydride (8 mg) in methanol (2 ml) at 0°C during one hour. The reaction was quenched by addition of acetic acid (two drops). After evaporation in vacuo, the residue was chromatographed on silica gel (1.6 g; eluting with 3 to 5% methanol in dichloromethane) to give 32 mg of crude crystals. Crystallization from a mixture of methanol and ether afforded 27 mg (51.5%) of (3R,4R)-4-(2-hydroxyethyl)-3-phthalimidoazetidin-2-one.

IR (Nujol): 3350 (br), 3250, 1750, and 1705 $cm^{-1}$.

NMR (DMSO-$d_6$) δ: 1.79 (q, J=6Hz, 2H), 3.46 (m, 2H), 3.97 (dt, J=2.5 and 6Hz, 1H), 4.47 (brs, 1H), 4.89 (d, J=2.5Hz, 1H), 7.86 (s, 4H), and 8.42 (brs, 1H).

Preparation 26

Boron trifluoride etherate (5 μl) was added to a suspension of (3R,4R)-4-(2-hydroxyethyl)-3-phthalimidoazetidin-2-one (126 mg) and 2,2-dimethoxypropane (74 μl) in dichloromethane (3 ml) at ambient temperature. The mixture was stirred for two hours. Additional dimethoxypropane (50 μl) was added and the mixture was stirred for an additional 1.5 hours. The resulting solution was poured into a saturated aqueous solution of sodium chloride and sodium bicarbonate and extracted twice with dichloromethane. The combined extracts were dried over magnesium sulfate and evaporated to give a crystalline solid. Chromatography on silica gel (4 g; eluting with 3% acetone in dichloromethane) and

washing the crystals with ether afforded 100 mg of (6R,7R)-2,2-dimethyl-7-phthalimido-1-aza-3-oxabicyclo[4.2.0]-octan-8-one:

IR ($CH_2Cl_2$): 1770 and 1720 $cm^{-1}$;

NMR ($CDCl_3$) δ: 1.56 (s, 3H), 1.81 (s, 3H), 1.7—2.0 (m, 2H), 3.7—4.1 (m, 3H), 5.01 (d, J=2Hz, 1H), and 7.5—7.9 (m, 4H).

Preparation 27

N,N-Dimethoylaminopropylamine (91 μl) was added to a solution of (6R,7R)-2,2-dimethyl-7-phthalimido-1-aza-3-oxabicyclo[4.2.0]octan-8-one (80 mg) in a mixture of methanol (1.4 ml) and dichloromethane (0.7 ml) at 0°C under a nitrogen atmosphere. The solution was stirred at the same temperature for two hours and left at ambient temperature for 24 hours. The mixture was evaporated and the residue was chromatographed on silica gel (1.5 g; eluting with 3 to 5% methanol in dichloromethane) to give 27 mg (59.5%) of (6R,7R)-7-amino-2,2-dimethyl-1-aza-3-oxabicyclo[4.2.0]octan-8-one:

IR ($CH_2Cl_2$): 3100—3600 (broad) and 1735 $cm^{-1}$.

NMR ($CDCl_3$) δ: 1.41 (s, 3H), 1.72 (s, 3H), 1.5—2.1 (m, 4H), 3.34 (ddd, J=2, 5 and 10Hz, 1H), and 3.7—3.9 (m, 3H).

Preparation 28

To a solution of methyl 2-[(3R,4R)-3-isocyano-2-oxo-4-allylazetidin-1-yl]-3-methylbut-2-enoate (106.9 mg) in tetrahydrofuran (2.1 ml) was added dropwise a solution of n-butyl lithium (0.375 ml of 1.38M solution in hexane) at −78°C. After stirring for 15 minutes at −78°C, the reaction mixture was added with a solution of acetone (0.0385 ml) in tetrahydrofuran (0.3465 ml) at −78°C. After stirring for 25 minutes at −78°C, the reaction mixture was added with acetic acid (0.0493 ml) at −78°C, diluted with ethyl acetate (30 ml), and washed with chilled brine (10 ml), aqueous sodium bicarbonate (10 ml), brine (10 ml), and saturated aqueous sodium chloride (10 ml). After drying over magnesium sulfate, the ethyl acetate extract was filtered and evaporated in vacuo. The residual oil was chromatographed on silica gel (1.2 g) eluting with a mixture of ethyl acetate and hexane (1:5—1:3) to give methyl 2-[(3R,4R)-3-(1-hydroxy-1-methylethyl)-3-isocyano-2-oxo-4-allylazetidin-1-yl]-3-methylbut-2-enoate (96.5 mg) as an oil.

IR ($CH_2Cl_2$): 2115, 1770, 1720 $cm^{-1}$.

NMR ($CDCl_3$) δ: 1.40 (s, 3H), 1.53 (s, 3H), 1.97 (s, 3H), 2.23 (s, 3H), 2.39 (s, 1H), 2.53 (5, 2H, J=7Hz), 3.76 (s, 3H), 4.24 (t, 1H, J=7Hz), 5.0—5.3 (m, 2H), 5.5—5.9 (m, 1H).

Preparation 29

Tributyltin hydride (0.707 ml) was added to a mixture of methyl 2-[(3R,4R)-3-(1-hydroxy-1-methylethyl)-3-isocyano-2-oxo-4-allylazetidin-1-yl]-3-methylbut-2-enoate (630 mg) and azobisisobutyronitrile (34 mg) in benzene (6 ml) at room temperature, and the mixture was refluxed for 15 minutes. The reaction mixture was cooled and chromatographed on silica gel (20 g) eluting with a mixture of hexane and ethyl acetate (3:1—1:1) to give a 3:1 mixture (559 mg) of methyl 2-[(3S,4R)-3-(1-hydroxy-1-methylethyl)-2-oxo-4-allylazetidin-1-yl]-3-methylbut-2-enoate and its (3R,4R)-isomer.

IR ($CH_2Cl_2$): 1740, 1715.

NMR ($CDCl_3$) δ: 1.31 (s, 3H × 3/4), 1.38 (s, 3H), 1.52 (s, 3H × 1/4), 1.96 (s, 3H × 3/4), 1.99 (s, 3H × 1/4), 2.18 (s, 3H), 2.1—2.8 (m, 2H), 2.90 (d, 1H × 3/4, J=3Hz), 3.30 (d, 1H × 1/4, J=6Hz), 3.74 (s, 3H × 1/4), 3.76 (s, 3H × 3/4), 3.8—4.2 (m, 1H), 4.9—5.2 (m, 2H), 5.5—5.9 (m, 1H).

Preparation 30

Potassium carbonate (113 mg) was added to a solution of methyl 2-[(3R,4R)-3-isocyano-2-oxo-4-allylazetidin-1-yl]-3-methylbut-2-enoate (203 mg) in acetone (2 ml) at 0°C, and the mixture was stirred for 20 hours at ambient temperature.

Potassium carbonate (210 mg) and acetone (1.5 ml) were added to the reaction mixture at ambient temperature, and the mixture was stirred for 18 hours at ambient temperature. The reaction mixture was diluted with dichloromethane (3 ml) and filtered. The filtrate was evaporated in vacuo, and the residue was chromatographed on silica gel (2 g) eluting with a mixture of hexane and ethyl acetate (3:1) to give methyl 2 - [(3R,4R) - 3 - allyl - 5,5 - dimethyl - 1 - oxo - 2,8 - diaza - 6 - oxaspiro[3,4]oct - 5 - en - 2 - yl] - 3 - methylbut - 2 - enoate (120 mg).

IR ($CH_2Cl_2$): 1755, 1715, 1610 $cm^{-1}$.

NMR ($CDCl_3$) δ: 1.40 (s, 3H), 1.64 (s, 3H), 2.01 (s, 3H), 2.23 (s, 3H), 2.3—2.6 (m, 2H), 3.77 (s, 3H), 4.11 (t, 1H, J=7Hz), 4.9—5.2 (m, 2H), 5.4—5.8 (m, 1H), 6.94 (s, 1H).

Preparation 31

A solution of 1,8-diazabicyclo[5.4.0]undec-7-ene (17 μl) in methylene chloride (0.1 ml) was added to a solution of methyl 2 - [(3,4R) - 3 - (1 - hydroxy - 1 - methylethyl) - 3 - isocyano - 2 - oxo - 4 - allylazetidin - 1 - yl] - 3 - methylbut - 2 - enoate (17 mg) in methylene chloride (1 ml) and acetone (1 ml) at 0°C, and the mixture was stirred for 20 hours at ambient temperature. Acetic acid (2 drops) was added to

the reaction mixture at 0°C, and the mixture was evaporated in vacuo. The residue was dissolved in ethyl acetae (20 ml), and the solution was washed with 10% aqueous phosphoric acid, brine, phosphate buffer (pH 6.9), aqueous sodium bicarbonate and brine, dried over magnesium sulfate and evaporated in vacuo to give methyl 2 - [(3R,4R) - 3 - allyl - 5,5 - dimethyl - 1 - oxo - 2,8 - diaza - 6 - oxaspiro[3,4]oct - 7 - en - 2 - yl] - 3 - methylbut - 2 - enoate (19 mg) as crude crystals.

Preparation 32

A solution of n-butyl lithium (0.42 ml of 1.55M solution in hexane) was added to a solution of methyl 2-[(3S,4R)-3-(1-hydroxy-1-methylethyl)-2-oxo-4-allylazetidin-1-yl]-3-methylbut-2-enoate (150 mg) in tetrahydrofuran (3 ml) during 3 minutes at −70°C and the mixture was stirred for 5 minutes at −70°C. A solution of p-nitrobenzyl chloroformate (161 mg) in tetrahydrofuran (1.6 ml) was added to the mixture during 5 minutes at −70°C and the mixture was stirred for 15 minutes at −70°C. The mixture was allowed to warm to 0°C during 30 minutes and stirred for 2 hours at 0°C. Acetic acid (0.037 ml) was added to the mixture at 0°C and the mixture was evaporated in vacuo. The residue was dissolved in ethyl acetate (50 ml), and the solution was washed with water (10 ml), 10% phosphoric acid (10 ml), phosphate buffer (pH 7.0) (10 ml × 2), aqueous sodium bicarbonate and brine. The organic layer was dried over magnesium sulfate and evaporated in vacuo. The residue was chromatographed on silica gel (10 g) eluting with a mixture of hexane and ethyl acetate (5:1—1:1) to give methyl 3 - methyl - 2 - [(3S,4R) - 3 - [1 - methyl - 1 - (4 - nitrobenzyloxycarbonyloxy)ethyl] - 2 - oxo - 4 - allylazetidin - 1 - yl]but - 2 - enoate (135 mg) asn an oil.

IR ($CH_2Cl_2$): 1740, 1710, 1520, 1345 $cm^{-1}$.

NMR ($CDCl_3$) δ: 1.66 (s, 3H), 1.69 (s, 3H), 1.97 (s, 3H), 2.19 (s, 3H), 2.2—2.5 (m, 2H), 3.40 (d, 1H, J=2.5Hz), 3.71 (s, 3H), 4.03 (dt, 1H, J=6.5Hz), 4.9—5.2 (m, 2H), 5.19 (s, 2H), 5.5—5.9 (m, 1H), 7.90 ($A_2B_3$, 4H, J=9Hz).

Preparation 33

A solution of n-butyl lithium (0.46 ml of 1.38M solution in hexane) was added to a solution of methyl 2 - [(3R,4R) - 3 - (1 - hydroxy - 1 - methylethyl) - 2 - oxo - 4 - allylazetidin - 1 - yl] - 3 - methylbut - 2 - enoate (150 mg) in tetrahydrofuran (3 ml) at −70°C and the mixture was stirred for 15 minutes at −70°C. A solution of p-nitrobenzyl chloroformate (161 mg) in tetrahydrofuran (3.2 ml) was added to the reaction mixture at −70°C and the mixture was stirred for 10 minues at −70°C. The mixture was allowed to warm to 0°C during 30 minutes and stirred at 0°C for 1.5 hours. Acetic acid (0.043 ml) was added to the mixture at 0°C and the mixture was evaporated in vacuo. The residue was dissolved in ethyl acetate (30 ml) and the solution was washed with brine, aqueous sodium bicarbonate, and brine. The organic layer was dried over magnesium sulfate and evaporated in vacuo. The residue was chromatographed on silica gel (10 g) eluting with a mixture of hexane and ethyl acetate (10:1—3:1) to give methyl 3 - methyl - 2 - [(3R,4R) - 3 - [1 - methyl - 1 - (4 - nitrobenzyloxycarbonyloxy)ethyl] - 2 - oxo - 4 - allylazetidin - 1 - yl]but - 2 - enoate (123 mg) as an oil.

IR ($CH_2Cl_2$): 1745, 1720, 1520, 1350 $cm^{-1}$.

NMR ($CDCl_3$) δ: 1.70 (s, 3H), 1.81 (s, 3H), 2.01 (s, 3H), 2.10 (s, 3H), 2.64 (t, 2H, J=7Hz), 3.59 (d, 1H, J=5Hz), 3.74 (s, 3H), 4.17 (dt, 1H, J=5, 7Hz), 4.9—5.3 (m, 2H), 5.21 (s, 2H), 5.5—5.9 (m, 1H), 7.87 ($A_2B_2$, 4H, J=9Hz).

Preparation 34

Ozone was bubbled into a solution of methyl 3-methyl-2-[(3S,4R)-3-[1-methyl-1-(4-nitrobenzyloxycarbonyloxy)ethyl]-2-oxo-4-allylazetidin-1-yl]but-2-enoate (50 mg) in ethyl acetate (3 ml) at −70°C until a blue color appeared. After stirring for 15 minutes at the same temperature, the mixture was purged with nitrogen. The reaction mixture was diluted with ethyl acetate (20 ml) and the solution was washed with an aqueous solution of sodium bisulfite and sodium sulfite (2:1), and brine. The organic layer was dried over magnesium sulfate and evaporated in vacuo.

The residue was dissolved in methylene chloride (3 ml) and m-chloroperbenzoic acid (48 mg) was added to the solution at 0°C. The mixture was stirred for 24 hours at ambient temperature. Additional m-chloroperbenzoic acid (48 mg) was added, and the mixture was stirred for 24 hours at ambient temperature. The mixture was evaporated in vacuo. The residue was dissolved in methanol (3 ml) and the mixture was heated for 24 hours at 50°C. The mixture was evaporated in vacuo and the residue was chromatographed on silica gel (4 g) eluting with a mixture of methylene chloride and methanol (20:1—5:1) to give {(2R,3S) - 3 - [1 - methyl - 1 - (4 - nitrobenzyloxycarbonyloxy)ethyl] - 4 - oxoazetidin - 2 - yl}acetic acid (43 mg) as an oil.

IR ($CH_2Cl_2$): 1740, 1520, 1350 $cm^{-1}$.

NMR ($CDCl_3$) δ: 1.60 (broad s, 6H), 2.3—2.8 (m, 2H), 3.36 (broad s, 1H), 3.7—4.0 (m, 1H), 5.16 (s, 2H), 7.1—7.3 (broad, 1H), 7.84 ($A_2B_2$, 4H, J=8Hz), 9.1—9.6 (broad, 1H).

Preparation 35

OCOOPNB
$(CH_3)_2C$
H
H
COOH
NH
O
OCOOPNB
$(CH_3)_2C$
H
H
$COOCH_3$
NH
O

A solution of diazomethane in ether (1 ml) was added to a solution of [(2R,3S) - 3 - (1 - methyl - 1 - (4 - nitrobenzyloxycarbonyloxy)ethyl] - 4 - oxoazetidin - 2 - yl] - acetic acid (29 mg) in a mixture of methylene chloride (1 ml) and methanol (1 ml) at 0°C and the mixture was stirred for 15 minutes at 0°C. Acetic acid (1 drop) was added to the mixture at 0°C and the mixture was evaporated in vacuo. The residue was chromatographed on silica gel (1 g) eluting with a mixture of methylene chloride and ethyl acetate (10:1) to give methyl [(2R,3S) - 3 - [1 - methyl - 1 - (4 - nitrobenzyloxycarbonyloxy)ethyl] - 4 - oxoazetidin - 2 - yl]acetate (31 mg).

IR ($CH_2Cl_2$): 1760, 1740, 1520, 1550 $cm^{-1}$.

NMR ($CDCl_3$) δ: 1.61 (s, 3H), 1.65 (s, 3H), 2.4—2.6 (m, 2H), 3.36 (d, 1H, J=3Hz), 3.69 (s, 3H), 3.8—4.0 (m, 1H), 5.17 (s, 2H), 6.40 (s, 1H), 7.87 ($A_2B_2$, 4H, J=9Hz).

Preparation 36

Ozone was bubbled into a solution of methyl 2 - [(3S,4R) - 3 - (1 - hydroxy - 1 - methylethyl) - 2 - oxo - 4 - allylazetidin - 1 - yl] - 3 - methylbut - 2 - enoate (159 mg) in ethyl acetate (5 ml) at −70°C until a blue color appeared. After stirring for 15 minutes at same temperature, the mixture was purged with nitrogen. The mixture was diluted with ethyl acetate (30 ml) and the solution was washed with an aqueous solution of sodium bisulfite and sodium sulfite (2:1). The aqueous layer was extracted with ethyl acetate (10 ml). The combined organic layers were washed with brine, dried over magnesium sulfate, and evaporated in vacuo.

The residue was dissolved in methylene chloride (3 ml) and m-chloroperbenzoic acid (244 mg) was added at ambient temperature. The mixture was refluxed for 24 hours and evaporated in vacuo. The residue was dissolved in methanol (3 ml) and m-chloroperbenzoic acid (244 mg) was added. The mixture was heated at 50°C for 20 hours and evaporated in vacuo. The residue was chromatographed on silica gel (3 g) eluting with a mixture of methylene chloride and methanol (9:1—2:1) to give [2R,3S) - 3 - (1 - hydroxy - 1 - methylethyl) - 4 - oxoazetidin - 2 - yl]acetic acid (25 mg).

IR ($CH_2Cl_2$): 1750, 1730, 1710 $cm^{-1}$.

NMR ($CD_3OD$) δ: 1.28 (s, 3H), 1.32 (s, 3H), 2.5—2.7 (m, 2H), 2.90 (d, 1H, J=2.5Hz), 3.8—4.0 (m, 1H).

Preparation 37

OH
(CH3)2C
H
H
COOH
NH
O
OH
(CH3)2C
H
H
COOCH3
NH
O

A solution of diazomethane in ether (2 ml) was added to a solution of [(2R,3S) - 3 - (1 - hydroxy - 1 - methylethyl) - 4 - oxoazetidin - 2 - yl]acetic acid (23 mg) in methanol (3 ml) at 0°C and the mixture was stirred for an hour at 0°C. Acetic acid (2 drops) was added to the mixture at 0°C and the mixture was evaporated in vacuo to give methyl [(2R,3S) - 3 - (1 - hydroxy - 1 - methylethyl) - 4 - oxoazetidin - 2 - yl]acetate (25 mg).

IR ($CH_2Cl_2$): 1760, 1735 $cm^{-1}$.

NMR ($CD_3OD$) δ: 1.28 (s, 3H), 1.34 (s, 3H), 2.6—2.8 (m, 2H), 2.89 (d, 1H, J=2.5Hz), 3.70 (s, 3H), 3.8—4.0 (m, 1H).

Preparation 38

Ozone was bubbled into a solution of methyl 3 - methyl - 2 - [(3R,4R) - 3 - [1 - methyl - 1 - (4 - nitrobenzyloxycarbonyloxy)ethyl] - 2 - oxo - 4 - allylazetidin - 1 - yl]but - 2 - enoate (120 mg) in ethyl acetate (5 ml) at −70°C until a blue color appeared. After stirring for 15 minutes at the same temperature, the mixture was purged with nitrogen. The reaction mixture was diluted with ethyl acetate (30 ml) and the solution was washed with an aqueous solution of sodium bisulfite and sodium sulfite (2:1), and brine. The

organic layer was dried over magnesium sulfate and evaporated in vacuo. The residue was dissolved in methylene chloride (3 ml) and m-chloroperbenzoic acid (112 mg) was added at 0°C. The mixture was stirred for 24 hours at ambient temperature, and evaporated in vacuo. The residue was dissolved in methanol (5 ml) and m-chloroperbenzoic acid (112 mg) was added. The mixture was heated at 50°C for 2 days and evaporated in vacuo. The residue was chromatographed on silica gel (6 g) eluting with a mixture of methylene chloride and methanol (20:1—5:1) to give [(2R,3R) - 3 - [1 - methyl - 1 - [4 - nitrobenzyloxycarbonyloxy)ethyl] - 4 - oxoazetidin - 2 - yl]acetic acid (43 mg) as an oil.

IR ($CH_2Cl_2$): 1735, 1520, 1350 $cm^{-1}$.

NMR ($CDCl_3$) δ: 1.60 (s, 3H), 1.72 (s, 3H), 2.5—3.2 (m, 2H), 3.4—3.7 (m, 1H), 3.9—4.2 (m, 1H), 5.18 (s, 2H), 7.82 ($A_2B_2$, 4H, J=9Hz), 8.4—9.1 (broad, 1H).

Preparation 39

Ozone was bubbled into a solution of methyl 2 - [(3R,4R) - 3 - (1 - hydroxy - 1 - methylethyl) - 2 - oxo - 4 - allylazetidin - 1 - yl] - 3 - methylbut - 2 - enoate (240 mg) in ethyl acetate (5 ml) at −70°C until a blue color appeared. After stirring for 15 minutes at −70°C. The mixture was purged with nitrogen. The mixture was diluted with ethyl acetate (30 ml) and the solution was washed with aqueous saturated sodium bisulfite. The aqueous layer was extracted with chloroform (10 ml). The combined organic layer was dried over magnesium sulfate and evaporated in vacuo. The residue was dissolved in methylene chloride (3 ml) and m-chloroperbenzoic acid (368 mg) was added at ambient temperature. After stirring for 20 hours at ambient temperature, the mixture was evaporated in vacuo. The residue was dissolved in methanol (5 ml) and m-chloroperbenzoic acid (368 mg) was added. The mixture was heated at 50°C for 2 days, left for a day at room temperature, and evaporated in vacuo. The residue was chromatographed on silica gel (3 g) eluting with a mixture of methylene chloride and methanol (10:1—2:1) to give (1R,6R) - 2,2 - dimethyl - 4,8 - dioxo - 7 - aza - 3 - oxabicyclo[4.2.0]octane (48 mg). m.p. 180—182°C.

IR ($CH_2Cl_2$): 1775, 1740 $cm^{-1}$.

NMR ($CDO_3OD$) δ: 1.46 (s, 3H), 1.60 (s, 3H), 2.82 (dd, 1H, J=17Hz, 2Hz), 2.96 (dd, 1H, J=17Hz, 4Hz), 3.50 (d, 1H, J=5Hz), 4.18 (ddd, 1H, J=5Hz, 4Hz, 2Hz), 4.62 (s, 3H).

| | | | |
|---|---|---|---|
| Elemental Analysis: | C 56.42%, | H 6.26%, | N 8.24% |
| Calcd for $C_8H_{11}NO_3$: | C, 56.80%, | H, 6.55%, | N, 8.28% |

Preparation 40

To a solution of methyl [(3R,4R) - 3 - amino - 4 - allyl - 2 - oxoazetidin - 1 - yl] - 3 - methylbut - 2 - enoate (7.0 g) in methylene chloride (110 ml) were added 2,6-lutidine (4.11 ml) and benzyl chloroformate (5.03 ml) at 0°C and the mixture was stirred for 30 minutes at 0°C. The reaction mixture was diluted with ethyl acetate (800 ml) and the solution was washed with diluted hydrochloric acid, brine, aqueous saturated sodium bicarbonate, and brine. The organic layer was dried over magnesium sulfate and evaporated in vacuo. The residue was chromatographed on silica gel (200 g) eluting with a mixture of methylene chloride and ethyl acetate (20:1—4:1) to give methyl [(3R,4R) - 3 - benzyloxycarbonylamino - 4 - allyl - 2 - oxoazetidin - 1 - yl] - 3 - methylbut - 2 - enoate (10.7 g) as an oil.

IR ($CH_2Cl_2$): 3380, 1750, 1750 $cm^{-1}$.

NMR ($CD_3OD$) δ: 1.95 (s, 3H), 2.17 (s, 3H), 2.44 (t, 2H, J=7Hz), 3.73 (s, 3H), 4.06 (dt, 1H, J=2.5, 7Hz), 4.44 (d, 1H, J=2.5Hz), 4.9—5.2 (m, 2H), 5.10 (s, 2H), 5.5—6.0 (m, 1H), 7.34 (s, 5H).

Preparation 41

Ozone was bubbled into a solution of methyl [(3R,4R) - 3 - benzyloxycarbonylamino - 4 - allyl - 2 - oxoazetidin - 1 - yl] - 3 - methylbut - 2 - enoate (5 g) in methanol (100 ml) at −60°C until a blue color was appeared. After stirring for 10 minutes at −60°C, the mixture was purged with nitrogen. Dimethyl sulfide (5 ml) was added to the mixture at −60°C and the mixture was allowed to warm to 0°C during 15 minutes. After stirring for an hour at 0°C, the mixture was left for 20 hours at ambient temperature. Dimethyl sulfide (2 ml), trimethyl orthoformate (5 ml), and p-toluenesulfonic acid monohydrate (150 mg) were added to the mixture and the mixture was heated at 50°C for 6 hours. Pyridine (0.076 ml) was added to the reaction mixture at ambient temperature and the mixture was evaporated in vacuo. The residue was dissolved in ethyl acetate and the solution was washed with water, dilute hydrochloric acid, brine, aqueous sodium bicarbonate, and brine.

The organic layer was dried over magnesium sulfate, and evaporated in vacuo. The residue was dissolved in methanol (100 ml), and the solution was heated at 50°C for 3 hours and left at ambient temperature for 2 days. The mixture was evaporated in vacuo and the residue was chromatographed on silica gel (100 g) eluting with a mixture of methylene chloride and acetone (10:1—2:1) to give (3R,4R) - 3 - benzyloxycarbonylamino - 4 - (2,2 - dimethoxyethyl)azetidin - 2 - one (2.83 g) as an oil.

IR ($CH_2Cl_2$): 1770, 1720 $cm^{-1}$.

NMR (acetone-$d_6$) δ: 1.8—2.2 (m, 2H), 3.30 (s, 6H), 3.66 (dt, 1H, J=3, 7Hz), 4.42 (dd, 1H, J=3, 9Hz), 4.51 (t, 1H, J=5.5Hz), 5.12 (s, 2H), 7.0 (brs. 1H), 7.2 (m, 1H), 7.40 (s, 5H).

Preparation 42

To a solution of (3R,4R)-3-benzyloxycarbonylamino-4-(2,2-dimethoxyethyl)azetidin-2-one (2.15 g) in acetic acid (35.2 ml) were added dimethylsulfide (2 ml) and water (8.8 ml) at ambient temperature and the mixture was heated at 50°C for 4.5 hours. The reaction mixture was evaporated in vacuo and the residue was dissolved in xylene (40 ml). The mixture was evaporated in vacuo and the residue was chromatographed on silica gel (10 g) eluting with a mixture of methylene chloride and acetone (5:1—2:1) to give a crystalline solid. This crude crystals were washed with ether and dried over phosphorus pentoxide to give (3R,4R)-3-benzyloxycarbonylamino-4-(2-oxoethyl)azetidin-2-one (863 mg).

IR ($CH_2Cl_2$): 1780, 1720 $cm^{-1}$.

NMR (acetone-$d_6$) δ: 2.7—3.1 (m, 2H), 3.8—4.0 (m, 1H), 4.43 (dd, 1H, J=2.8Hz), 5.07 (s, 2H), 6.9—7.2 (broad, 1H), 7.30 (s, 5H), 9.69 (s, 1H).

Preparation 43

To a solution of (3R,4R)-3-benzyloxycarbonylamino-4-(2-oxoethyl)azetidin-2-one (850 mg) in methanol (17 ml) was added sodium borohydride (123 mg) at 0°C and the mixture was stirred for 15 minutes at 0°C. Acetic acid (0.37 ml) was added to the mixture at 0°C. After stirring for 15 minutes at 0°C, the mixture was

evaporated in vacuo. The residue was chromatographed on silica gel (17 g) eluting with a mixture of methylene chloride and methanol (20:1—10:1) to give a crystalline solid. This crude crystals were washed with ether and dried over phosphorus pentoxide to give (3R,4R)-3-benzyloxycarbonylamino-4-(2-hydroxyethyl)azetidin-2-one (748 mg). m.p. 120—121°C.

IR ($CH_2Cl_2$): 1770, 1720 $cm^{-1}$.

NMR (acetone-$d_6$) δ: 1.7—2.11 (m, 2H), 2.82 (s, 1H), 3.5—3.8 (m, 3H), 4.39 (dd, 1H, J=3, 8Hz), 5.05 (s, 2H), 6.9—7.3 (broad, 2H), 7.30 (s, 5H).

Preparation 44

A solution of (3R,4R)-3-benzyloxycarbonylamino-4-(2,2-dimethoxyethyl)azetidin-2-one (0.60 g) in a mixture of tetrahydrofuran (10 ml) and 0.5N hydrochloric acid (2 ml) was heated at 50°C for 1 hour. The mixture was cooled to 0°C and 1N aqueous sodium bicarbonate (1.2 ml) was added to the mixture at 0°C. After stirring for 5 minutes at 0°C, sodium borohydride (0.10 g) was added to the mixture at 0°C. After stirring for 15 minutes at 0°C, acetone (0.77 ml) and acetic acid (0.30 ml) were added to the mixture at 0°C. The mixture was evaporated in vacuo and the residue was dissolved in methylene chloride (60 ml). The solution was dried over magnesium sulfate and evaporated in vacuo. The residue was dissolved in xylene (20 ml) and the solution was evaporated in vacuo. The residue was chromatographed on silica gel (12 g) eluting with a mixture of methylene chloride and methanol (20:1—10:1) to give (3R,4R)-3-benzyloxycarbonylamino-4-(2-hydroxyethyl)azetidin-2-one (314 mg) as a crystalline solid.

Preparation 45

To a suspension of (3R,4R)-3-benzyloxycarbonylamino-4-(2-hydroxyethyl)azetidin-2-one (735 mg) in methylene chloride (30 ml) were added 2,2-dimethoxypropane (0.513 ml) and boron trifluoride etherate (0.026 ml) at 0°C. The mixture was stirred for 10 minutes at 0°C and for 5 hours at ambient temperature. The reaction mixture was poured into a mixture of methylene chloride (30 ml) and aqueous sodium bicarbonate and sodium chloride (1:1). The organic layer was separated and the aqueous layer was extracted with methylene chloride (20 ml). The combined organic layers were washed with brine, dried over magnesium sulfate, and evaporated in vacuo. The residue was chromatographed on silica gel (16 g) eluting with a mixture of methylene chloride and acetone (10:1—5:1) to give (6R,7R)-7-benzyloxycarbonylamino-2,2-dimethyl-1-aza-3-oxabicyclo[4.2.0]octan-8-one (753 mg) as an oil.

IR ($CH_2Cl_2$): 1760, 1725 $cm^{-1}$.

NMR (acetone-$d_6$) δ: 1.36 (s, 3H), 1.61 (s, 3H), 1.4—2.1 (m, 2H), 3.5—3.9 (m, 3H), 4.38 (dd, 1H, J=2, 8Hz), 5.08 (s, 2H), 7.0 (broad, 1H), 7.35 (s, 5H).

Preparation 46

A mixture of (6R,7R)-7-benzyloxycarbonylamino-2,2-dimethyl-1-aza-3-oxabicyclo[4.2.0]octan-8-one (748 mg) and 10% palladium charcoal (180 mg) in ethyl acetate (15 ml) was stirred under a hydrogen atmosphere for 1.5 hours at ambient temperature. The mixture was filtered and the filtrate was evaporated in vacuo to give (6R,7R) - 7 - amino - 2,2 - dimethyl - 1 - aza - 3 - oxabicyclo[4.2.0]octan - 8 - one (382 mg) as a crystalline solid.

IR ($CH_2Cl_2$): 1740 $cm^{-1}$.

NMR ($CDCl_3$) δ: 1.42 (s, 3H), 1.72 (s, 3H), 1.5—2.1 (m, 2H), 1.86 (s, 2H), 3.34 (ddd, 1H, J=2, 5, 10Hz), 3.6—3.9 (m, 3H).

Preparation 47

To a solution of (6R,7R) - 7 - amino - 2,2 - dimethyl - 1 - aza - 3 - oxabicyclo[4.2.0]octan - 8 - one (300 mg) were added pyridine (0.25 ml) and freshly prepared acetic formic anhydride (0.38 ml) at 0°C. After stirring for 45 minutes at 0°C, the reaction mixture was evaporated in vacuo. The residue was dissolved in xylene and the solution was evaporated in vacuo. The residue was chromatographed on silica gel (6 g) eluting with a mixture of methylene chloride and methanol (25:1—10:1) to give (6R,7R) - 2,2 - dimethyl - 7 - formamido - 1 - aza - 3 - oxabicyclo[4.2.0]octan - 8 - one (378 mg) as an oil.

IR ($CH_2Cl_2$): 1755, 1685 $cm^{-1}$.

NMR ($CDCl_3$) δ: 1.43 (s, 3H), 1.72 (s, 3H), 1.8—2.1 (m, 2H), 3.56 (ddd, 1H, J=2, 5, 10Hz), 3.7—3.9 (m, 2H), 4.37 (dd, 1H, J=2, 8Hz), 7.33 (broad d, 1H, J=8Hz), 8.11 (s, 1H).

Preparation 48

To a solution of (6R,7R)-2,2-dimethyl-7-formamido-1-aza-3-oxabicyclo[4.2.0]octan-8-one (345 mg) were added 2,6-lutidine (2.05 ml) and phosphorus oxychloride (0.52 ml) at 0°C. After stirring for 3 hours at 0°C, the mixture was poured into a mixture of ethyl acetate (80 ml) and ice-water. The organic layer was separated, washed in turn with 10% phosphoric acid, water, brine, an aqueous mixture of sodium bicarbonate and sodium chloride (1:1), and brine, dried over magnesium sulfate, and evaporated in vacuo. The crystalline residue was washed with a mixture of petroleum ether and ether (1:1) and dried over phosphorus pentoxide to give (6R,7R) - 2,2 - dimethyl - 7 - isocyano - 1 - aza - 3 - oxabicyclo[4.2.0]octan - 8 - one (205 mg).

IR ($CH_2Cl_2$): 2140, 1770 $cm^{-1}$.

NMR ($CDCl_3$) δ: 1.45 (s, 3H), 1.72 (s, 3H), 1.4—2.1 (m, 2H), 3.7—3.9 (m, 3H), 4.30 (d, 1H, J=2Hz).

Preparation 49

Ozone was passed into a solution of methyl 2 - {(3S,4R) - 3 - [(1R) - 1 - (4 - nitrobenzyloxycarbonyloxy)ethyl] - 2 - oxo - 4 - allylazetidin - 1 - yl} - 3 - methylbut - 2 - enoate (100 mg) in methanol (3 ml) at −70°C until a blue color was appeared. In the course of the reaction, white crystals were precipitated from the reaction mixture. After stirring for 10 minutes at −70°C, the mixture was purged with nitrogen. Cold methylene chloride (2 ml) and dimethyl sulfide (0.6 ml) were added to the mixture at −70°C and the mixture was warm to 0°C during 1 hour. Then the mixture was left at 0°C for 3 hours and at ambient temperature for 12 hours. The mixture was evaporated in vacuo and the residue was dissolved in methanol (5 ml).

Dimethyl sulfide (0.3 ml), trimethyl orthoformate (0.5 ml) and p-toluenesulfonic acid monohydrate (4.3 mg) were added to the solution and the mixture was heated for an hour at 58—55°C. Pyridine (2 drops) was

added to the mixture at ambient temperature and the mixture was evaporated in vacuo. The residue was dissolved with ethyl acetate (30 ml) and the solution was washed in turn with water, brine containing 0.1N hydrochloric acid, brine, an aqueous saturated sodium bicarbonate containing sodium chloride, and brine, dried over magnesium sulfate, and evaporated in vacuo. The residue was dissolved in methanol (5 ml) and the solution was left for 15 hours at ambient temperature. The mixture was evaporated in vacuo and the residue was chromatographed on silica gel (2 g) eluting with a mixture of methylene chloride and acetone (20:1—5:1) to give (3S,4R) - 4 - (2,2 - dimethoxyethyl) - 3 - [(1R) - 1 - (4 - nitrobenzyloxycarbonyloxy)ethyl]azetidin - 2 - one (75 mg) as an oil.

IR ($CH_2Cl_2$): 1760, 1730, 1520, 1350 $cm^{-1}$.

NMR ($CDCl_3$) δ: 1.44 (d, 3H, J=7Hz), 1.94 (t, 2H, J=6Hz), 3.03 (dd, 1H, J=2.5Hz), 3.32 (s, 6H), 3.70 (dt, 1H, J=2.6Hz), 4.43 (t, 1H, J=6Hz), 5.0—5.3 (m, 1H), 5.24 (s, 2H), 6.65 (s, 1H), 7.86 ($A_2B_2$, 4H, J=9Hz).

Preparation 50

A mixture of (6R,7R) - 2,2 - dimethyl - 7 - isocyano - 1 - aza - 3 - oxabicyclo[4.2.0]octan - 8 - one (150 mg), tribulyltinhydride (0.263 ml), and azobisisobutyronitrile (14 mg) in benzene (4.5 ml) was refluxed for 15 minutes. The resulting solution was chromatographed on silica gel (4.5 g) eluting with a mixture of benzene and acetone (15:1—6:1) to give the crude product (140 mg) as an oil, which was purified by silica gel (5 g) chromatography (eluate: a mixture of methylene chloride and ethyl acetate 30:1—5:1) to give (6R) - 2,2 - dimethyl - 1 - aza - 3 - oxabicyclo[4.2.0]octan - 8 - one (98 mg) as an oil.

IR ($CH_2Cl_2$): 1740 $cm^{-1}$.

NMR ($CDCl_3$) δ: 1.42 (s, 3H), 1.77 (s, 3H), 1.4—2.1 (m, 2H), 2.56 (dd, 1H, J=2.5, 14.5Hz), 3.08 (dd, 1H, J=5, 14.5Hz), 3.5—3.7 (m, 1H), 3.87 (dd, 2H, J=2.5, 8Hz).

Preparation 51

To a solution of (6R,7R) - 2,2 - dimethyl - 7 - isocyano - 1 - aza - 3 - oxabicyclo[4.2.0]octan - 8 - one (200 mg) in tetrahydrofuran (4 ml) was added a solution of n-butyllithium in hexane (0.93 ml of 1.55M solution) at −70°C and the mixture was stirred at −70°C for 15 minutes. A solution of acetaldehyde (73 mg) in tetrahydrofuran (0.8 ml) was added to the mixture at −70°C and the mixture was stirred at −70°C for 30 minutes. Acetic acid (0.19 ml) was added to the mixture at −70°C and the mixture was poured into a mixture of ethyl acetate (100 ml) and aqueous sodium bicarbonate containing a small amount of sodium chloride. The aqueous layer was separated and extracted with ethyl acetate (20 ml). The combined organic layers were washed with brine, dried over magnesium sulfate, and evaporated in vacuo. The residue was chromatographed on silica gel (3 g) eluting with a mixture methylene chloride and ethyl acetate (20:1—5:1) to give a mixture of (6R,7R) - 2,2 - dimethyl - 7 - [(1S) - 1 - hydroxyethyl] - 7 - isocyano - 1 - aza - 3 - oxabicyclo[4.2.0]octan - 8 - one, its (1R,6R,7R) isomer, its (1R,6R,7S) isomer, and its (1S,6R,7S) isomer as a solid (230 mg).

IR ($CH_2Cl_2$): 3600, 2140, 1770 $cm^{-1}$.

NMR ($CDCl_3$) δ: 1.3—1.6 (m, 3H), 1.44 (s, 3H), 1.76 (s, 3H), 1.6—2.2 (m, 3H), 2.8—3.1 (m, 1H), 3.7—4.1 (m, 2H), 4.0—4.4 (m, 1H).

Preparation 52

OH
CH3CH
H
CN
N
O
O
OH
CH3CH
H
H
N
O
O

To a mixture of (6R,7R) - 2,2 - dimethyl - 7 - [(1S) - 1 - hydroxyethyl] - 7 - isocyano - 1 - aza - 3 - oxabicyclo[4.2.0]octan - 8 - one, its (1R,6R,7R) isomer, its (1R,6R,7S) isomer, and its (1S,6R,7S) isomer (210 mg) in benzene (10 ml) were added tributyltinhydride (0.297 ml) and azobisisobutyronitrile (15 mg) at ambient temperature and the mixture was refluxed for 30 minutes. The resulting solution was chromatographed on silica gel (7.5 g) eluting with a mixture of methylene chloride and acetone (10:1—5:1) to give the crude product, which was purified by silica gel (10 g) chromatography (a mixture of hexane and ethyl acetate (5:1—1:5) to give a mixture of (6R,7R) - 2,2 - dimethyl - 7 - [(1S) - 1 - hydroxyethyl] - 1 - aza - 3 - oxabicyclo[4.2.0]octan - 8 - one, its (1R,6R,7R) isomer, its (1R,6R,7S) isomer, and its (1S,6R,7S) isomer as an oil (157 mg).

IR ($CH_2Cl_2$): 3400 1740 $cm^{-1}$.

NMR ($CDCl_3$) δ: 1.1—1.4 (m, 3H), 1.43 (2, 3H), 1.76 (s, 3H), 1.6—2.2 (m, 3H), 2.2—3.0 (m, 1H), 3.0—3.2 (m, 1H), 3.6—4.0 (m, 2H), 4.0—4.4 (m, 1H).

Preparation 53

OH
CH3CH
H
H
N
O
O
OCOOPNB
CH3CH
H
H
N
O
O

A solution of p-nitrobenzyl chloroformate (251 mg) in methylene chloride (2.5 ml) was added to a mixture of (6R,7R) - 2,2 - dimethyl - 7 - [(1S) - 1 - hydroxyethyl] - 1 - aza - 3 - oxabicyclo[4.2.0]octan - 8 - one, its (1R,6R,7R) isomer, its (1R,6R,7S) isomer, its (1S,6R,7S) isomer, and 4-(dimethylamino)pyridine (143 mg) in methylene chloride (3 ml) at 0°C. After stirring at 0°C for 30 minutes, the mixture was allowed to warm to ambient temperature during 45 minutes and stirred at ambient temperature for 1.5 hours. A solution of p-nitrobenzyl chloroformate (100 mg) in methylene chloride (1 ml) and 4-(dimethylamino)pyridine (58 mg) were added to the mixture at 0°C and the mixture was stirred at ambient temperature for 1.5 hours. 3-Dimethylaminopropylamine (0.158 ml) was added to the mixture at 0°C and the mixture was stirred at 0°C for 15 minutes. The mixture was poured into a mixture of ethyl acetate (70 ml) and dilute hydrochloric acid. The organic layer was separated, washed with brine, aqueous sodium bicarbonate, and brine, dried over magnesium sulfate, and evaporated in vacuo. The residue was chromatographed on silica gel (10 g) eluting with a mixture of methylene chloride and ethyl acetate (10:1—2:1) to give a 9:11:4:1 mixture of (6R,7R) - 2,2 - dimethyl - 7 - [(1S) - 1 - (4 - nitrobenzyloxycarbonyloxy)ethyl) - 1 - aza - 3 - oxabicyclo[4.2.0]octan - 8 - one, its (1R,6R,7R) isomer, its (1R,6R,7S) isomer, and its (1S,6R,7S) isomer as an oil (167 mg).

The residue was chromatographed on silica gel (15 g) eluting with a mixture of cyclohexane and ethyl acetate (5:1→1:1) to give 70 mg of the (1S,6R,7R) isomer and 60 mg of the (1R,6R,7R)-isomer, and 10 mg of the (1R,6R,7S)-isomers.

Deta for the (1S,6R,7R)-isomer:

IR ($CH_2Cl_2$): 1745, 1525, 1350 $cm^{-1}$.

NMR ($CDCl_3$) δ: 1.2—2.0 (m, 2H), 1.50 (d, 3H, J=6Hz), 1.74 (s, 3H), 3.42 (dd, 1H, J=5, 11Hz), 3.6—4.0 (m, 1H), 5.22 (dq, 1H, J=11, 6Hz), 5.30 (s, 2H), 7.94 ($A_2B_2$, 4H, J=9Hz).

Deta for the (1R,6R,7R)-isomer:

IR ($CH_2Cl_2$): 1745, 1525, 1350 $cm^{-1}$.

NMR ($CDCl_3$) δ: 1.3—2.0 (m, 2H), 1.4 (d, 3H, partially hidden), 1.44 (s, 3H), 1.75 (s, 3H), 3.39 (dd, 1H, J=5.5, 9Hz), 3.7—4.1 (m, 3H), 5.1—5.4 (m, 1H), 5.34 (s, 2H), 7.98 ($A_2B_2$, 4H, J=9Hz).

Deta for the (1R,6R,7S)-isomer:

IR ($CH_2Cl_2$): 1745, 1525, 1350 $cm^{-1}$.

NMR ($CDCl_3$) δ: 1.3—2.0 (m, 2H), 1.42 (s, 3H), 1.47 (d, 3H, J=6Hz), 1.76 (s, 3H), 3.00 (dd, 1H, J=2, 8Hz), 3.5—4.0 (m, 3H), 5.1 (m, 1H), 5.30 (s, 2H), 7.96 ($A_2B_2$, 4H, J=9Hz).

Preparation 54

CN
OH
$(CH_3)_2C$
H
CN
O
N
O
+
OH
CN
H
$(CH_3)_2C$
O
N
O

To a solution of (6R,7R)-2,2-dimethyl-7-isocyano-1-aza-3-oxabicyclo[4.2.0]octan-8-one (500 mg) in tetrahydrofuran (15 ml) was added a solution of n-butyllithium in hexane (2.33 ml of 1.55M solution) at −70°C and the mixture was stirred at −70°C for 15 minutes. A solution of acetone (0.265 ml) in tetrahydrofuran (2.4 ml) was added to the reaction mixture at −70°C and the mixture was stirred at −70°C for 25 minutes. Acetic acid (0.477 ml) was added to the mixture at −70°C. After stirring at −70°C for 20 minutes, the mixture was evaporated in vacuo. The residue was dissolved in ethyl acetate (80 ml) and the solution was washed with aqueous sodium bicarbonate. The aqueous layer was separated and extracted with ethyl acetate (20 ml). The combined organic layers were washed with brine, dried over magnesium sulfate, and evaporated in vacuo. The crystalline residue was washed with isopropyl ether and filtered to give (6R,7R)-2,2-dimethyl-7-(1-hydroxy-1-methylethyl)-7-isocyano-1-aza-3-oxabicyclo[4.2.0]octan-8-one (460 mg). The mother liquor was chromatographed on silica gel (5 g) eluting with a mixture of hexane and ethyl acetate (20:1—1:1) to give additional (6R,7R) - 2,2 - dimethyl - 7 - (1 - hydroxy - 1 - methylethyl) - 7 - isocyano - 1 - aza - 3 - oxabicyclo[4.2.0]octan - 8 - one (65 mg) [(6R,7R)-isomer] and its (6R,7S)-isomer (103 mg).

For (6R,7R)-isomer:

IR ($CH_2Cl_2$): 2125, 1765 $cm^{-1}$.

NMR ($CDCl_3$) δ: 1.38 (s, 3H), 1.43 (s, 3H), 1.50 (s, 3H), 1.74 (s, 3H), 1.5—2.2 (m, 2H), 2.10 (s, 1H), 3.7—4.0 (m, 3H).

For (6R,7S)-isomer:

IR ($CH_2Cl_2$): 2125, 1765 $cm^{-1}$.

NMR ($CDCl_3$) δ: 1.40 (s, 3H), 1.48 (s, 3H), 1.63 (s, 3H), 1.74 (s, 3H), 1.5—1.9 (m, 1H), 2.21 (s, 1H), 2.6—3.0 (m, 1H), 3.7—4.0 m, 3H).

Preparation 55

To a mixture of (6R,7R) - 2,2 - dimethyl - 7 - (1 - hydroxy - 1 - methylethyl) - 7 - isocyano - 1 - aza - 3 - oxabicyclo[4.2.0]octan - 8 - one and its (6R,7S)-isomer (2.5 g) in benzene (75 ml) were added tributyltinhydride (3.07 ml) and azobisisobutyronitrile (170 mg) at ambient temperature and the mixture was refluxed for 30 minutes. The reaction mixture was chromatographed on silica gel (65 g) eluting with a mixture of methylene chloride and acetone (10:1—2:1) to give a crude product, which was purified by silica gel (75 g) chromatography (hexane and ethyl acetate) to give (6R,7S) - 2,2 - dimethyl - 7 - (1 - hydroxy - 1 - methylethyl) - 1 - aza - 3 - oxabicyclo[4,2,0]octan - 8 - one (0.545 g) [(6R,7S)-isomer] and its (6R,7R)-isomer (1.406 g).

For (6R,7S)-isomer:
IR ($CH_2Cl_2$): 1735 $cm^{-1}$.
NMR ($CDCl_3$) δ: 1.29 (s, 3H), 1.36 (s, 3H), 1.42 (s, 3H), 1.74 (s, 3H), 1.5—2.0 (m, 2H), 1.96 (s, 1H), 2.81 (d, 1H, J=2Hz), 3.52 (ddd, 1H, J=2, 5.5, 11Hz), 3.83 (dd, 2H, J=3, 8.5Hz).
For (6R,7R)-isomer:
IR ($CH_2Cl_2$): 1735 $cm^{-1}$.
NMR ($CDCl_3$) δ: 1.28 (s, 3H), 1.40 (s, 3H), 1.47 (s, 3H), 1.74 (s, 3H), 1.5—2.0; (m, 1H), 1.98 (s, 1H), 2.5—2.9 (m, 1H), 3.15 (d, 1H, J=5Hz), 3.6—3.9 (m, 3H).

In the same manner described above, (6R,7S) - 2,2 - dimethyl - 7 - (1 - hydroxy - 1 - methylethyl - 7 - isocyano - aza - 3 - oxabicyclo[4.2.0]octan - 8 - one (50 mg) afforded a 2:5 mixture of (6R,'7S) - 2,2 - dimethyl - 7 - (1 - hydroxy - 1 - methylethyl) - 1 - aza - 3 - oxabicyclo[4.2.0]octan - 8 - one and its (6R,7R)-isomer (40 mg).

Preparation 56

To a solution of methyl 2 - [(3R, 4R) - 3 - isocyano - 2 - oxo - 4 - allylazetidin - 1 - yl] - 3 - methylbut - 2 - enoate (127.6 mg) in tetrahydrofuran (2.55 ml) was added dropwise a solution of n-butyl lithium (0.409 ml of 1.51 M solution in hexane) at −78°C over a period of 3 minutes. After stirring for 15 minutes at −78°C, the reaction mixture was added dropwise with a solution of acetone (0.0459 ml) in tetrahydrofuran (0.459 ml) at −78°C over a period of 3 minutes. After stirring for 25 minutes at −78°C, the reaction mixture was added dropwise with a solution of 4-nitrobenzyl chloroformate (124.1 mg) in tetrahydrofuran (1.24 ml) at −78°C over a period of 3 minutes. The resulting solution was allowed to warm to 0°C over a period of 40 minutes and added with acetic acid (0.0412 ml) at 0°C. The solvent was distilled off in vacuo at 0°C and the residue was dissolved in ethyl acetate (30 ml) and washed with brine, aqueous sodium bicarbonate, brine and saturated aqueous sodium chloride. After drying over magnesium sulfate, the ethyl acetate extract was filtered and evaporated in vacuo. The residual oil (285.4 mg) was chromatographed on silica gel (5.7 g) eluting with a mixture of ethyl acetate and hexane (1:5→1:3) to give methyl 2 - {(3R, 4R) - 3 - isocyano - 3 - [1 - methyl - 1 - (4 - nitrobenzyloxycarbonyloxy)ethyl] - 2 - oxo - 4 - allylazetidin - 1 - yl} - 3 - methylbut - 2 - enoate (211.2 mg) as an oil.
IR ($CH_2Cl_2$): 2130, 1775, 1750, 1720, 1520, 1350 $cm^{-1}$.
NMR ($CDCl_3$) δ: 1.74 (s, 3H), 1.83 (s, 3H), 1.97 (s, 3H), 2.22 (s, 3H), 2.53 (t, 2H, J=7Hz), 3.71 (s, 3H), 4.32 (t, 1H, J=7Hz), 5.0—5.3 (m, 4H), 5.5—5.9 (m, 1H), 7.84 ($A_2B_2$, 4H, J=9Hz).

Preparation 57

To a solution of methyl 2 - [(3R, 4R) - 3 - isocyano - 2 - oxo - 4 - allylazetidin - 1 - yl] - 3 - methylbut - 2 - enoate (198.0 mg) in tetrahydrofuran (3.96 ml) was added dropwise a solution of n-butyl lithium (0.634 ml of 1.51 M solution in hexane) at −78°C over a period of 5 minutes. After stirring for 15 minutes at −78°C, the reaction mixture was added with a solution of acetone (0.0713 ml) in tetrahydrofuran (0.642 ml) at −78°C over a period of 5 minutes. After stirring for 25 minutes at −78°C, the reaction mixture was added dropwise with a solution of benzyl chloroformate (0.1274 ml) in tetrahydrofuran (1.15 ml) at −78°C over a period of 5 minutes. The resulting solution was allowed to warm to 0°C over a period of 40 minutes and added with acetic acid (0.0639 ml) at 0°C. The solvent was distilled off in vacuo at 0°C and the

residue was dissolved in ethyl acetate (30 ml) and washed with brine, aqueous sodium bicarbonate, brine, and saturated aqueous sodium chloride. After drying over magnesium sulfate, the ethyl acetate extract was filtered and evaporated in vacuo. The residual oil (381.2 mg) was chromatographed on silica gel (11 g) eluting with a mixture of ethyl acetate and hexane (1:10→1:5) to give methyl 2 - [(3R, 4R) - 3 - (1 - benzyloxycarbonyloxy - 1 - methylethyl) - 3 - isocyano - 2 - oxo - 4 - allylazetidin - 1 - yl] - 3 - methylbut - 2 - enoate (221.1 mg) as an oil.

IR ($CH_2Cl_2$): 2130, 1770, 1745, 1720 $cm^{-1}$.

NMR ($CDCl_3$) δ: 1.71 (s, 3H), 1.85 (s, 3H), 1.95 (s, 3H), 2.20 (s, 3H), 2.53 (t, 2H, J=7Hz), 3.63 (s, 3H), 4.35 (t, 1H, J=7Hz), 5.0—5.35 (m, 4H), 5.5—6.0 (m, 1H), 7.3—7.5 (m, 5H).

Preparation 58

A mixture of methyl 2 - [(3R, 4R) - 3 - (1 - benzyloxyccarbonyloxy - 1 - methylethyl) - 3 - isocyano - 2 - oxo - 4 - allylazetidin - 1 - yl] - 3 - methylbut - 2 - enoate (42.7 mg), tri-n-butyltinhydride (0.0308 ml) of azobisisobutyronitrile (0.622 mg) in benzene (2.14 ml) was refluxed for 30 minutes. The resulting solution was chromatogaphed on silica gel (2.1 g) eluting with a mixture of ethyl acetate and hexane (1:10→1:2) to give a mixture of methyl 2 - [(3R, 4R) - 3 - (1 - benzyloxycarbonyloxy - 1 - methylethyl) - 2 - oxo - 4 - allylazetidin - 1 - yl] - 3 - methylbut - 2 - enoate and methyl 2 - [(3R, 4R) - 3 - (1 - benzyloxycarbonyloxy - 1 - methylethyl) - 2 - oxo - 4 - allylazetidin - 1 - yl] - 3 - methylbut - 2 - enoate (39.8 mg; ratio = 1:2.3) as an oil.

IR ($CH_2Cl_2$): 1750, 1740, 1730 $cm^{-1}$.

NMR ($CDCl_3$) δ: 1.59 (s, 2.1H), 1.61 (s, 0.9H), 1.65 (s, 2.1H), 1.67 (s, 0.9H), 1.75 (s, 0.9H), 1.91 (s, 2.1H), 1.96 (s, 0.9H), 2.18 (s, 2.1H), 2.31 (t, 1.4H, J=7Hz), 2.61 (t, 0.6H, J=7Hz), 3.42 (d, 0.7H, J=2Hz), 3.5—3.8 (m, 3.3H), 3.88—4.22 (m, 1H), 4.8—5.2 (m, 4H), 5.3—5.9 (m, 1H), 7.0—7.5 (m, 5H).

Preparation 59

To a solution of methyl 2 - {(3S, 4R) - 3 - [1 - methyl - 1 - (4 - nitrobenzyloxycarbonyloxy)ethyl] - 2 - oxo - 4 - allylazetidin - 1 - yl} - 3 - methylbut - 2 - enoate (72.9 mg) in acetone (2.03 ml) was added a 0.7 M solution of phosphate butter (pH 6.8, 1.35 ml) at ambient temperature. To the resulting suspension was added dropwise a solution of sodium periodate (86.0 mg) in water (0.594 ml) at ambient temperature. After stirring for 10 minutes, the reaction mixture was added with sodium periodate (117.2 mg) and potassium permanganate (37.5 mg) and stirred at ambient temperature overnight. The resulting mixture was diluted with water-acetone (15 ml — 15 ml) and filtered with celite and the filter cake was washed with water-acetone (1:1). The combined filtrates were concentrated in vacuo at 0°C, acidified to pH 1 with 10% aqueous phosphoric acid, and extracted with chloroform. The organic layer was extracted with chilled 10% aqueous sodium bicarbonate (X3). The aqueous extracts were acidified with 3N hydrochloric acid and reextracted with chloroform (X3). The chloroform extracts were washed with saturated aqueous sodium chloride containing a small amount of 3N hydrochloric acid, dried over magnesium sulfate, filtered, and evaporated to give {(2R, 3S) - 3 - [1 - methyl - 1(4 - nitrobenzyloxycarbonyloxy)ethyl] - 4 - oxoazetidin - 2 - yl}acetic acid (36.4 mg) as an amorphous solid.

IR ($CH_2Cl_2$): 1740, 1520, 1350 $cm^{-1}$.

Preparation 60

b-Butyllithium (1.51N in hexane, 0.99 ml) was added dropwise to a solution of methyl 2 - [(3R, 4R) - 3 - isocyano - 2 - oxo - 4 - allylazetidin - 1 - yl] - 3 - methylbut - 2 - enoate (308 mg) in tetrahydrofurane (6 ml) during a three-minutes period at −78°C under a nitrogen atmosphere. After 15 minutes at −78°C a solution of acetaldehyde in tetrahydrofuran (2M, 0.93 ml) was added dropwise. The mixture was stirred at −78°C for 30 minutes and quenched by addition of acetic acid (143 μl). The solution was allowed to warm to 0°C, poured into a mixture of a saturated aqueous solution of sodium bicarbonate and brine, and extracted with ethyl acetate (50 ml). The extract was washed with brine, dried over magnesium sulfate and evaporated to give an oil (274 mg). The residue was chromatographed on silica gel (5 g; eluting with 5—10% acetone in dichloromethane) to give 199 mg (54.9%) of four diastereomeric mixture of methyl 2 - [(4R) - 3 - (1 - hydroxyethyl) - 3 - isocyano - 2 - oxo - 4 - allylazetidin - 1 - yl] - 3 - methylbut - 2 - enoate.

IR ($CH_2Cl_2$): 3450, 2140, 1770, 1725 $cm^{-1}$.

Preparation 61

Tri(n-butyl)tin hydride (0.20 ml) and azobisisobutyronitrile (11.5 mg) were added to a solution of the four isomeric mixture of methyl 2 - [3 - (1 - hydroxyethyl) - 3 - isocyano - 2 - oxo - 4(R)allylazetidin - 1 - yl] - 3 - methylbut - 2 - enoate (195 mg) in anhydrous benzene (4 ml). The stirring mixture was heated to reflux for 15 minutes. The reaction mixture was cooled to ambient temperature and chromatographed on silica gel (6g; eluting with a mixture of 5—20% acetone in dichloromethane to give 155 mg (87.3%) of the four-isomeric mixture of methyl 2 - [(4R) - 3 - (1 - hydroxyethyl) - 2 - oxo - 4 - allylazetidin - 1 - yl] - 3 - methylbut - 2 - enoate as a crude oil. This crude oil (118 mg) was chromatographed on silica gel (10 g; eluting with 4—20% acetone in benzene) to give 47 mg of methyl 2 - [(3S, 4R) - 3 - ((1RS) - 1 - hydroxyethyl) - 2 - oxo - 4 - allylazetidin - 1 - yl] - 3 - methylbut - 2 - enoate and 54 mg of methyl 2 - [(3R, 4R) - 3 - ((1RS) - 1 - hydroxyethyl) - 2 - oxo - 4 - allylazetidin - 1 - yl] - 3 - methylbut - 2 - enoate.

IR ($CH_2Cl_2$) for the mixture of four isomers, 3640, 3550, 3100—3600, 1735, and 1715 $cm^{-1}$.

Preparation 62

A solution of 4-nitrobenzyl chloroformate (64.7 mg) in dichloromethane (0.65 ml) was added to a solution of methyl 2 - [(3S, 4R) - 3 - ((1RS - 1 - hydroxyethyl) - 2 - oxo - 4 - allylazetidin - 1 - yl] - 3 - methylbut - 2 - enoate (54 mg) and dimethylaminopyridine (36.7 mg) in dichloromethane (2.35 ml) at 0°C under a nitrogen atmosphere. After three minutes the heterogeneous mixture was allowed to warm to ambient temperature (27°C). After two hours-stirring, additional 4-nitrobenzyl chloroformate (45 mg) and dimethylaminopyridine (25 mg) were added at 0°C. The mixture was stirred at ambient temperature for 15 hours and the resulting solution was evaporated. The residue was taken up into ethyl acetate (20 ml), washed in turn with dilute hydrochloric acid, water, a dilute aqueous solution of sodium bicarbonate, and brine dried over magnesium sulfate, and evaporated. The residue was chromatographed on silica gel (2.5 g; eluting with 2% acetone in dichloromethane) to give 76 mg (84.3%) of a 4:7 mixture of methyl 2 - [(3S, 4R) - 3 - [(1RS) - 1 - (4 - nitrobenzyloxycarbonyloxy)ethyl] - 2 - oxo - 4 - allylazetidin - 1 - yl] - 3 - methylbut - 2 - enoate.

IR ($CH_2Cl_2$): 1750, 1720, 1525, amd 1350 $cm^{-1}$.

NMR ($CDCl_3$) δ: 1.46 (d, J=7Hz, 1.2H), 1.48 (d, J=7Hz, 1.8H), 1.97 (s, 3H), 2.20 (s, 3H), 2.4 (m, 2H), 3.06 (dd, J=3, 8Hz, 0.4H), 3.19 (dd, J=3, 5Hz, 0.6H), 3.76 (s, 3H), 3.92 (m, 1H), 4.9—5.4 (m, 3H), 5.28 (s, 2H), 5.5—6.0 (m, 1H), 7.56 (d, J=9Hz, 0.8H), 7.58 (d, J=9Hz, 1.2H), and 8.23 (d, J=9Hz, 2H).

Preparation 63

In a manner similar to the Preparation 62, except using five molar excess of reagents, 47.0 mg of methyl 2 - [(3R, 4R) - 3 - (1RS) - 1 - hydroxyethyl) - 2 - oxo - 4 - allylazetidin - 1 - yl] - 3 - methylbut - 2 - enoate afforded 64 mg (81.5%) of a 3:7 mixture of methyl 2 - {(3R, 4R) - 3 - [(1RS) - 1 - (4 - nitrobenzyloxycarbonyloxy)ethyl] - 2 - oxo - 4 - allylazetidin - 1 - yl}) - 3 - methylbut - 2 - enoate and 7 mg (15%) of the starting material.

This mixture (60 mg) was chromatographed on a silica gel plate (20 cm × 20 cm × 2 mm, three developments with a mixture of 6:4 hexane-ethyl acetate) to give 39 mg of the 1S isomer and 20 mg of the 1R isomer.

Data for the 1S isomer:

IR ($CH_2Cl_2$): 1745, 1720, 1525, 1350 $cm^{-1}$.

NMR ($CDCl_3$) δ: 1.57 (d, J=6Hz, 3H), 1.94 (s, 3H), 2.20 (s, 3H), 2.40 (t, J=7Hz, 2H), 3.51 (dd, J=5.5 and 11Hz, 1H), 3.74 (s, 3H), 4.14 (dt, J=5.5 and 7Hz), 4.9—5.3 (m, 3H), 5.27 (s, 2H), 5.4—5.9 (m, 1H), 7.57 (d, J=9Hz, 2H), and 8.25 (d, J=9Hz, 2H)ppm.

Data for the 1R isomer:

IR ($CH_2Cl_2$): 1745, 1720, 1525, 1350 $cm^{-1}$.

NMR ($CDCl_3$) δ: 1.53 (d, J=6Hz, 3H), 2.03 (s, 3H), 2.22 (s, 3H), 2.40 (t, J=7Hz, 2H), 3.39 (dd, J=4 and 6Hz, 1H), 3.76 (s, 3H), 4.13 (dt, J=6 and 7Hz, 1 H), 4.7—5.3 (m, 3H), 5.30 (s, 2H), 5.4—5.9 (m, 1H), 7.56 (d, J=9Hz, 2H), and 8.25 (d, J=9Hz, 2H) ppm.

Preparation 64

OH
(CH3)2C
H
H
N
O
O
OCOOPNB
(CH3)2C
H
H
N
O
O

To a solution of (6R, 7R) - 2,2 - dimethyl - 7 - (1 - hydroxy - 1 - methylethyl) - 1 - aza - 3 - oxabicyclo[4.2.0] - octan - 8 - one (600 mg) in tetrahydrofuran (24 ml) was added a solution of n-butyllithium (2.36 ml of 1.55 M solution in hexane) at −70°C and the mixture was stirred at −70°C for 15 minutes.

A solution of 4-nitrobenzyl chloroformate (788 mg) in tetrahydrofuran (7 ml) was added to the mixture at −70°C. After stirring at −70°C for 15 minutes, the reaction mixture was allowed to warm to 0°C during 50 minutes, stirred at 0°C for 30 minutes, and evaporated in vacuo. The residue was dissolved in ethyl acetate (100 ml) and the solution was washed with aqueous sodium bicarbonate. The aqueous layer was separated and extracted with ethyl acetate (20 ml). The combined organic layers were washed with aqeuous saturated sodium chloride, dried over magnesium sulfate, and evaporated in vacuo. The residue was chromatographed on silica gel (40 g) eluting with a mixture of hexane and ethyl acetate (10:1 to 1:1) to give a crystalline residue. The residue was washed with isopropyl ether and filtered to give (6R, 7R) - 2,2 - dimethyl - 7 - [1 - methyl - 1 - (4 - nitrobenzyloxycarbonyloxy)ethyl] - 1 - aza - 3 - oxabicylco[4.2.0]octan - 8 - one (520 mg).

IR ($CH_2Cl_2$): 1740, 1520, 1350 $cm^{-1}$.

NMR ($CDCl_3$) δ: 1.41 (s, 3H), 1.62 (s, 3H), 1.77 (s, 6H), 1.5—1.9 (m, 1H), 2.2—2.5 (m, 1H), 3.42 (d, 1H, J=5.5Hz), 3.6—4.0 (m, 3H), 5.20 (s, 2H), 7.88 ($A_2B_2$, 4H, J=9Hz).

Preparation 65

OCOOPNB
(CH3)2C
H
H
N
O
O
OCOOPNB
(CH3)2C
H
H
NH
OH
O

A solution of (6R, 7R) - 2,2 - dimethyl - 7 - [1 - methyl - 1 - (4 - nitrobenzyloxycarbonyloxy)ethyl] - 1 - aza - 3 - oxabicyclo[4.2.0]octan - 8 - one (200 mg) in a mixture of acetic acid (3.2 ml) and water (0.8 ml) was heated at 65°C for 1 hour. The mixture was cooled to room temperature and evaporated in vacuo. The crystalline residue was washed with ether to give (3R, 4R) - 4 - (2 - hydroxyethyl) - 3 - [1 - methyl - 1 - (4 - mitrobenzyloxycarbonyloxy)ethyl]azetidin - 2 - one (151 mg).

IR ($CH_2Cl_2$): 3575, 3375, 1750, 1520, 1350 $cm^{-1}$.

NMR (DMSO-$d_6$) δ: 1.57 (s, 3H), 1.64 (s, 3H), 1.88 (q, 2H, J=7Hz), 3.1—3.6 (m, 3H), 3.6—3.9 (m, 1H), 4.47 (t, 1H, J=5Hz), 5.23 (s, 2H), 7.90 ($A_2B_2$, 4H, J=9Hz), 8.08 (s, 1H).

Preparation 66

OCOOPNB
(CH3)2C
H
H
NH
OH
O
OCOOPNB
(CH3)2C
H
H
COOH
NH
O

To a solution of (3R, 4R) - 4 - (2 - hydroxyethyl) - 3 - [1 - methyl - 1 - (4 - nitrobenzyloxycarbonyloxy)ethyl]azetidin - 2 - one (10 mg) in acetone (0.6 ml) was added 2N Jones reagent (50 μl) at 0°C and the mixture was stirred at 0°C for 20 minutes. Isopropyl alcohol (100 μl) was added to the mixture at 0°C and the mixture was evaporated in vacuo. The residue was dissolved in chloroform (10 ml) and the solution was washed with brine. The aqueous layer was separated and extracted with chloroform (5 ml). The combined organic layers were washed with aqueous saturated sodium chloride, dried over magnesium sulfate, and evaporated in vacuo. The residue was chromatographed on silica gel (0.5 g) eluting with a mixture of methylene chloride and methanol (20:1 to 5:1) to give pure {(2R, 3R) - 3 -

[1 - methyl - 1 - (4 - nitrobenzyloxycarbonyloxy)ethyl] - 4 - oxoazetidin - 2 - yl} acetic acid (3 mg) and impure material (5 mg).

IR ($CH_2Cl_2$): 1735, 1520, 1350 $cm^{-1}$.

Preparation 67

OCOOPNB
$(CH_3)_2C$
H
H
NH
OH
O
OCOOPNB
$(CH_3)_2C$
H
H
CHO
NH
O


A solution of (3R, 4R) - 4 - (2 - hydroxyethyl) - 3 - [1 - methyl - 1 - (4 - nitrobenzyloxycarbonyloxy)ethyl]azetidin - 2 - one (10 mg) in dichloromethane (1.5 ml) was added to a suspension of pyridinium chlorochromate (9.2 mg) in dichloromethane at 0°C. The stirring mixture was allowed to warm to ambient temperature and kept at the same temperature for 1.5 hours. The reaction mixture was poured into a mixture of dichloromethane (20 ml) and dilute hydrochloric acid. The organic layer was separated and washed in turn with brine, dilute aqueous solution of sodium bicarbonate containing sodium thiosulfate, and brine. The organic layer was dried over magnesium sulfate and evaporated to give a crystalline solid (12 mg). A silica gel chromatography (0.5 g) eluting with a mixture of dichloromethane and acetone (10:1 to 5:1) afforded (3R, 4R) - 3 - [1 - methyl - 1 - (4 - nitrobenzyloxycarbonyloxy)ethyl) - 4 - (2 - oxoethyl)azetidin - 2 - one (9.7 mg) as a crystalline solid.

IR ($CH_2Cl_2$): 3375, 1760, 1750 (shoulder), 1715, 1520, 1350 $cm^{-1}$.

Preparation 68

OCOOPNB
$(CH_3)_2C$
H
H
CHO
NH
O
OCOOPNB
$(CH_3)_2C$
H
H
COOH
NH
O


To a solution of (3R, 4R) - 3 - [1 - methyl - 1 - (4 - nitrobenzyloxycarbonyloxy)ethyl] - 4 - (2 - oxoethyl)azetidin - 2 - one (9 mg) in acetone (1 ml) was added 2N Jones reagent (60 µl) at 0°C and the mixture was stirred at 0°C for 30 minutes. After addition of isopropyl alcohol (100 µl), the mixture was evaporated in vacuo. The residue was taken up in chloroform (20 ml) and washed with brine. The aqueous layer was extracted with chloroform (5 ml). The combined organic layers were washed with aqueous saturated sodium chloride, dried over magnesium sulfate, and evaporated to give {(2R, 3R) - 3 - [1 - methyl - 1 - (4 - nitrobenzyloxycarbonyloxy)ethyl] - 4 - oxoazetidin - 2 - yl}acetic acid (7 mg).

IR ($CH_2Cl_2$): 1735, 1520, 1350 $cm^{-1}$.

Preparation 69

$CH_2OCONH$
H
H
$CH(OCH_3)_2$
NH
O
$CH_2OCONH$
H
H
NH
OH
O


To a solution of (3R, 4R) - 3 - (benzyloxycarbonylamino) - 4 - (2,2 - dimethoxyethyl)azetidin - 2 - one (1.014 g) in tetrahydrofuran (15 ml) were added portionwise a solution of sodium cyanoborohydride (460 mg) in tetrahydrofuran (2.30 ml) and 2N hydochloric acid (7.3 ml) at ambient temperature during a period of 90 minutes. The solution was adjusted to pH 7 with saturated aqueous sodium hydrogen carbonate and concentrate in vacuo. The concentrate was acidified to pH 4 with 2N hydrochloric acid, saturated with sodium chloride and extracted four times with chloroform. The combined extracts were dried over magnesium sulfate and evaporated in vacuo. The residue was chromatographed on silica gel (15 g, eluting with 3—10% methanol in dichloromethane) to give a crystalline solid, which was washed with ether and collected by filtration to give 553 mg of (3R, 4R) - 3 - benzyloxycarbonylamino - 4 - (2 - hydroxyethyl)azetidin - 2 - one.

IR ($CH_2Cl_2$): 1770, 1720 $cm^{-1}$.

Preparation 70

Ozone was bubbled into a solution of methyl 2 - [(3R, 4R) - 3 - benzyloxycarbonylamino - 2 - oxo - 4 - (prop - 2 - enyl)azetidin - 1 - yl) - 3 - methylbut - 2 - enoate (119 mg) in ethyl acetate (6 ml) at −78°C until a permanent blue color developed. The excess ozone was removed by a stream of nitrogen and dimethyl sulfide (0.3 ml) was added. The mixture was allowed to warm to 4°C, kept in a refrigerator (4°C) for 7 hours and left at room temperature for 39 hours. The yellow solution was concentrated under reduced pressure and the residue was dissolved in methanol (4 ml). Aqueous potassium hydrogen carbonate (1.0M, 0.4 ml) was added therein and the resulting mixture was stirred at room temperature for 30 minutes. The solution was then cooled to 0°C and sodium borohydride (22 mg) was added to the solution. After 17 minutes stirring, acetic acid (0.15 ml) was added to the mixture and the mixture was evaporated in vacuo. The residue was dissolved in chloroform and washed with saturated aqueous sodium choride containing sodium hydrogen carbonate. The aqueous layer was extracted twice with chloroform. The combined extracts were dried over magnesium sulfate and evaporated in vacuo. The residue was chromatographed on silica gel (2 g, eluting with 5—10% methanol in dichloromethane) to give 50 mg of (3R, 4R) - 3 - benzyloxycarbonylamino - 4 - (2 - hydroxyethyl)azetidin - 2 - one.

IR ($CH_2Cl_2$): 1770, 1720 $cm^{-1}$.

Preparation 71

Ozone was bubbled into a solution of methyl 2 - [(3R, 4R) - 3 - benzyloxycarbonylamino - 2 - oxo - 4 - (prop - 2 - enyl)azetidin - 1 - yl] - 3 - methylbut - 2 - enoate (382 mg) in methanol (8 ml) at −78°C until a permanent blue color developed. After five minutes stirring the excess ozone was removed by a stream of nitrogen. Dimethylsulfide (0.4 ml) was added and the solution was stirred at −78°C for 35 minuites. Sodium borohydride (80 mg) was then added the solution and the reaction was continued for 40 minutes at the same temperature and quenched by addition of acetic acid (0.15 ml). The resulting mixture was stirred at −78°C for 30 minutes, allowed to warm to 0°C during 50 minutes and concentrated in vacuo. The residue was dissolved in a 10:1 mixture of ethyl acetate and chloroform (30 ml) and washed in turn with saturated aqueous sodium chloride (twice), a mixture of saturated aqueous sodium chloride and saturated aqueous sodium hydrogen carbonate, and saturated aqueous sodium chloride. The resultant mixture was dried over magnesium sulfate and concentrated under reduced pressure to give 352 mg of methyl 2 - [(3R, 4R) - 3 - benzyloxycarbonylamino - 2 - oxo - 4 - (2 - oxoethyl)azetidin - 1 - yl] - 2 - hydroxyacetate.

IR ($CH_2Cl_2$): 3300—3700 (br), 3380, 1770, 1740 (sh) 1720 $cm^{-1}$.

NMR ($CDCl_3$) δ: 2.0 (1H, br), 2.97 (2H, brd, J=6Hz), 3.72 (3H, s), 4.16 (1H, dt, J=2, 6Hz), 4.47 (1H, dd, J=2, 7Hz), 5.06 (2H, s), 5.52 (1H, brs), 5.91 (1H, d, J=7Hz), 7.28 (5H, s), 9.65 (1H, s).

The aldehyde (318 mg) obtained above was dissolved in methanol (5 ml) and the solution was cooled to 0°C. 1N aqueous potassium hydrogen carbonate (0.5 ml) was added and the mixture was stirred at 0°C for 5 minutes and at ambient temperature for 30 minutes. Sodium borohydride (38 mg) was then added to the mixture at 0°C and the mixture was stirred at the same temperature for 15 minutes and quenched by

addition of acetic acid (0.15 ml). After concentration in vacuo, the residue was dissolved in chloroform and washed with brine containing sodium hydrogen carbonate. The aqueous layer was extracted three times with chloroform. The combined extracts were dried over magnesium sulfate and evaporated in vacuo to give a crystalline solid, which was chromatographed on silica gel (6 g, eluting with 3—10% methanol in dichloromethane) to give 113 mg of (3R, 4R) - 3 - benzyloxycarbonylamino - 4 - (2 - hydroxyethyl)azetidin - 2 - one.

IR ($CH_2Cl_2$): 1770, 1720 $cm^{-1}$.

Preparation 72

Ozone was bubbled into a solution of methyl 2 - [(3R, 4R) - 3 - benzyloxycarbonylamino - 2 - oxo - 4 - (prop - 2 - enyl)azetidin - 1 - yl] - 3 - methylbut - 2 - enoate (255 mg) in ethyl acetate (6.5 ml) at −78°C until a permanent blue color developed. The excess ozone was removed by a stream of nitrogen. The solution was allowed to warm to 0°C and washed with dilute aqueous sodium hydrogen sulfite and brine. The resultant mixture was dried over magnesium sulfate and concentrated under reduced pressure to give 251 mg of methyl 2 - [(3R, 4R) - 3 - benzyloxycarbonylamino - 2 - oxo - 4 - (1,2,4-trioxolan - 3 - yl)methylazetidin - 1 - yl] - 2 - oxoacetate.

IR ($CH_2Cl_2$): 3390, 1810, 1745, 1720, 1705 $cm^{-1}$.

Preparation 73

To a solution of methyl 2 - [(3R, 4R) - 3 - benzyloxycarbonylamino - 2 - oxo - 4 - (1,2,4 - trioxolan - 3 - yl)methylazetidin - 1 - yl] - 2 - oxoacetate (205 mg) in methanol (4 ml) was added 1N aqueous potassium hydrogen carbonate (0.5 ml) at ambient temperature. The mixture was stirred for 25 minutes and cooled to 0°C. Sodium borohydride (40 mg) and 1N aqueous potassium hydrogen carbonate were added to the mixture at 0°C. The reaction mixture was stirred at 0°C for 30 minutes and at ambient temperature for an additional 30 mintues, and quenched by addition of acetic acid (0.20 ml). The resulting mixture was concentrated under reduced pressure and the residue was dissolved in chloroform and washed with brine containing sodium hydrogen carbonate. The aqueous layer was extracted three times with chloroform. The combined extracts were dried over magnesium sulfate, evaporated in vacuo, and chromatographed on silica gel (3 g, eluting with 10—30% acetone in dichloromethane) to give 66 mg of (3R, 4R) - 3 - benzyloxycarbonylamino - 4 - (2 - hydroxyethyl)azetidin - 2 - one.

IR ($CH_2Cl_2$): 1770, 1720 $cm^{-1}$.

Preparation 74

A solution of osmium tetroxide (0.61 mg) in water (0.05 ml) was added to a solution of methyl 2 - [(3R, 4R) - 3 - benzyloxycarbonylamino - 2 - oxo - 4 - (prop - 2 - enyl)azetidin - 1 - yl] - 3 - methylbut - 2 - enoate (58 mg) and sodium chlorate (50 mg) in a mixture of tetrahydrofuran (1 ml) and water (1 ml) and the

mixture was stirred for 17 hours at ambient temperature. Sodium periodate (60 mg) was then added and stirring was continued for 30 minutes. The resulting mixture was diluted with brine and extracted with ethyl acetate. The extract was washed successively with brine, brine containing sodium bicarbonate, and brine. The mixture was dried over magnesium sulfate and concentrated under reduced pressure to give an oil, which was chromatographed on silica gel (1.5 g, eluting with 2.5% methanol in dichloromethane) to give 49 mg of methyl 2 - [(3R, 4R) - 3 - benzyloxycarbonylamino - 2 - oxo - 4 - (2 - oxoethyl)azetidin - 1 - yl) - 3 - methylbut - 2 - enoate.

IR ($CH_2Cl_2$): 3420, 1765, 1725 $cm^{-1}$.

Preparation 75

A solution of osmium tetroxide (0.61 mg) in water (0.05 ml) was added to a mixture of methyl 2 - [(3R, 4R) - 3 - benzyloxycarbonylamino - 2 - oxo - 4 - (prop - 2 - enyl)azetidin - 1 - yl] - 3 - methylbut - 2 - enoate (106 mg) and sodium periodate (36.5 mg) in a mixture of tetrahydrofuran (1 ml) and water (1 ml). After 25 minutes stirring at ambient temperature, additional sodium periodate (101.2 mg) was added and the mixture was stirred at ambient temperature for 4.5 hours. The resulting mixture was diluted with brine containing sodium bicarbonate and extracted twice with dichloromethane. The combined extracts were dried over magnesium sulfate and evaporated to give 110 mg of methyl 2 - [(3R, 4R) - 3 - benzyloxycarbonylamino - 2 - oxo - 4 - (2 - oxoethyl)azetidin - 1 - yl] - 3 - methylbut - 2 - enoate as an oil.

IR ($CH_2Cl_2$): 3420, 1765, 1725 $cm^{-1}$.

Preparation 76

Sodium borohydride (10 mg) was added to a solution of methyl 2 - [(3R, 4R) - 3 - benzyloxycarbonylamino - 2 - oxo - 4 - (2 - oxoethyl)azetidin - 1 - yl] - 3 - methylbut - 2 - enoate (49 mg) in methanol (1 ml) at 0°C. The mixture was stirred at the same temperature for 20 minutes and quenched with acetic acid (~0.05 ml). The resulting solution was evaporated in vacuo. The residue was disssolved in dichloromethane and washed with brine containing sodium bicarbonate. The extract was dried over magnesium sulfate, evaporated, and chromatographed on silica gel (1.5 g, eluting with 5—10% acetone in dichloromethane) to give 39 mg of methyl 2 - [(3R, 4R) - 3 - benzyloxycarbonylamino - 4 - (2 - hydroxyethyl) - 2 - oxoazetidin - 1 - yl] - 3 - methylbut - 2 - enoate.

IR ($CH_2Cl_2$): 1765, 1720 (br) $cm^{-1}$.

NMR ($CDCl_3$): 1.7—2.2 (2H, m), 1.90 (3H, s), 2.17 (3H, s), 3.4—4.0 (4H, m), 3.71 (3H, s), 4.56 (1H, dd, J=2, 7Hz), 5.11 (2H, s), 6.10 (1H, m), 7.33 (5H, s).

Preparation 77

A stream of ozone was bubbled into a solution of methyl 2 - [(3R, 4R) - 3 - benzyloxycarbonylamino - 4 - (2 - hydroxyethyl) - 2 - oxoazetidin - 1 - yl] - 3 - methylbut - 2 - enoate (76 mg) in methanol (6 ml) at

−78°C until the starting material disappeared. The excess ozone was removed by a stream of nitrogen. Dimethyl sulfide (0.3 ml) was added and the resulting solution was allowed to warm to ambient temperature for five hours. The solution was cooled to 0°C, to which a solution of sodium methoxide in methanol (0.1M, 0.25 ml) was added. The mixture was stirred at 0°C for 30 minutes and quenched by addition of acetic acid (excess). The solution was evaporated in vacuo and the residue was chromatographed on silica gel (1.5 g, eluting with 2.5—10% methanol in dichloromethane) to give 46 mg of a crystalline solid, which was triturated in diethyl ether and filtered to give 40 mg of (3R, 4R) - 3 - benzyloxycarbonylamino - 4 - (2 - hydroxyethyl)azetidin - 2 - one.

IR ($CH_2Cl_2$): 1770, 1720 $cm^{-1}$.

Preparation 78

A stream of ozone was bubbled into a solution of methyl 2 - [(3R, 4R) - 3 - benzyloxycarbonylamino - 2 - oxo - 4 - (prop - 2 - enyl)azetidin - 1 - yl] - 3 - methylbut - 2 - enoate (85.6 g) in methanol (1.54 l) at −78°C until a permanent blue color developed. After 30 minutes the excess ozone was removed by purging nitrogen into the solution and dimethyl sulfide (85.6 ml) was then added. The solution was allowed to warm to 4°C, kept at the same temperature for 15 hours and left at ambient temperature for 24 hours. To the resulting solution were added methyl orthoformate (171.2 ml) and p-toluenesulfonic acid monohydrate (1.18 g). The solution was heated at 50°C for three hours, cooled to ambient temperature, neutralized with pyridine (0.71 ml) and concentrated in vacuo. The residue was dissolved in ethyl acetate (1.5 l) and washed with brine containing 1N hydrochloric acid, and brine. The aqueous layers were combined and extracted with ethyl acetate. The combined organic layers were washed with brine, brine containing sodium bicarbonate, and brine, dried over magnesium sulfate and evaporated in vacuo. The residue was dissolved in methanol (1.54 l) and cooled to 4°C. A 4.9M methanolic sodium methoxide (0.151 ml) was then added and the resultant solution was stirred for 15 minutes, quenched with acetic acid (0.151 ml) and concentrated under reduced pressure. The residue was dissolved in ethyl acetate, washed in turn with brine containing sodium bicarbonate and brine, dried over magnesium sulfate, and evaporated in vacuo. The residue was subjected to column chromatography on silica gel (1 kg, eluting with 10 to 30% acetone in dichloromethane). The eluate was concentrated under reduced pressure to give a residue, which was crystallized from a 1:1 mixture of diethyl ether and diisopropyl ether to give 48.23 g of (3R, 4R) - 3 - benzyloxycarbonylamino - 4 - (2,2 - dimethoxyethyl)azetidin - 2 - one.

IR ($CH_2Cl_2$): 1770, 1720 $cm^{-1}$.

Preparation 79

A solution of (6R, 7RS) - 2,2 - dimethyl - 7 - (1 - hydroxy - 1 - methylethyl) - 7 - isocyano - 1 - aza - 3 - oxabicyclo[4.2.0]octan - 8 - one (920 mg), triphenyltin hydride (1.694 g) and azobisisobutyronitrile (63.4 mg) in benzene (46 ml) was heated at 50°C for thirty minutes under a nitrogen atmosphere. The mixture was cooled to ambient temperature. The precipitates were filtered off and washed with benzene. The filtrate and washings were combined and chromatographed on silica gel (27.6 g, eluting with 10 to 25% acetone in dichloromethane) to give a crude product mixture, which was re-chromatographed on silica gel (46 g; 230—400 mesh, eluting with a 6:1 to 1:2 mixture of hexane and ethyl acetate) to give 513 mg of (6R, 7R) - 2,2 - dimethyl - 7 - (1 - hydroxy - 1 - methylethyl) - 1 - aza - 3 - oxabicyclo[4.2.0]octan - 8 - one after crystallization from diisopropyl ether and 170 mg of its 7S isomer as a crystalline solid.

Preparation 80

Ozone was bubbled into a solution of methyl 3 - methyl - 2 - {(3R, 4R) - 3 - [1 - methyl - 1 - (4 - nitrobenzyloxycarbonyloxy)ethyl] - 2 - oxo - 4 - (prop - 2 - enyl)azetidin - 1 - yl}but - 2 - enoate (100 mg) in a mixture of methanol (3 ml) and ethyl acetate (3 ml) at −70°C until a blue color appeared. After stirring for 20 minutes at −70°C, the excess ozone was removed by a stream of nitrogen at the same temperature and dimethyl sulfide (1 ml) was added to the mixture, which was allowed to warm to ambient temperature over a period of an hour and to stand at 20°C for 20 hours. After evaporation of the solvent, the residue was dissolved in methanol (5 ml). Trimethyl orthoformate (0.5 ml), dimethyl sulfide (0.3 ml) and p-toluenesulfonic acid monohydrate (4.1 mg) were added to the solution and the mixture was heated at 50°C for 50 minutes. After cooling, pyridine (3 drops) was added to the mixture. The resulting solution was evaporated in vacuo, dissolved in ethyl acetate (20 ml) and washed in turn with water, 0.1N hydrochloric acid, water, aqueous sodium bicarbonate and brine. The ethyl acetate layer was dried over magnesium sulfate and evaporated in vacuo. The residue was dissolved in methanol (5 ml) and the solution was heated at 50°C for 5 hours. After evaporation of the solvent, the residue was chromatographed on silica gel (2 g) eluting with a mixture of methylene chloride and ethyl acetate (10:1 to 1:2) to give (3R, 4R) - 4 - (2,2 - dimethoxyethyl) - 3 - [1 - methyl - 1 - (4 - nitrobenzyloxycarbonyloxy)ethyl]azetidin - 2 - one (75 mg) as an oil.

IR ($CH_2Cl_2$): 3400, 1760, 1520, 1350 $cm^{-1}$.

NMR ($CDCl_3$) δ: 1.70 (3H, s), 1.78 (3H, s), 2.0—2.5 (2H, m), 3.42 (6H, s), 3.67 (1H, d, J=5Hz), 3.8—4.1 (1H, m), 4.55 (1H, t, J=5Hz), 5.31 (2H, s), 6.56 (1H, s), 7.68 (2H, d, J=9Hz), 8.38 (2H, d, J=9Hz).

Preparation 81

A solution of (3R, 4R) - 4 - (2,2 - dimethoxyethyl) - 3 - [1 - methyl - 1 - (4 - nitrobenzyloxycarbonyloxy)ethyl]azetidin - 2 - one (64 mg) in 80% aqueous acetic acid (3.0 ml) was heated at 50—55°C for 4 hours. After evaporation of the solvent, the residue was dissolved in ethyl acetate (20 ml) and the solution was washed with aqueous sodium bicarbonate. The aqueous layer was extracted with ethyl acetate (10 ml). The organic layers were combined, washed with water and brine, dried over magnesium sulfate and evaporated in vacuo to give (3R, 4R) - 3 - (1 - methyl - 1 - (4 - nitrobenzyloxycarbonyloxy)ethyl) - 4 - (2 - oxoethyl)azetidin - 2 - one (55 mg) as a solid.

IR ($CH_2Cl_2$): 1735, 1520, 1350 $cm^{-1}$.

Preparation 82

A solution of (3R, 4R) - 4 - (2 - hydroxyethyl) - 3 - [1 - methyl - 1 - (4 - nitrobenzyloxycarbonyloxy)ethyl]azetidin - 2 - one (50 mg) in acetone (3 ml) was added to a solution of 2N Jones reagent (0.284 ml) in acetone (3 ml) at ambient temperature over a period of 30 minutes and the mixture was stirred for 30 minutes. After addition of an excess of isopropyl alcohol to the mixture, the solvent was evaporated off. The residue was dissolved in chloroform and washed with water. The aqueous layer was extracted twice with chloroform. The organic layers were combined, washed with brine, dried over magnesium sulfate and evaporated in vacuo. The residue was dissolved in chloroform (5 ml) and the solution was extracted with

0.1N aqueous solution of sodium bicarbonate (2 ml), 0.05N aqueous solution of sodium bicarbonate (2 ml) and water. The aqueous extracts were combined, acidified with 1N hdyrochloric acid and extracted three times with chloroform. The combined chloroform extracts were washed with brine, dried over magnesium sulfate and evaporated to give {(2R, 3R) - 3 - (1 - methyl - 1 - (4 - nitrobenzyloxycarbonyloxy)ethyl] - 4 - oxoazetidin - 2 - yl}acetic acid (56 mg).

IR ($CH_2Cl_2$): 1735, 1520, 1350 $cm^{-1}$.

Preparation 83

To a solution of methyl 2 - {(3R, 4R) - 3 - [1 - methyl - 1 - (4 - nitrobenzyloxycarbonyloxy)ethyl] - 2 - oxo - 4 - (prop - 2 - enyl)azetidin - 1 - yl} - 3 - methylbut - 2 - enoate (50 mg) in acetone (2.79 ml) were added water (0.93 ml) and a 0.7M solution of phosphate buffer (pH 6.8, 1.86 ml) at ambient temperature. Sodium periodate (139 mg) was added to the resulting suspension at ambient temperature and the mixture was stirred for 20 minutes. Potassium permanganate (26 mg) was then added and the resulting mixture was stirred for 26 hours at ambient temperature. The mixture was acidified with 2N hydrochloric acid (2 ml) and filtered using diatomaceous earth, and the filter-cake was washed with acetone and water. The filtrates were combined and concentrated under reduced pressure to a half volume. The concentrate was extracted three times with chloroform. The organic layers were combined, washed with brine and extracted with 5% aqueous sodium bicarbonate and water. The combined aqueous extracts were acidified with 2N hydrochloric acid and extracted three times with chloroform. The organic extracts were combined, washed with brine, dried over magnesium sulfate and evaporated in vacuo to give {(2R, 3R) - 3 - [1 - methyl - 1 - (4 - nitrobenzyloxycarbonyloxy)ethyl] - 4 - oxoazetidin - 2 - yl}acetic acid (16 mg).

IR ($CCH_2Cl_2$): 1735, 1520, 1350 $cm^{-1}$.

Preparation 84

Pyridinium dichromate (3.74 g) was added to a solution of (3R, 4R) - 4 - (2 - hydroxyethyl) - 3 - [1 - methyl - 1 - (4 - nitrobenzyloxycarbonyloxy)ethyl] - 2 - oxo - azetidine (1.00 g) in N,N-dimethylformamide (20 ml) at 0°C, and the stirring mixture was allowed to warm to ambient temperature. After stirring at ambient temperature for 16 hours, the reaction mixture was poured into a mixture of ethyl acetate (150 ml), 10% aqueous sodium bisulfite (40 ml) and 3N hydrochloric acid (15 ml). The organic layer was separated and the remaining aqueous layer was extracted with ethyl acetate (100 ml × 2). The organic layers were combined, washed in turn with water and an aqueous sodium chloride, dried over magnesium sulfate, and evaporated in vacuo.

The residue was dissolved in chloroform (30 ml) and the resultant solution was extracted with 5% aqueous sodium bicarbonate (20 ml), 2.5% aqeuous sodium bicarbonate (20 ml) and then water (15 ml × 2). The aqueous solutions were combined and acidified with 3N hydrochloric acid, followed by re-extraction three times with chloroform (20 ml). The combined extracts were washed with an aqueous sodium chloride, dried over magnesium sulfate, and evaporated to give {(2R, 3R) - 3 - [1 - methyl - 1 - (4 - nitrobenzyloxycarbonyloxy)ethyl] - 4 - oxo - azetidin - 2 - yl}acetic acid (810 mg).

IR ($CH_2Cl_2$): 1735, 1520, 1350 $cm^{-1}$.

Example 1

A) Preparation of the object compound:

1)

To a solution of [(2R, 3S) - 3 - [1 - methyl - 1 - (4 - nitrobenzyloxycarbonyloxy)ethyl] - 4 - oxoazetidin - 2 - yl]acetic acid (56.4 mg) in tetrahydrofuran (1.13 ml) was added N,N-carbonyldiimidazole (27.5 mg) at ambient temperature. After stirring for 6 hours at ambient temperature, the magnesium salts of the mono-p-nitrobenzyl ester of malonic acid (84.8 mg) was added and the resulting mixture was stirred overnight at ambient temperature. The solvent was distilled off and the residue was dissolved in ethyl acetate (30 ml) and filtered with a diatomaceous earth. The filtrate was washed with 1N hydrochloric acid, water, phosphate butter solution (pH 6.8), and saturated aqueous sodium chloride. After drying over magnesium sulfate, the ethyl acetate extract was filtered and evaporated in vacuo. The residual oil (78.8 mg) was chromatographed on silica gel (1.6 g) eluting with a mixture of methylene chloride and ethyl acetate (10:1 to 2:1) to give 4 - nitrobenzyl - 4 - {(2R, 3S) - 3 - [1 - methyl - 1 - (4 - nitrobenzyloxycarbonyloxy)ethyl] - 4 - oxoacetidine - 2 - yl} - 3 - oxobutanoate (64.2 mg) as an amorphous solid.

IR ($CH_2Cl_2$): 3380, 1760, 1740, 1720, 1520, 1340 $cm^{-1}$.

NMR ($CDCl_3$) δ: 1.62 (s, 3H), 1.66 (s, 3H), 2.86—3.12 (m, 2H), 3.41 (d, 1H, J=2Hz), 3.65 (s, 2H), 3.94—4.18 (m, 1H), 5.25 (s, 2H), 5.34 (s, 2H), 6.60 (broad s, 1H), 7.63 (d, 4H, J=9Hz), 8.30 (d, 4H, J=9Hz).

2)

To a solution of 4-nitrobenzyl 4 - {(2R, 3S) - 3 - [1 - methyl - 1 - (4 - nitrobenzyloxycarbonyloxy)ethyl] - 4 - oxoazetidine - 2 - yl} - 3 - oxobutanoate (58.3 mg) in acetonitrile (1.17 ml) was added a solution of p-toluenesulfonyl azide (25.3 mg) in acetonitrile (0.228 ml) at 0°C. After stirring for 5 minutes at 0°C, a solution of triethylamine (53.7 µl) in acetonitrile (0.483 ml) was added dropwise. The stirring mixture was allowed to warm to ambient temperature during 15 minutes and kept at the same temperature for 20 minutes. The solvent was distilled off and the residue (85.9 mg) was flash chromatographed on silica gel (1 g) eluting with a mixture of methylene chloride and ethyl acetate (50:1 to 3:1) to give 4 - nitrobenzyloxycarbonyloxy)ethyl] - 4 - oxoazetidine - 2 - yl} - 3 - oxobutanoate (58.4 mg) as an amorphous solid.

IR ($CH_2Cl_2$): 3380, 2140, 1760, 1740, 1715, 1645, 1520, 1345 $cm^{-1}$.

NMR ($CDCl_3$) δ: 1.63 (s, 3H), 1.67 (s, 3H), 2.88—3.34 (m, 2H), 3.46 (d, 1H, J=2Hz), 3.90—4.20 (m, 1H), 5.26 (s, 2H), 5.45 (s, 2H), 6.48 (broad s, 1H), 7.65 (d, 4H, J=9Hz), 8.30 (d, 2H, J=9Hz), 8.34 (d. 2H, J=9Hz).

3)

A mixture of 4-nitrobenzyl 2 - diazo - 4 - {(2R, 3S) - 3 - [1 - methyl - 1 - (4 - nitrobenzyloxycarbonyloxy)ethyl] - 4 - oxoazetidine - 2 - yl} - 3 - oxobutanoate (58.0 ml) and rhodium (II) acetate (0.1 mg) in benzene (3.4 ml) was refluxed for 30 minutes. After cooling to ambient temperature, the mixture was filtered with a diatomaceous earth and evaporated to give 4 - nitrobenzyl(2R, 5R, 6S) - 3,7 - dioxo - 6 - [1 - methyl - 1 - (4 - nitrobenzyloxycarbonyloxy)ethyl] - 1 - azabicyclo[3.2.0]heptane - 2 - carboxylate (47.8 mg) as an amorphous solid.

IR ($CH_2Cl_2$): 1760, 1735, 1730, 1515, 1345 $cm^{-1}$.

NMR ($CDCl_3$) δ: 1.70 (s, 3H), 2.49 (AB part of ABX system centered at 2.49, 2H, JAB=18Hz, JAX=JBX=7Hz), 3.78 (d, 1H, J=2Hz), 4.21 (dt, 1H, J=2, 7Hz), 4.79 (s, 1H), 5.21 (s, 2H), 5.30 (ABq, 1H, J=12Hz), 7.53 (d, 2H, J=9Hz), 8.22 (d, 2H, J=9Hz).

B) Preparation of active end products starting from the object compound

1)

To a solution of 4-nitrobenzyl (2R, 5R, 6S) - 3,7 - dioxo - 6 - [1 - methyl - 1 - (4 - nitrobenzyloxycarbonyloxy)ethyl] - 1 - azabicyclo[3.2.0]heptane - 2 - carboxylate (47.8 mg) and 4-(N,N-dimethylamino)pyridine (1.08 mg) in acetonitrile (2.39 ml) was added dropwise a solution of di-isopropylethylamine (18.5 μl) in acetonitrile (166.5 μl) followed by addition of a solution of diphenyl chlorophosphate (19.2 μl) in acetonitrile (172.8 μl) at 0°C. After stirring for an hour at 0°C, a solution of di-isopropylethylamine (61.5 μl) in acetonitrile (553.5 μl) was added followed by addition of a solution of N-(2-mercaptoethyl)acetamide (11.05 mg) in acetonitrile (99.45 μl) at 15°C. The mixture was allowed to stand overnight at −15°C. The resulting solution was diluted with ethyl acetate (34 ml) and washed with dilute aqueous sodium chloride and saturated aqueous sodium chloride. After drying over magnesium sulfate, the ethyl acetate extract was filtered and evaporated in vacuo. The residual oil (76.4 mg) was chromatographed on silica gel (1.5 g) eluting with a mixture of methylene chloride and acetone (20:1 to 2:1) to give 4 - nitrobenzyl(5R, 6S) - 3 - (2 - acetamido - ethylthio) - 6 - [1 - methyl - 1 - (4 - nitrobenzyloxycarbonyloxy)ethyl] - 7 - oxo - 1 - aza - bicyclo[3.2.0]hept - 2 - ene - 2 - carboxylate (54.0 mg) as an amorphous solid.

IR ($CH_2Cl_2$): 3410, 1775, 1725, 1710, 1685, 1520, 1350 $cm^{-1}$.

NMR ($CDCl_3$) δ: 1.65 (s, 6H), 1.98 (s, 3H), 2.7—3.6 (m, 6H), 3.77 (d, 1H, J=3Hz), 4.27 (dt, 1H, J=3, 8Hz), 5.18 (s, 2H), 5.33 (ABq centered at 5.33, 2H, J=13Hz), 6.21 (broad s, 1H), 7.50 (d, 2H, J=9Hz), 7.58 (d, 2H), J=9Hz), 8.14 (d, 2H, J=9Hz), 8.16 (d, 2H, J=9Hz).

2)

A mixture of 5% palladium -charcoal (40.0 mg), water (0.96 ml) and dioxane (2.24 ml) was shaken for 30 minutes under hydrogen atmosphere (40 psi) at ambient temperature. The mixture was added to a solution of 4-nitrobenzyl (5R, 6S) - 3 - (2 - acetamidoethylthio) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (40.0 mg) in a mixture of water (0.24 ml) and dioxane (0.56 ml), and shaken for 2 hours under hydrogen atmosphere (2.76 bar, 40 psi) at ambient temperature. The resulting mixture was added with 1% aqueous sodium hydrogen carbonate (0.522 ml) at 0°C and filtered by using diatomaceous earth. The filtered cake was washed with water (3 × 5 ml) and the combined filtrates were concentrated, washed with ethyl acetate (3 × 20 ml) and evaporated in vacuo. The residue was dissolved in water (25 ml) containing sodium chloride (0.7 g) and chromatographed on a non-ionic adsorption resin HP—20 (trademark, made by Mitsubishi Chemical Industries) (1.0 × 20 cm) eluting with a mixture of water and acetone (100:0 to 7:3, 200 ml). The fractions containing the product were combined and lyophilized to give sodium (5R, 6S) - 3 - (2 - acetamido - ethylthio) - 6 - (1 - hydroxy - 1 - methylethyl) - 7 - oxo - 1 - azabicyclo[3.2.0]hept - 2 - ene - 2 - carboxylate (6.9 mg) as a white solid.

IR ($CH_2Cl_2$): 3310, 1745, 1655, 1600, 1555, 1400 $cm^{-1}$. UV (in $H_2O$):301 nm.

3)

To a solution of 4-nitrobenzyl (2R, 5R, 6S) - 3,7 - dioxo - 6 - [1 - methyl - 1 - (4 - nitrobenzyloxycarbonyloxy)ethyl] - 1 - azabicyclo[3.2.0]heptane - 2 - carboxylate (148.5 mg) and 4-N,N-dimethylaminopyridine (3.35 mg) in acetonitrile (7.4 ml) was added dropwise a solution of di-isopropylethylamine (57.3 μl) in acetonitrile (0.516 ml). To the mixture was further added a solution of diphenyl chlorophosphate (59.6 μl) in acetonitrile (0.536 ml) at 0°C. After stirring for an hour at 0°C, a solution of diisopropylethylamine (190.9 μl) in acetonitrile (1.72 ml) was added to the mixture. To the mixture was further added a solution of N - (4 - nitrobenzyloxycarbonyl)cysteamine (73.8 mg) in acetonitrile (0.664 ml) at −15°C. The mixture was allowed to stand overnight at −15°C. The resulting solution was diluted with ethyl acetate (15 ml) and washed in turn with water and saturated aqeuous sodium chloride. After drying over magnesium sulfate, the ethyl acetate extract was filtered and evaporated in vacuo. The residue was chromatographed on silica gel (3 g) eluting with a mixture of methylene chloride and ethyl acetate (50:1 to 5:1) to give 4 - nitrobenzyl[5R, 6S) - 6 - [1 - methyl - 1 - (4 - nitrobenzyloxycarbonyloxy)ethyl] - 3-[2 - (4 - nitrobenzyloxycarbonylamino)ethylthio] - 7 - oxo - 1 - azabicyclo[3.2.0]hept - 2 - ene - 2 - carboxylate (167.8 mg) as an amorphous solid.

IR ($CH_2Cl_2$): 3390, 1775, 1730, 1725, 1610, 1520, 1350 $cm^{-1}$.

NMR ($CDCl_3$) δ: 1.62 (6H, s), 2.80—3.60 (6H, m), 3.78 (1H, d, J=3.0Hz), 4.29 (1H, dt, J=3.8Hz), 5.17 (2H, s), 5.34 (4H, ABq, J=14Hz), 7.30—7.80 (6H, m), 8.05—8.30 (6H, m).

4)

OCOOPNB, $(CH_3)_2C$, H, H, $S(CH_2)_2NHCOCH_3$, N, O, COOPNB → OCOOPNB, $(CH_3)_2C$, H, H, O, ↑, $S(CH_2)_2NHCOCH_3$, N, O, COOPNB

To a solution of 4-nitrobenzyl (5R, 6S) - 3 - (2 - acetamidoethylthio) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (17.6 mg) in methylene chloride (0.70 ml) was added dropwise a solution of m-chloroperbenzoic acid (6.50 mg) in methylene chloride (0.319 ml) over a period of 5 minutes at −20°C. After stirring for 30 minutes at −20°C, the reaction mixture was diluted with ethyl acetate (10 ml), washed in turn with chilled 10% aqueous sodium hydrogen carbonate (0.5 ml) and saturated aqueous sodium chloride, filtered and concentrated under reduced pressure to give an oil (20.6 mg). The residual oil was chromatographed on silica gel (400 mg) eluting with a mixture of methylene chloride and acetone (10:1 to 2:1) to give 4-nitrobenzyl (5R, 6S) - 3 - (2 - acetamidoethylsulfinyl) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (15.9 mg) as an oil.

IR ($CH_2Cl_2$): 1780, 1740, 1705, 1670, 1605, 1530, 1350 $cm^{-1}$.

NMR ($CDCl_3$) δ: 1.27 (3H, s), 1.66 (3H, s), 2.02 (2.1H, s), 2.18 (0.9H, s), 2.50—4.05 (7H, m), 4.10—4.70 (1H, m), 5.18 (2H, s), 5.37 (2H, ABq, J=13Hz), 6.45 (1H, broad s), 7.30—7.80 (2H, m), 8.00—8.45 (2H, m).

5)

OCOOPNB, $(CH_3)_2C$, H, H, $S(CH_2)_2NHCOOPNB$, N, O, COOPNB → OH, $(CH_3)_2C$, H, H, $S(CH_2)_2NH_2$, N, O, COOK

A mixture of platinum oxide (60.0 mg), water (4.2 ml) aqueous dipotassium hydrogen phosphate (1.0M, 0.385 ml) and ethanol (0.6 ml) was shaken for 30 minutes under a hydrogen atmosphere (3.45 bar, 50 psi) at ambient temperature. The mixture was added to a solution of 4-nitrobenzyl (5R, 6S) - 6 - [1 - methyl - 1 - (4 - nitrobenzyloxycarbonyloxy)ethyl] - 3 - [2 - (4 - nitrobenzyloxycarbonylamino)ethylthio] - 7 - oxo - 1 - azabicyclo[3.2.0]hept - 2 - ene - 2 - carboxylate (60.0 mg) in dioxane (7.2 ml) and shaken for 30 minutes under a hydrogen atmosphere (3.45 bar, 50 psi) at ambient temperature. The reaction mixture was filtered with diatomaceous earth. The filter-cake was washed with water (20 ml) and the combined filtrates (32 ml) were lyophilized over a period of 6 hours. The residue was chromatographed on non-ionic adsorption resin, Diaion HP—20—AG (trade mark, made by Mitsubishi Chemical Industries) (15 × 260 mm) eluting with water (230 ml) and then a mixture of water and methanol (7:3, 140 ml). The fractions showing UV maxima at 298 nm were collected, concentrated and lyophilized to give potassium (5R, 6S) - 3 - (2 - aminoethylthio) - 6 - (1 - hydroxy - 1 - methylethyl) - 7 - oxo - 1 - azabicyclo[3.2.0]hept - 2 - ene - 2 - carboxylate (12.1 mg) as pale yellow powder.

IR (Nujol): 3350, 1750, 1575, 1380 $cm^{-1}$.

UV ($H_2O$): 298 nm (ε=7970).
NMR ($D_2O$) δ: 1.34 (3H, s), 1.38 (3H, s), 3.00—3.38 (6H, m), 3.47 (1H, d, J=3Hz), 4.22 (1H, dt, J=3.8Hz).

Example 2

A) Preparation of the object compound:

1)

N,N'-Carbonyldiimidazole (540 mg) was added to a solution of [(2R,3R)-3-{1-methyl-1-(4-nitrobenzyloxycarbonyloxy)ethyl}-4-oxoazetidin-2-yl]acetic acid (1.06 g) in tetrahydrofuran (50 ml) at ambient temperature. After stirring for 7 hours at the same temperature, magnesium salt of mono-p-nitrobenzyl malonate (1.45 g) was added thereto, and the resultant mixture was stirred overnight at ambient temperature. The solvent was distilled off in vacuo. The residue was dissolved in ethyl acetate (100 ml) and filtered. The filtrate was washed in turn with 0.1N hydrochloric acid, water, saturated aqueous sodium chloride, aqueous sodium bicarbonate, water, saturated aqueous sodium chloride, 5% aqueous citric acid, water and saturated aqueous sodium chloride. After drying over magnesium sulfate, the solution was evaporated. The residue was chromatographed on silica gel (30 g) eluting with a mixture of methylene chloride and ethyl acetate (50:1 to 2:1) to give formy substance, which was crystallized from a mixture of chloroform and diisopropyl ether, washed with diisopropyl ether and then dried in vacuo to give 4 - nitrobenzyl 4 - [(2R,3R) - 3 - {1 - methyl - 1 - (4 - nitrobenzyloxycabonyloxy)ethyl} - 4 - oxoazetidin - 2 - yl] - 3 - oxobutanoate (1.31 g), mp 123—125°C (dec.).

IR ($CH_2Cl_2$): 3400, 1760, 1750, 1720, 1525, 1350 $cm^{-1}$.

NMR (acetone-$d_6$) δ: 1.64 (3H, s), 1.74 (3H, s), 3.35 (2H, d, J=6.5Hz), 3.65 (1H, d, J=6Hz), 3.74 (2H, s), 4.20 (1H, m), 5.28 (2H, s), 5.31 (2H, s), 7.18 (1H, s), 7.93 (8H, $A_2B_2$, J=8Hz).

2)

A solution of p-toluenesulfonyl azide (435 mg) in acetonitrile (3.9 ml) was added to a solution of 4-nitrobenzyl 4 - [(2R,3R) - 3 - {1 - methyl - 1 - (4 - nitrobenzyloxycarbonyloxy)ethyl} - 4 - oxoaxetidin - 2 - yl] - 3 - oxobutanoate (1.00 g) in acetonitrile (20 ml) at 0°C. After stirring at 0°C for 5 minutes, triethylamine (0.023 ml) was added dropwise thereto. After stirring at 0°C for additional 20 minutes, the reaction mixture was evaporated. The residue was chromatographed on silica gel (20 g) eluting with a mixture of n-hexane and ethyl acetate (10:1 to 1:4) to give 4 - nitrobenzyl 2 - diazo - 4 - [(2R,3R) - 3 - {1 - methyl - 1 - (4 - nitrobenzyloxycarbonyloxy)ethyl} - 4 - oxoazetidin - 2 - yl] - 3 - oxobutanoate (1.08 g).

IR ($CH_2Cl_2$): 3400, 2150, 1765, 1750, 1720, 1655, 1525, 1350 $cm^{-1}$.

NMR ($CDCl_3$) δ: 1.68 (3H, s), 1.79 (3H, s), 3.4—3.7 (2H, m), 3.46 (2H, d, J=4.5Hz), 4.0—4.3 (1H, m), 5.20 (2H, s), 5.35 (2H, s), 6.22 (1H, s), 7.80 (4H, $A_2B_2$, J=9Hz), 7.85 (4H, $A_2B_2$, J=9Hz).

3)

A mixture of 4-nitrobenzyl 2-diazo-4-[(2R,3R)-3-{1-methyl-1-(4-nitorbenzyloxycarbonyloxy)ethyl}-4-oxoazetidin-2-yl]-3-oxobutanoate (1.00 g) and rhodium (II) actate (5 mg) in benzene (50 ml) was refluxed for 25 minutes. After cooling to ambient temperature, the mixture was filtered through a cellulose powder. The filtrate was evaporated to give 4-nitrobenzyl (2R, 5R, 6R) - 3,7 - dioxo - 6 - (1 - methyl - 1 - (4 -

nitrobenzyloxycarbonyloxy)ethyl] - 1 - azabicyclo[3.2.0]heptane - 2 - carboxylate (0.94 g) as an amorphous solid.

IR ($CH_2Cl_2$): 1770, 1750, 1525, 1350 $cm^{-1}$.

NMR ($CDCl_3$) δ: 1.64 (3H, s), 1.84 (3H, s), 2.74 (1H, dd, J=19Hz, 8Hz), 3.34 (1H, dd, J=19Hz, 8Hz), 3.77 (1H, d, J=6Hz), 4.34 (1H, dt, J=6Hz, 8Hz), 4.76 (1H, s), 5.18 (2H, s), 5.32 (2H, ABq, J=16Hz), 7.88 (4H, $A_2B_2$, J=9Hz), 7.91 (4H, $A_2B_2$, J=9Hz).

B) Preparation of active end products starting from the object compound:

1)

OCOOPNB, $(CH_3)_2C$, H, H, O, N, O, COOPNB → OCOOPNB, $(CH_3)_2C$, H, H, $S(CH_2)_2NHCOCH_3$, O, N, COOPNB

To a solution of 4-nitrobenzyl (2R,5R,6R) - 3,7 - dioxo - 6 - (1 - methyl - 1 - (4 - nitrobenzyloxycarbonyloxy)ethyl] - 1 - azabicyclo[3.2.0]heptane - 2 - carboxylate (58 mg) and 4-(N,N-dimethylamino)pyridine (1.3 mg) in acetonitrile (3 ml) was added a solution of diisopropylethylamine (22.4 μl) in acetonitrile (0.21 ml) at 0°C. After stirring at 0°C for 1.5 hours, the reaction mixture was cooled to −15°C, to which a solution of diisopropylethylamine (74.6 μl) in acetonitrile (0.73 ml) was added. To the mixture was added a solution of N-(2-mercaptoethyl)acetamide (13.4 mg) in acetonitrile (0.12 ml). After standing overnight at −15°C, the mixture was diluted with ethyl acetate (50 ml), washed in turn with water and saturated aqueous sodium chloride, and dried over magnesium sulfate. After evaporation of the solvent, the residue was chromatographed on silica gel (2 g) eluting with a mixture of methylene chloride and acetone (20:1 to 2:1) to give 4-nitrobenzyl (5R,6R) - 3 - (2 - acetamidoethylthio) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (56 mg) as an amorphous solid.

IR ($CH_2Cl_2$): 3400, 1775, 1745, 1695, 1675, 1530, 1350 $cm^{-1}$.

NMR ($CDCl_3$) δ: 1.68 (3H, s), 1.81 (3H, s), 1.96 (3H, s), 2.8—4.0 (6H, m), 3.76 (1H, d, J=6Hz), 4.2—4.6 (1H, m), 5.18 (2H, s), 5.38 (2H, ABq, J=15Hz), 6.30 (1H, broad t, J=6Hz), 7.88 (4H, $A_2B_2$, J=9Hz), 7.95 (4H, $A_2B_2$, J=9Hz).

2)

OCOOPNB, $(CH_3)_2C$, H, H, $S(CH_2)_2NHCOCH_3$, O, N, COOPNB → OH, $(CH_3)_2C$, H, H, $S(CH_2)_2NHCOCH_3$, O, N, COONa

A mixture of 5% palladium on activated carbon (175 mg), dioxane (12.5 ml) and 1/30M sodium phosphate buffer (pH 7.0, 5.5 ml) was shaken for an hour under a hydrogen atmosphere (2.76 bar, 40 psi) at ambient temperature. A solution of 4-nitrobenzyl (5R,6R) - 3 - (2 - acetamidoethylthio) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (175 mg) in a mixture of dioxane (12 ml) and 1/30M sodium phosphate buffer (pH7.0, 5 ml) was added to the mixture at 0°C and the resulting mixture was shaken under a hydrogen atmosphere (2.76 bar, 40 psi) for an hour, during which time the reaction temperature rose to ambient temperature. 1% Aqueous solution of sodium bicarbonate (2.29 ml) was added therein at 0°C and the mixture was filtered through cellulose powder. The filter-cake was washed with water (3 × 20 ml). The combined filtrates were concentrated to a half volume, washed with ethyl acetate (3 × 50 ml), and evaporated in vacuo. The residue was dissolved in water (220 ml) containing sodium chloride (6.6 g) and chromatographed on Diaion non-ionic adsorption resin, HP—20 AG (trade mark, made by Mitsubishi Chemical Industries) 150—300 μ, ϕ2.2 × 36 cm) eluting with water (360 ml) and a mixture of water and acetone (10:0 to 7:3, 600 ml). The fractions, whose UV spectra showed λmax 300 nm, were combined and lyophilized to give sodium (5R,6R) - 3 - (2 - acetamidoethylthio) - 6 - [1 - hydroxy - 1 - methylethyl) - 7 - oxo - 1 - azabicyclo[3.2.0]hept - 2 - ene - 2 - carboxylate (46.5 mg) as white powder.

IR (KBr): 1750, 1650, 1590, 1550, 1400 $cm^{-1}$.

NMR ($D_2O$) δ: 1.29 (3H, s), 1.40 (3H, s), 1.97 (3H, s), 2.90 (2H, t, J=8Hz), 3.04 (1H, dd, J=17Hz, 10Hz), 3.36 (2H, t, J=8Hz), 3.67 (1H, d, J=6Hz), 3.76 (1H, dd, J=17Hz, 9Hz), 4.14 (1H, ddd, J=6Hz, 9Hz, 10Hz).

3)

To a solution of 4-nitrobenzyl (2R,5R,6R) - 3,7 - dioxo - 6 - [1 - methyl - 1 - (4 - nitrobenzyloxycarbonyloxy)ethyl] - 1 - azabicylco[3.2.0]heptane - 2 - carboxylate (602 mg) and 4-(N,N-dimethylamino)-pyridine (13.6 ml) in acetonitrile (30 ml) was added a solution of diisopropylethylamine (0.232 ml) in acetonitrile (2.08 ml) at 0°C. To the mixture was further added a solution of diphenyl chlorophosphate (0.242 ml) in acetonitrile (2.18 ml). After stirring at 0°C for an hour, the mixture was cooled to −15°C. To the mixture was added a solution of diisopropylethylamine (0.775 ml) in acetonitrile (6.97 ml) and N-(4-nitrobenzyloxycarbonyl)cysteamine (299 mg) were added. After standing overnight at −15°C, the mixture was evaporated in vacuo. The residue was dissolved in ethyl acetate (100 ml) and the solution was washed in turn with water and saturated aqueous sodium chloride, dried over magnesium sulfate, and evaporated in vacuo. The residue was chromatographed on silica gel (18 g) eluting with a mixture of benzene and acetone (20:1 to 6:1) to give 4-nitrobenzyl (5R,6R) - 6 - [1 - methyl - 1 - (4 - nitrobenzyloxycarbonyloxy)ethyl] - 3 - [2 - (4 - nitrobenzyloxycarbonylamino)ethylthio] - 7 - oxo - 1 - azabicyclo[3.2.0]hept - 2 - ene - 2 - carboxylate (716 mg) as an amorphous solid.

IR ($CH_2Cl_2$): 3430, 1780, 1745, 1730, 1710, 1605, 1520, 1345 $cm^{-1}$.

NMR ($CDCl_3$) δ: 1.64 (3H, s), 1.80 (3H, s), 2.7—3.5 (5H, m), 3.5—4.0 (1H, m), 3.70 (1H, d, J=5Hz), 4.3 (1H, m), 5.09 (2H, s), 5.13 (2H, s), 5.32 (2H, ABq, J=13Hz), 7.78 (8H, $A_2B_2$, J=8Hz), 7.85 (4H, $A_2B_2$, J=8Hz).

4)

A mixture of platinum oxide (60.0 mg), water (4.2 ml), aqueous dipotassium hydrogen phosphate (1.0M, 0.385 ml) and ethanol (0.6 ml) was shaken for 30 minutes under a hydrogen atmosphere (3.45 bar, 50 psi) at ambient temperature. The mixture was added to a solution of 4-nitrobenzyl (5R,6R) - 6 - [1 - methyl - 1 - (4 - nitrobenzyloxycarbonyloxy)ethyl] - 3 - [2 - (4 - nitrobenzyloxycarbonylamino)-ethylthio] - 7 - oxo - 1 - azabicyclo - [3.2.0]hept - 2 - ene - 2 - carboxylate (60.0 mg) in dioxane (7.2 ml) and shaken for 2 hours under a hydrogen atmosphere (3.45 bar, 50 psi) at ambient temperature. The reaction mixture was filtered using diatomaceous earth and the filter-cake was washed with water (20 ml). The combined filtrates (32 ml) were lyophilized over a period of 4 hours. The residue was chromatographed on non-ionic adsorption resin, Diaion HP—20—AG (trade mark, made by Mitsubishi Chemical Industries) (13 × 230 mm) eluting with water (120 ml) and then a mixture of water and isopropanol (19:1, 60 ml). The fractions showing UV maxima at 296 nm were collected, concentrated and lyophilized to give potassium (5R,6R) - 3 - (2 - aminoethylthio) - 6 - (1 - hydroxy - 1 - methylethyl) - 7 - oxo - 1 - azabicyclo[3.2.0]hept - 2 - ene - 2 - carboxylate (10.4 mg) as pale yellow powder.

IR (Nujol): 3350, 1750, 1580, 1375 $cm^{-1}$.

UV ($H_2O$): 296 nm (ε = 6580).

NMR ($D_2O$) δ: 1.31 (3H, s), 1.43 (3H, s), 2.70—3.38 (6H, m), 3.69 (1h, d, J=5Hz), 4.25 (1H, dt, J=5,8Hz).

5)

A solution of diisopropylethylamine (0.386 ml) in acetonitrile (0.34 ml) was added to a solution of 4-nitrobenzyl (2R,5R,6R) - 3,7 - dioxo - 6 - [1 - methyl - 1 - (4 - nitrobenzyloxycarbonyloxy)ethyl] - 1 - azabicyclo[3.2.0]heptane - 2 - carboxylate (100 mg) and 4-(N,N-dimethylamino)pyridine (2.3 mg) in acetonitrile (5 ml) at 0°C.

A solution of diphenyl chlorophosphate (0.0402 ml) in acetonitrile (0.36 ml) was then added at 0°C.

After stirring at 0°C for 30 minutes, the mixture was evaporated in vacuo and the residue was dissolved in ethyl acetate. The organic solution was washed in turn with water and brine, dried over magnesium sulfate, and evaporated to give 4-nitrobenzyl (5R,6R) - 3 - (diphenoxyphosphoryloxy) - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (148 mg).

IR ($CH_2Cl_2$; 1780, 1745, 1725, 1640, 1520, 1485, 1345 $cm^{-1}$.

NMR ($CDCl_3$): 1.61 (s, 3H), 1.80 (s, 3H), 3.14 (dd, 1H, J=9.16Hz), 3.73 (d, 1H, J=6Hz), 3.5—4.0 (m, 1H), 4.0—4.3 (m, 1H), 5.00 (s, 2H), 5.18 (ABq, 2H, J=14 Hz), 7.0—7.7 (m, 14H), 7.8—8.2 (m, 4H).

6)

OCOOPNB, $(CH_3)_2C$, H, H, O, N, O, COOPNB → OCOOPNB, $(CH_3)_2C$, H, H, S, $NHCOCH_3$, O, N, H, H, COOPNB

A solution of diisopropylethylamine (0.0579 ml) in acetonitrile (0.5 ml) was added to a solution of 4-nitrobenzyl ((2R,5R,6R) - 3,7 - dioxo - 6 - [1 - methyl - 1 - (4 - nitrobenzyloxycarbonyloxy)ethyl] - 1 - azabicyclo[3.2.0]heptane - 2 - carboxylate (150 mg) and 4-(N,N-dimethylamino)pyridine (3.38 mg) in acetonitrile (20 ml) at 0°C. A solution of diphenylchlorophosphate (78 mg) in acetonitrile (0.54 ml) was added to the mixture which was stirred at 0°C for 15 minutes. Sodium iodide (415 mg) and silver (Z)-2-acetamidoethylenethiolate (93 mg) were added at 0°C and the mixture was stirred at 0°C for 2.5 hours.

After the precipitate was filtered off, the filtrate was evaporated in vacuo and the residue was dissolved in ethylacetate (50 ml). The organic solution was washed in turn with water and brine, dried over magnesium sulfate and evaporated in vacuo. The residue was chromatographed on silica gel (4 g) eluting with a mixture of benzene and acetone (20:1—8:1) to give 4-nitrobenzyl (5R,6R) - 3 - [(Z) - 2 - acetamidovinyl - thio] - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (155 mg) as an oil.

IR ($CH_2Cl_2$): 1785, 1750, 1710, 1630, 1515, 1350 $cm^{-1}$.

NMR ($CDCl_3$) δ: 1.62 (s, 3H), 1.78 (s, 3H), 2.06 (s, 3H), 2.96 (dd, 1H, J=11, 18 Hz), 3.66 (dd, 1H, J=8, 18Hz), 3.76 (d, 1H, J=5Hz);, 4.0—4.5 (m, 1H), 5.14 (s, 2H), 5.28 (d, 1H, J=7Hz), 5.37 (ABq 2H, J=13Hz), 7.3—7.8 (m, 6H), 8.22 (d, 4H, J=8Hz).

7)

OCOOPNB, $(CH_3)_2C$, H, H, S, $NHCOCH_3$, O, N, H, H, COOPNB → OH, $(CH_3)_2C$, H, H, S, $NHCOCH_3$, O, N, H, H, COOK

A mixture of 4-nitrobenzyl (5R,6R) - 3 - [(Z) - 2 - acetamidovinyl - thio] - 6 - [1 - methyl - 1 - (4 - nitrobenzyloxycarbonyloxy)ethyl] - 7 - oxo - 1 - azabicyclo[3.2.0]hept - 2 - ene - 2 - carboxylate (100 mg), platinum (IV) oxide monohydrate (50 mg), ethanol (1 ml) and 0.1 M aqueous solution of dipotassium hydrogen phosphate (4.68 ml) in a mixture of water (5 ml) and dioxane (12 ml) was shaken under a hydrogen atmosphere (2.76 bar, 40 psi) at ambient temperature for an hour. After the catalyst was filtered off, the filtrate was evaporated to the half volume.

The aqueous solution was washed three times with ether and evaporated. The residue was dissolved in water (50 ml) and potassium chloride (2.5 g) was added. The aqueous solution was chromatographed on a column of non-ionic adsorption resin Diaion HP—20 AG (trade mark, Mitsubishi Chemical Industries) eluting with water (200 ml) and 10% aqueous isopropyl alcohol (200 ml). The fractions, whose UV spectra showed λ max 308 nm, were combined and lyophilized to give potassium (5R,6R) - 3 - [(Z) - 2 - acetamido - vinylthio] - 6 - (1 - hydroxy - 1 - methylethyl) - 7 - oxo - 1 - azabicyclo[3.2.0]hept - 2 - ene - 2 - carboxylate (30 mg) as powder.

IR (nujol): 1750, 1670, 1620, 1585 $cm^{-1}$.

UV ($H_2O$): λ max 234 nm (ε = 11460), 308 nm (11650).

**Claims for the Contracting States: BE CH DE FR GB IT LI LU NL SE**

1. A carbapenam compound of the formula

wherein
$R^1$ is 1-protected hydroxy-1-methylethyl and
$R^2$ is carboxy or a protected carboxy and a salt thereof.

2. A compound according to claim 1, in which $R^1$ is 1-protected hydroxy-1-methylethyl and $R^2$ is a protected carboxy.

3. A compound according to claim 2, in which $R^1$ is 1-[mono(or di or tri)phenyl($C_1$—$C_6$)-alkoxycarbonyloxy]-1-methylethyl the phenyl moiety(ies) being optionally substituted by nitro and $R^2$ is mono(or di or tri)phenyl(lower)alkoxycarbonyl optionally substituted by nitro.

4. A compound according to claim 3, in which $R^1$ is 1-(4-nitrobenzyloxycarbonyloxy)-1-methylethyl and $R^2$ is 4-nitrobenzyloxycarbonyl.

5. A process for preparing a compound of claim 1, which comprises subjecting a compound of the formula:

wherein $R^1$ and $R^2$ are each as defined in claim 1 or a salt thereof, to cyclization.

**Claims for the Contracting State: AT**

1. A process for preparing a carbapenam compound of the formula:

(I)

wherein
$R^1$ is 1-protected hydroxy-1-methylethyl and
$R^2$ is carboxy or a protected carboxy and a salt thereof,
which comprises subjecting a compound of the formula:

wherein $R^1$ and $R^2$ are each as defined above or a salt thereof, to cyclization.

2. A process according to claim 1 for preparing a carbapenam compound in which $R^1$ is 1-protected hydroxy-1-methylethyl and $R^2$ is a protected carboxy.

3. A process according to claim 2 for preparing a carbapenam compound in which $R^1$ is 1-[mono(or di or tri)phenyl($C_1$—$C_6$)alkoxycarbonyloxy]-1-methylethyl the phenyl moiety(ies) being optionally substituted by nitro and $R^2$ is mono(or di or tri)phenyl(lower)alkoxycarbonyl optionally substituted by nitro.

4. A process according to claim 3 for preparing a carbapenam compound in which $R^1$ is 1-(4-nitrobenzyloxycarbonyloxy)-1-methylethyl and $R^2$ is 4-nitrobenzyloxycarbonyl.

**Patentansprüche für die Vertragsstaaten: BE CH DE FR GB IT LI LU NL SE**

1. Carbapenam-Verbindung der Formel:

(I)

worin
$R^1$ 1-geschütztes Hydroxy-1-methylethyl und
$R^2$ Carboxy oder geschütztes Carboxy bedeuten, und ein Salz davon.

2. Verbindung nach Anspruch 1, worin $R^1$ 1-geschütztes Hydroxy-1-methylethyl und $R^2$ ein geschütztes Carboxy bedeuten.

3. Verbindung nach Anspruch 2, worin $R^1$ 1-[Mono(oder Di-oder Tri)phenyl($C_1$—$C_6$)alkoxycarbonyloxy]-1-methylethyl, dessen Phenylrest(e) gegebenenfalls durch Nitro substituiert ist (sind), und $R^2$ Mono (oder Di- oder Tri)phenyl(niedrig)alkoxycarbonyl, ggf. substituiert durch Nitro, bedeuten.

4. Verbindung nach Anspruch 3, worin $R^1$ 1-(4-Nitrobenzyloxycarbonyloxy)-1-methylethyl und $R^2$ 4-Nitrobenzyloxycarbonyl bedeuten.

5. Verfahren zur Herstellung einer Verbindung nach Anspruch 1, bei dem man eine Verbindung der Formel

worin $R^1$ und $R^2$ jeweils wie in Anspruch 1 definiert sind, oder ein Salz davon einer Cyclisierung unterwirft.

**Patentansprüche für den Vertragsstaat: AT**

1. Verfahren zur Herstellung einer Carbapenam-Verbindung der Formel

(I)

worin
$R^1$ 1-geschütztes Hydroxy-1-methylethyl und
$R^2$ Carboxy oder geschütztes Carboxy bedeuten, oder eines Salzes davon,
bei dem man eine Verbindung der Formel

worin $R^1$ und $R^2$ jeweils wie oben definiert sind, oder ein Salz davon einer Cyclisierung unterwirft.

2. Verfahren nach Anspruch 1 zur Herstellung einer Carbapenam-Verbindung, worin $R^1$ 1-geschütztes Hydroxy-1-methylethyl und $R^2$ ein geschütztes Carboxy bedeuten.

3. Verfahren nach Anspruch 2 zur Herstellung einer Carbapenam-Verbindung, worin $R^1$ 1-[Mono(oder Di-oder Tri)phenyl($C_1$—$C_6$)alkoxycarbonyloxy]-1-methylethyl, dessen Phenylrest(e) ggf. durch Nitro substituiert ist (sind), und $R^2$ Mono (oder Di- oder Tri)phenyl(niedrig)alkoxycarbonyl, ggf. substituiert durch Nitro, bedeuten.

4. Verfahren nach Anspruch 3 zur Herstellung einer Carbapenam-Verbindung, worin $R^1$ 1-(4-Nitrobenzyloxycarbonyloxy)-1-methylethyl und $R^2$ 4-Nitrobenzyloxycarbonyl bedeuten.

**Revendications pour les Etats contractants: BE CH DE FR GB IT LI LU NL SE**

1. Carbapéname répondant à la formule:

(I)

dans laquelle

$R^1$ est un groupe hydroxy-1-méthyléthyle protégé en 1 et

$R^2$ est un groupe carboxy ou carboxy protégé et un de ses sels.

2. Composé selon la revendication 1, dans lequel $R^1$ est un groupe hydroxy-1-méthyléthyle protégé en 1 et $R^2$ est un groupe carboxy protégé.

3. Composé selon la revendication 2, dans lequel $R^1$ est un groupe 1-[mono(ou di ou tri)phényl(alcoxy en $C_1$ à $C_6$)carbonyloxy]-1-méthyléthyle, la ou les parties phényle étant substituées si on le désire par un groupe nitro, et $R^2$ est un groupe mono(ou di ou tri)phényl(alcoxy inférieur)carbonyle éventuellement substitué par un groupe nitro.

4. Composé selon la revendication 3, dans lequel $R^1$ est un groupe 1-(4-nitrobenzyloxycarbonyloxy)-1-méthyléthyle et $R^2$ est un groupe 4-nitrobenzyloxycarbonyle.

5. Procédé de préparation d'un composé selon la revendication 1 qui consiste à soumettre un composé répondant à la formule:

dans laquelle $R^1$ et $R^2$ sont chacun tels que définis dans la revendication 1 ou un de ses sels, à une cyclisation.

**Revendications pour l'Etat contractant: AT**

1. Procédé de préparation d'un carbapéname répondant à la formule:

(I)

dans laquelle

$R^1$ est un groupe hydroxy-1-méthyléthyle protégé en 1 et

$R^2$ est un groupe carboxy ou carboxy protégé ou un de ses sels,

qui consiste à soumettre un composé répondant à la formule:

dans laquelle $R^1$ et $R^2$ sont chacun tels que définis ci-dessus, ou un de ses sels à une cyclisation.

2. Procédé selon la revendication 1 pour préparer un carbapéname dans lequel: $R^1$ est un groupe hydroxy-1-méthyléthyle protégé en 1 et $R^2$ est un groupe carboxy protégé.

3. Procédé selon la revendication 2, pour préparer un carbapéname dans lequel; $R^1$ est un groupe 1-[mono(ou di ou tri)phényl(alcoxy en $C_1$ à $C_6$)carbonyloxy]-1-méthyléthyle dont la ou les parties phényle sont substituées si on le désire par un groupe nitro, et $R^2$ est un groupe mono(ou di ou tri)phényl(alcoxy inférieur)carbonyle substitué si on le désire par un groupe nitro.

4. Procédé selon la revendication 3, pour préparer un carbapéname dans lequel: $R^1$ est un groupe 1-(4-nitrobenzyloxycarbonyloxy)-1-méthyléthyle et $R^2$ est un groupe 4-nitrobenzyloxycarbonyl.